(19) Europäisches Patentamt
European Patent Office
Office européen des brevets

(11) EP 4 711 463 A1

(12) EUROPEAN PATENT APPLICATION
published in accordance with Art. 153(4) EPC

(43) Date of publication:
**18.03.2026 Bulletin 2026/12**

(21) Application number: **24807166.4**

(22) Date of filing: **10.05.2024**

(51) International Patent Classification (IPC):
***C12N 15/48*** (2006.01) ***A61K 9/14*** (2006.01)
***A61K 31/7088*** (2006.01) ***A61K 47/22*** (2006.01)
***A61K 47/24*** (2006.01) ***A61K 47/28*** (2006.01)
***A61K 47/34*** (2017.01) ***A61P 37/04*** (2006.01)
***C07K 14/15*** (2006.01) ***C12N 15/88*** (2006.01)

(52) Cooperative Patent Classification (CPC):
**A61K 9/14; A61K 31/7088; A61K 47/22; A61K 47/24; A61K 47/28; A61K 47/34; A61P 37/04; C07K 14/15; C12N 15/88**

(86) International application number:
**PCT/JP2024/017517**

(87) International publication number:
**WO 2024/237214 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.05.2023 JP 2023079064**

(71) Applicants:
- **Eisai R&D Management Co., Ltd.**
**Tokyo 112-8088 (JP)**
- **National University Corporation Kumamoto University**
**Kumamoto-shi, Kumamoto 860-8555 (JP)**

(72) Inventors:
- **SUZUKI, Yuta**
**Tsukuba-shi, Ibaraki 300-2635 (JP)**
- **KUBARA, Kenji**
**Tsukuba-shi, Ibaraki 300-2635 (JP)**
- **MIYAZAKI, Takayuki**
**Tsukuba-shi, Ibaraki 300-2635 (JP)**
- **MATSUOKA, Masao**
**Kumamoto-shi, Kumamoto 860-8555 (JP)**
- **YASUNAGA, Jun-ichirou**
**Kumamoto-shi, Kumamoto 860-8555 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) LIPID COMPLEX

(57) [Problem] A composition and a method that can be used to induce an immune response to HTLV-1 are required.

[Solution] A lipid complex comprising at least one nucleic acid selected from: a nucleic acid comprising a polynucleotide that encodes an immunogenic fragment of human T-cell leukemia virus 1 (HTLV-1) antigenic Gag protein; a nucleic acid comprising a polynucleotide that encodes an immunogenic fragment of HTLV-1 antigenic Tax protein; and a nucleic acid comprising a polynucleotide that encodes an immunogenic fragment of HTLV-1 antigenic HBZ protein; wherein the at least one nucleic acid is encapsulated in a lipid.

EP 4 711 463 A1

## Description

### Technical Field

The present disclosure relates to a lipid complex in which a nucleic acid is encapsulated by a lipid, and to a pharmaceutical composition that comprises this lipid complex. The present disclosure more particularly relates to a lipid complex that can be used to induce an immune response to HTLV-1, and to a pharmaceutical composition that comprises this lipid complex.

### Background Art

Human T-cell leukemia virus type-I (HTLV-1) is a retrovirus known to cause the development of diseases such as adult T-cell leukemia-lymphoma (ATL) and HTLV-1-associated inflammatory diseases, e.g., HTLV-1-associated myelopathy (HAM) and HTLV-1-associated uveitis (HU). These HTLV-1-associated diseases develop in HTLV-1-infected individuals (carriers). Approximately 800,000 HTLV-1-infected individuals (carriers) reportedly reside in Japan. In addition, there are no subjective symptoms in most cases even when HTLV-1 infection occurs.

Since ATL is resistant to treatment and has a poor prognosis, preventing infection with HTLV-1 is regarded as important for preventing the onset of ATL. In addition, ATL patients who have undergone hematopoietic stem cell transplantation may experience a relapse of ATL after transplantation. For these reasons, in order to improve the prognosis for ATL treatment, it is also important to induce an immune response against HTLV-1. However, effective treatment methods and onset prevention methods have not been established for these HTLV-1-associated diseases.

### Citation List

#### Non Patent Literature

NPL 1: Cook, Lucy B. et al., "Revised Adult T-Cell Leukemia-Lymphoma International Consensus Meeting Report." Journal of Clinical Oncology: official journal of the American Society of Clinical Oncology vol. 37, 8 (2019): 677-687. doi:10.1200/JC0.18.00501

### Summary of Invention

#### Technical Problem

A composition and method that can be used to induce an immune response to HTLV-1 are required.

#### Solution to Problem

The present disclosure is, for example, as follows.

[1] A lipid complex comprising at least one nucleic acid selected from:

- a nucleic acid comprising a polynucleotide that encodes an immunogenic fragment of human T-cell leukemia virus 1 (HTLV-1) antigenic Gag protein;
- a nucleic acid comprising a polynucleotide that encodes an immunogenic fragment of HTLV-1 antigenic Tax protein; and
- a nucleic acid comprising a polynucleotide that encodes an immunogenic fragment of HTLV-1 antigenic HBZ protein;

wherein the at least one nucleic acid is encapsulated in a lipid.

[2] The lipid complex according to [1], wherein the nucleic acid is at least one selected from:

- a nucleic acid comprising a polynucleotide that encodes HTLV-1 antigenic Gag protein p15 (Gag p15) or an immunogenic fragment thereof;
- a nucleic acid comprising a polynucleotide that encodes HTLV-1 antigenic Gag protein p19 (Gag p19) or an immunogenic fragment thereof;
- a nucleic acid comprising a polynucleotide that encodes HTLV-1 antigenic Gag protein p24 (Gag p24) or an immunogenic fragment thereof;

a nucleic acid comprising a polynucleotide that encodes HTLV-1 antigenic Tax protein or an immunogenic fragment thereof; and
a nucleic acid comprising a polynucleotide that encodes HTLV-1 antigenic HBZ protein or an immunogenic fragment thereof.

[3] The lipid complex according to [1] or [2], wherein the lipid comprises a cationic lipid and at least one lipid selected from the group consisting of neutral lipids, polyethylene glycol-modified lipids, and sterols.
[4] The lipid complex according to any of [1] to [3], wherein the nucleic acid comprises a polynucleotide that encodes at least one selected from Gag p15 or an immunogenic fragment thereof, Gag p19 or an immunogenic fragment thereof, and Gag p24 or an immunogenic fragment thereof.
[5] The lipid complex according to any of [1] to [4], wherein the nucleic acid is nucleic acid that comprises a polynucleotide that encodes;

(i) at least one amino acid sequence selected from SEQ ID NOs: 1, 4, 7, 8, and 11 to 14;
(ii) an amino acid sequence having at least 80% homology with at least one amino acid sequence selected from SEQ ID NOs: 1, 4, 7, 8, and 11 to 14;
(iii) an amino acid sequence in which 1 to 31 amino acids are deleted, substituted, inserted, and/or added in an amino acid sequence with SEQ ID NO: 1, 4, 7, 8, 11, 12, 13, or 14;
(iv) an amino acid sequence comprising at least one amino acid sequence selected from SEQ ID NO: 11 or SEQ ID NO: 12, SEQ ID NO: 13, or SEQ ID NO: 14, and having at least 80% homology with SEQ ID NO: 1;
(v) an amino acid sequence comprising at least one of the amino acid sequence at positions 1 to 59, 52 to 109, or 102 to 130 of SEQ ID NO: 1;
(vi) an amino acid sequence comprising at least one of the amino acid sequence at positions 1 to 59 , 52 to 109, 102 to 130 of SEQ ID NO: 1, and having at least 80% homology with SEQ ID NO: 1;
(vii) an amino acid sequence comprising at least one of the amino acid sequence at positions 151 to 208 or 251 to 308 of SEQ ID NO: 4;
(viii) an amino acid sequence comprising at least one of the amino acid sequence at positions 151 to 208 or 251 to 308 of SEQ ID NO: 4, and having at least 80% homology with SEQ ID NO: 4;
(ix) the amino acid sequence at positions 1 to 58 of SEQ ID NO: 8; or (x) an amino acid sequence comprising the amino acid sequence at positions 1 to 58 of SEQ ID NO: 8 and having at least 80% homology with SEQ ID NO: 8.

[6] The lipid complex according to any one of [1] to [5], wherein the nucleic acid comprises a polynucleotide that encodes an antigenic Gag protein.
[7] The lipid complex according to any of [1] to [6], wherein the lipid complex is a lipid nanoparticle (LNP).
[8] The lipid complex according to any of [1] to [7], wherein the nucleic acid is mRNA.
[9] The lipid complex according to [8], wherein the nucleic acid is nucleic acid that comprises at least one selected from:

(i) a polynucleotide composed of at least one base sequence selected from the group consisting of SEQ ID NOs: 2, 3, 5, 6, 9, 10, and 15 to 17;
(ii) a polynucleotide composed of a base sequence in which 1 to 94 bases are deleted, inserted, substituted, and/or added in a base sequence according to (i);
(iii) a polynucleotide composed of a base sequence having at least 80% identity with a base sequence according to (i); and
(iv) a polynucleotide that hybridizes under stringent conditions with polynucleotide composed of a base sequence complementary to a base sequence according to (i);
(v) a polynucleotide composed of at least one base sequence of the base sequence at positions 1 to 177, 154 to 327, or 304 to 390 of SEQ ID NO: 3;
(vi) a polynucleotide composed of a base sequence that comprises at least one of the base sequence at positions 1 to 177, 154 to 327, 304 to 390 of SEQ ID NO: 3, and that has at least 80% identity with the base sequence of SEQ ID NO: 3;
(vii) polynucleotide composed of at least one base sequence of the base sequence at positions 451 to 624 or 751 to 924 of SEQ ID NO: 6;
(viii) polynucleotide composed of a base sequence that comprises at least one of the base sequence at positions 451 to 624 or 751 to 924 of SEQ ID NO: 6, and that has at least 80% identity with the base sequence of SEQ ID NO: 6;
(ix) polynucleotide composed of the base sequence at positions 1 to 174 of SEQ ID NO: 10; (x) polynucleotide composed of a base sequence that comprises the base sequence at positions 1 to 174 of SEQ ID NO: 10 and that has at least 80% identity with the base sequence of SEQ ID NO: 10.

[10] The lipid complex according to any of [1] to [9], wherein the cationic lipid includes a compound represented by the following formula (I)

[C1]

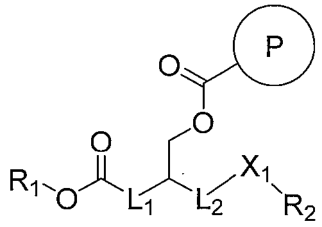

(I)

[In the formula, $L_1$ and $L_2$ each independently represent a $C_{3-10}$ alkylene group, $R_1$ and $R_2$ each independently represent a $C_{4-22}$ alkyl group or a $C_{4-22}$ alkenyl group, $X_1$ represents a single bond or - CO - O -, and the ring P represents any of the following formulas (P-1) to (P-6).]

[C2]

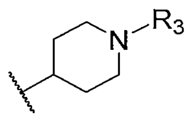
(P-1)

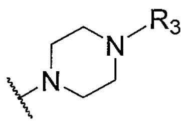
(P-2)

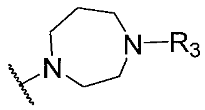
(P-3)

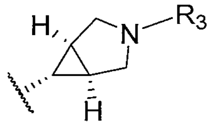
(P-4)

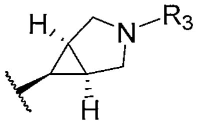
(P-5)

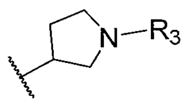
(P-6)

[In the formulas, $R_3$ represents a $C_{1-3}$ alkyl group.]
or a pharmaceutically acceptable salt thereof.
[11] The lipid complex according to any of [1] to [10], wherein the cationic lipid comprises 2-{9-oxo-9-[(3-pentyloctyl) oxy]nonyl}dodecyl 1-methylpiperidine-4-carboxylate represented by the following formula (II)

[C3]

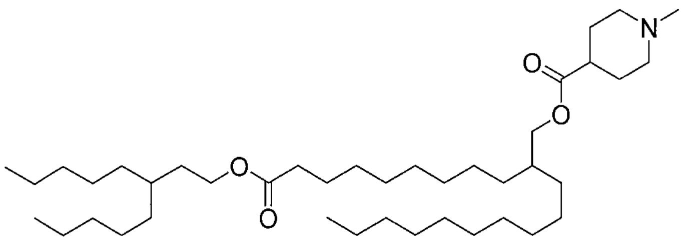

(II)

or a pharmaceutically acceptable salt thereof.
[12] A pharmaceutical composition comprising a lipid complex according to any of [1] to [11].
[13] The pharmaceutical composition according to [12], wherein the pharmaceutical composition is a vaccine.
[14] The pharmaceutical composition according to [11] or [12], for use for the prevention and/or treatment of an HTLV-1-associated disease.
[15] Use of a lipid complex according to any of [1] to [11] in the manufacture of a pharmaceutical composition.
[16] A method for the prevention and/or treatment of an HTLV-1-associated disease, comprising administering a pharmaceutical composition according to [12] or [13] to a subject.
[17] Use of the lipid complex according to any of [1] to [11], or the pharmaceutical composition according to [12] or [13], for use in the prevention and/or treatment of an HTLV-1-associated disease.

Effects of Invention

The present disclosure provides a lipid complex and pharmaceutical composition that can induce an immune response to HTLV-1. The pharmaceutical composition of the present disclosure, because it can induce an immune

response to HTLV-1, can be used for the prevention and/or treatment of HTLV-1-associated diseases. In some embodiments, the pharmaceutical composition of the present disclosure can be used as an mRNA vaccine for the prevention of HTLV-1-associated diseases. In some embodiments, the lipid complex (lipid nanoparticles in particular) of the present disclosure has an excellent stability.

Brief Description of Drawings

[Fig. 1] Fig. 1 is a graph that reports results for the ELISpot assay in Example 2. Fig. 1 gives results that show the number of spots for each peptide pool.
[Fig. 2] Fig. 2 is a graph that reports results for the ELISpot assay in Example 2. Fig. 2 gives results that show the number of spots for each peptide pool.
[Fig. 3] Fig. 3 is a graph that reports results for the ELISpot assay in Example 2. Fig. 3 gives results that show the number of spots for each peptide pool.
[Fig. 4] Fig. 4 contains graphs that report results for the LIPS assay in Reference Example 1.
[Fig. 5] Fig. 5 contains graphs that report results for the RT-qPCR in Reference Example 1.
[Fig. 6] Fig. 6 contains photographs that report results for the proximity ligation assay in Reference Example 1.
[Fig. 7] Fig. 7 contains photographs that report results for the ELISpot assay in Reference Example 1.
[Fig. 8] Fig. 8 contains photographs that report results for the ELISpot assay in Reference Example 1.

Description of Embodiments

The present disclosure is described in detail in the following by providing, inter alia, embodiments and examples, but the present disclosure is not limited to or by the embodiments, examples, and so forth that are provided in the following, and the present disclosure can be implemented through freely selected alterations and modifications in a range that does not depart from the essential features of the present disclosure. All of the documents and publications referenced in the present Description are incorporated in their entirety in the present Description by reference regardless of their purpose.

<Terminology>

Below, the terms and so forth used in the present Description are defined and the present disclosure is described in detail.

"Cationic lipid" refers to an amphiphilic molecule that has both a lipophilic region comprising at least one hydrocarbon group and a hydrophilic region comprising a polar group that is protonated at a prescribed pH.
"Neutral lipid" refers to a lipid that is present, at physiological pH, either in an uncharged form or in the form of a neutral zwitterion.

A "polyethylene glycol-modified lipid" (PEG lipid) refers to a lipid that has a polyethylene glycol (PEG) group.

A "sterol" refers to an alcohol that has a steroid skeleton.

"Alkyl" is defined as a straight-chain, cyclic, or branched saturated aliphatic hydrocarbon group having a designated number of carbon atoms.

"Alkenyl" is defined as a straight-chain or branched hydrocarbon group that has a designated number of carbon atoms and at least one carbon-carbon double bond. Examples are monoenes, dienes, trienes, and tetraenes, but there is no limitation to these.

"Alkylene" is defined as a straight-chain, cyclic, or branched divalent saturated aliphatic hydrocarbon group having a designated number of carbon atoms.

A "lipid complex" indicates an aggregate that comprises a plurality of lipids physically bound to each other by intermolecular forces. A "lipid complex" is typically a "lipid particle".

A "lipid particle" is a particle composed of an aggregate of lipid molecules. The "particle" is typically a particle having an average particle diameter from nanosizes to microsizes (1 nm to 1 μm or um) and known as, for example, a nanosphere, microsphere, nanocapsule, or microcapsule. The particle can be, for example, the phase dispersed in an emulsion or the internal phase in a suspension. A "lipid particle" is typically a microparticle composed of a membrane-like molecular aggregate formed by the association of lipids.

A "lipid nanoparticle" (LNP) denotes a lipid particle having a nanosize average particle diameter (for example, approximately 1 nm to 1,000 nm).

"Protein" denotes a peptide polymer composed of unmodified amino acids (natural amino acids), modified amino acids, and/or artificial amino acids. The configuration of this polymer is, for example, straight-chain, branched, or cyclic. A protein can also be referred to as a peptide or a polypeptide.

A "retrovirus" is a type of RNA virus and denotes an RNA virus that has a reverse transcriptase. It is known that a retrovirus infects a host cell and propagates as follows. The retrovirus infects the host cell that is the infection target, double-stranded DNA is synthesized by reverse transcription of the viral RNA, and the viral DNA is incorporated as a provirus into the genomic DNA of the host cell. The host cell then produces viral RNA and viral proteins, a retroviral protease cleaves the immature viral proteins, and the mature viral proteins are assembled to form viral particles. The viral particles then bud from the surface of the host cell.

"HTLV-1" is a type of retrovirus and refers to human T-cell leukemia virus type 1. HTLV-1 is a virus known to cause diseases such as adult T-cell leukemia-lymphoma (ATL (or ATLL)), HTLV-1-associated myelopathy (HAM, TSP: tropical spastic paraparesis), and HTLV-1 uveitis (HU). HTLV-1 is a virus belonging to the Oncovirinae subfamily of the Retroviridae family. HTLV-1 has viral structural genes such as Gag, Pol, and Env. HTLV-1 also has regulatory genes such as Tax and Rex. HTLV-1 additionally has accessory genes such as p12, p13, p30, and HBZ. The proteins composed of the amino acid sequences registered at Genbank under the accession number AB513134.1 are an example for proviral HTLV-1.

"HTLV-1-associated diseases" is a general term for diseases caused by HTLV-1 infection. Examples of "HTLV-1-associated diseases" are 1) ATL, 2) HAM, and 3) HU.

"ATL" or "ATLL" refers to adult T-cell leukemia-lymphoma. It is known that in HTLV-1-infected individuals (carriers), HTLV-1 infects, inter alia, CD4+ T cells, resulting in the development of ATL by causing the infected cells to become cancerous.

"HAM" refers to HTLV-1-associated myelopathy (TSP: tropical spastic paraparesis).

"HU" refers to HTLV-1 uveitis or HTLV-1-associated uveitis.

"Processing" refers to reactions whereby the translated polypeptide serving as a precursor is cleaved by, e.g., a protease, or is modified and is thereby processed into a more mature protein.

"Gag protein (also referred to as Gag in the following)" refers to a retroviral capsid precursor protein. Gag is known to play a role in, e.g., the assembly and budding of virus particles. Gag is processed by retroviral protease. The Gag of HTLV-1 is processed by the protease into p15 (nucleocapsid protein), p19 (matrix protein), p24 (capsid protein), and so forth. An example of the Gag of HTLV-1 is the protein (SEQ ID NO: 1) composed of the amino acid sequence registered at Genbank under accession number AAA85841.1.
Amino acid sequence (SEQ ID NO: 1) of HTLV-1 Gag

MGQIFSRSASPIPRPPRGLAAHHWLNFLQAAYRLEPGPSSYDFHQLKKFLKIALETPV

WICPINYSLLASLLPKGYPGRVNEILHILIQTQAQIPSRPAPPPPSSPTHDPPDSDPQIPPP

YVEPTAPQVLPVMHPHGAPPNHRPWQMKDLQAIKQEVSQAAPGSPQFMQTIRLAVQ

QFDPTAKDLQDLLQYLCSSLVASLHHQQLDSLISEAETRGITGYNPLAGPLRVQANN

PQQQGLRREYQQLWLAAFAALPGSAKDPSWASILQGLEEPYHAFVERLNIALDNGLP

EGTPKDPILRSLAYSNANKECQKLLQARGHTNSPLGDMLRACQTWTPKDKTKVLVV

QPKKPPPNQPCFRCGKAGHWSRDCTQPRPPPGPCPLCQDPTHWKRDCPRLKPTIPEPE

PEEDALLLDLPADIPHPKNSIGGEV

"Tax protein (also referred to as Tax in the following)" refers to a transcription activator that activates both the retroviral LTR (long terminal repeat) and intracellular promoters. Tax is known to play an important role in viral infectivity and malignant transformation of infected cells. An example of the TAX of HTLV-1 is the protein (SEQ ID NO: 4) composed of the amino acid sequence registered at Genbank under accession number P03409.2.
Amino acid sequence (SEQ ID NO: 4) of HTLV-1 TAX

MAHFPGFGQSLLFGYPVYVFGDCVQGDWCPISGGLCSARLHRHALLATCPEHQITW

DPIDGRVIGSALQFLIPRLPSFPTQRTSKTLKVLTPPITHTTPNIPPSFLQAMRKYSPFRN

GYMEPTLGQHLPTLSFPDPGLRPQNLYTLWGGSVVCMYLYQLSPPITWPLLPHVIFC

HPGQLGAFLTNVPYKRIEELLYKISLTTGALIILPEDCLPTTLFQPARAPVTLTAWQNG

LLPFHSTLTTPGLIWTFTDGTPMISGPCPKDGQPSLVLQSSSFIFHKFQTKAYHPSFLLS

HGLIQYSSFHSLHLLFEEYTNIPISLLFNEKEADDNDHEPQISPGGLEPPSEKHFRETEV

"HBZ protein (also referred to as HBZ in the following)" refers to a retroviral bZIP factor. HBZ is known to contribute to control of viral RNA transcription. An example of HTLV-1 HBZ is the protein (SEQ ID NO: 7) composed of the amino acid sequence registered at Genbank under accession number BAE06226.1. In addition, HTLV-1 HBZ may be, for example, the protein composed of the amino acid sequence given by SEQ ID NO: 8.

Amino acid sequence (SEQ ID NO: 7) of HTLV-1 HBZ

MAASGLFRCLPVSCPEDLLVEELVDGLLSLEEELKDKEEEEAVLDGLLSLEEESRGRL

RRGPPGEKAPPRGETHRDRQRRAEEKRKRKKEREKEEEKQTAEYLKRKEEEKARRR

RRAEKKAADVARRKQEEQERRERKWRQGAEKAKQHSARKEKMQELGIDGYTRQL

EGEVESLEAERRKLLQEKEDLMGEVNYWQGRLEAMWLQ

Amino acid sequence (SEQ ID NO: 8) of HTLV-1 HBZ

MAASGLFRCLPVSCPEDLLVEELVDGLLSLEEELKDKEEEEAVLDGLLSLEEESRGRL

RRGPPGEKAPPRGETHRDRQRRAEEKRKRKKEREKEEEKQIAEYLKRKEEEKARRR

RRAEKKAADVARRKQEEQERRERKWRQGAEKAKQHSARKEKMQELGIDGYTRQL

EGEVESLEAERRKLLQEKEDLMGEVNYWQGRLEAMWLQ

"T cells" refer to cells that are a type of leukocyte, are classified as lymphocytes, and express a T cell receptor (TCR). Examples of T cells include CD4+ helper T cells and CD8+ cytotoxic T cells.

"Antigenic" denotes the property wherein an antigen induces an immune response, e.g., antibody production or cellular immunity.

"Immunogenic" denotes the property wherein an antigen induces an immune response, e.g., antibody production or cellular immunity. "Immunogenic fragment", also referred to as an immunogenic site, refers to an immune response-inducing fragment of a protein or polypeptide or an immune response-inducing truncated protein or polypeptide.

A "vaccine" denotes a composition that produces an immune response for the prevention and/or treatment of a disease or condition. A vaccine is thus a pharmaceutical product that comprises an immunogenic drug and is designed for use to produce a specific defense and protective substances in humans or animals by vaccination.

A "pharmaceutically acceptable carrier" refers to a solvent and/or additive that can be generally used in pharmaceutical composition formulation technology. This pharmaceutically acceptable carrier is preferably almost or completely free of toxicity for organisms.

A "nucleic acid" denotes polymers of deoxyribonucleotides (DNA), ribonucleotides (RNA), and/or modified nucleotides. In the present Description, when "nucleic acid" is used in combination with a specific protein, the "nucleic acid" then denotes a nucleotide polymer that encodes the amino acid sequence of the protein. The nucleic acid may be, for

example, genomic DNA, cDNA, mRNA, and so forth. The nucleic acid may be, for example, single-stranded or double-stranded. The nucleic acid may be interchangeably read as "polynucleotide" or "oligonucleotide". In the present disclosure, when the nucleic acid is DNA, for example, thymine (t) can be read for uracil (u) in the examples of the RNA polynucleotide base sequences given in the individual SEQ ID NOs provided in the following, and this description can be used for the base sequence of the DNA.

"Treatment" denotes reversing, alleviating, delaying the development of, or inhibiting the progression of the diseases described in the present Description.

"Prevention" denotes reducing the potential for the onset of a disease or pathology; suppressing, delaying, or stopping the onset of a disease or pathology; suppressing, alleviating, delaying, or stopping the progression of a pathology; suppressing, reducing, delaying, or stopping the aggravation of a disease or pathology; or suppressing, delaying, or stopping the recurrence of a disease or pathology. This "prevention" may be, for example, the treatment of a subject (patient) who has developed the target disease, or the treatment of a model animal for the target disease.

"Aggravation" denotes a worsening of the degree (severity) of a disease or pathology.

"Isolated" denotes a state of being identified and separated, and/or a state of being recovered from components in the natural state. This "isolation" can be achieved, for example, by adopting at least one purification step.

The upper limit value and lower limit value for the numerical value ranges provided in the present Description can be freely combined. For example, when "A to B" and "C to D" are provided, the ranges "A to D" and "C to B" are also included in the scope of the present disclosure as numerical value ranges. In addition, the numerical value ranges provided in the present Description in the form of "lower limit value to upper limit value" denote equal to or greater than the lower limit value and equal to or less than the upper limit value.

In the present Description, the expression "A and/or B" includes "A only", "B only", and "both A and B".

In the present Description, the numerical values used to indicate component contents, numerical value ranges, and so forth should be understood as being modified by the term "approximately" unless specifically indicated otherwise.

1. The lipid complex

One aspect of the present disclosure relates to a lipid complex (hereinafter also referred to as the "lipid complex of the present disclosure" or the "lipid complex") comprising at least one nucleic acid selected from: a nucleic acid comprising a polynucleotide that encodes an immunogenic fragment of human T-cell leukemia virus 1 (HTLV-1) antigenic Gag protein; a nucleic acid comprising a polynucleotide that encodes an immunogenic fragment of HTLV-1 antigenic Tax protein; and a nucleic acid comprising a polynucleotide that encodes an immunogenic fragment of HTLV-1 antigenic HBZ protein; wherein the at least one nucleic acid is encapsulated in the lipid.

(The nucleic acids)

The lipid complex of the present disclosure, for example, may comprise any one of the following, a plurality of the following, or all of the following: a nucleic acid comprising a polynucleotide that encodes an immunogenic fragment of the aforementioned Gag, a nucleic acid comprising a polynucleotide that encodes the aforementioned Tax or an immunogenic fragment thereof, and a nucleic acid comprising a polynucleotide that encodes the aforementioned HBZ or an immunogenic fragment thereof.

The lipid complex of the present disclosure characteristically comprises a nucleic acid comprising a polynucleotide that encodes an immunogenic fragment of HTLV-1 antigenic Gag protein, a nucleic acid comprising a polynucleotide that encodes HTLV-1 antigenic Tax protein or an immunogenic fragment thereof, and a nucleic acid comprising a polynucleotide that encodes HTLV-1 antigenic HBZ protein or an immunogenic fragment thereof. Because it comprises these nucleic acids, the lipid complex of the present disclosure, when administered to a subject, can induce an immune response to HTLV-1 and in particular to HTLV-1 Gag, Tax, and/or HBZ. The lipid complex of the present disclosure can thus be used to induce an immune response to HTLV-1. In addition, the lipid complex of the present disclosure can be used to prevent and/or treat HTLV-1-associated diseases.

In some embodiments, the nucleic acid is at least one selected from the following:

- nucleic acid comprising a polynucleotide that encodes HTLV-1 antigenic Gag protein p15 (Gag p15) or an immunogenic fragment thereof;
- nucleic acid comprising a polynucleotide that encodes HTLV-1 antigenic Gag protein p19 (Gag p19) or an immunogenic fragment thereof;
- nucleic acid comprising a polynucleotide that encodes HTLV-1 antigenic Gag protein p24 (Gag p24) or an immunogenic fragment thereof;
- nucleic acid comprising a polynucleotide that encodes HTLV-1 antigenic Tax protein or an immunogenic fragment thereof; and

- nucleic acid comprising a polynucleotide that encodes HTLV-1 antigenic HBZ protein or an immunogenic fragment thereof.

In some embodiments, the nucleic acid includes nucleic acid comprising polynucleotide that encodes at least one selected from Gag p15 or an immunogenic fragment thereof, Gag p19 or an immunogenic fragment thereof, and Gag p24 or an immunogenic fragment thereof.

The nucleic acid may be composed of deoxynucleotide residues, or may be composed of ribonucleotide residues, or may be composed of both. The nucleic acid may be composed of natural nucleic acid residues, or may be composed of non-natural nucleic acid residues, or may be composed of both. As specific examples, the nucleic acid may comprise, for example, DNA, RNA, and/or DNA/RNA composed of natural and/or non-natural nucleic acid residues. The non-natural nucleic acid residues can be exemplified by modified nucleotide residues or modified ribonucleotide residues in which the base, sugar residue, or sugar phosphate backbone of the nucleotide residue has been modified. In the case of modification of the sugar residue, the non-natural nucleic acid residue can be exemplified by cEt (constrained ethyl bicyclic nucleic acid, Ionis Pharmaceuticals, Inc.), LNA (trademark, Locked Nucleic Acid), and ENA (registered trademark, 2'-O,4'-C-Ethylenebridged Nucleic Acid). The nucleic acid may have, for example, a 5' cap at the 5' end.

The nucleic acid may be a single-stranded nucleic acid molecule or a double-stranded nucleic acid molecule.

The nucleic acid residing in the lipid complex and comprising a polynucleotide that encodes a particular protein as described above, for example, can be designed by substituting the corresponding codons based on the amino acid sequence of the particular protein. The base sequence of the nucleic acid in the pharmaceutical composition of the present disclosure, for example, may be codon optimized.

The various nucleic acids and proteins for the lipid complex can be synthesized, for example, by genetic engineering techniques or organic synthesis techniques, and can also be referred to as synthetic DNA, e.g., cDNA, or synthetic RNA.

The nucleic acid can be RNA or DNA in some embodiments.

The nucleic acid is mRNA in specific embodiments.

When the nucleic acid in the present disclosure is mRNA, this nucleic acid may comprise, e.g., a 5' cap structure, a poly-A sequence, and so forth, at the terminals.

Nucleic acid that can be comprised in the lipid complex of the present disclosure is described in the following.

(I) Nucleic acid comprising a polynucleotide that encodes an immunogenic fragment of HTLV-1 antigenic Gag protein

The nucleic acid can be a nucleic acid that comprises a polynucleotide that encodes an immunogenic fragment of HTLV-1 antigenic Gag protein (Gag). The polynucleotide that encodes an immunogenic fragment of Gag is a polynucleotide that necessarily comprises a region that encodes an immunogenic fragment of Gag. This immunogenic fragment of Gag should be range that is a protein having immunogenicity with respect to HTLV-1.

The nucleic acid may be a nucleic acid that comprises a polynucleotide that encodes an immunogenic fragment of Gag. In addition, the nucleic acid may be composed of a polynucleotide that encodes an immunogenic fragment of Gag. For example, when the nucleic acid is mRNA, the nucleic acid may comprise, for example, a 5' cap structure, a 5'-UTR, a 3'-UTR, a poly-A sequence, and so forth in addition to a polynucleotide that encodes an immunogenic fragment of Gag.

The amino acid length of the immunogenic fragment of Gag may be a length of at least 5, 9, 10, 20, 30, 40, 50, 60, 70, 80, 90, 100, or more than 100 amino acids.

In some embodiments, the polynucleotide encoding an immunogenic fragment of Gag comprises a polynucleotide that encodes at least one selected from HTLV-1 antigenic Gag protein p15 (Gag p15) or an immunogenic fragment thereof, Gag protein p19 (Gag p19) or an immunogenic fragment thereof, and Gag protein p24 (Gag p24) or an immunogenic fragment thereof. Thus, in some embodiments, the nucleic acid is a nucleic acid comprising a polynucleotide that encodes at least one selected from Gag p15 or an immunogenic fragment thereof, Gag p19 or an immunogenic fragment thereof, and Gag p24 or an immunogenic fragment thereof.

The nucleic acid may be a nucleic acid that comprises a polynucleotide that encodes Gag p15 or an immunogenic fragment thereof, Gag p19 or an immunogenic fragment thereof, and/or Gag p24 or an immunogenic fragment thereof. In addition, the nucleic acid may be a polynucleotide that encodes Gag p19 or an immunogenic fragment thereof and/or Gag p24 or an immunogenic fragment thereof. For example, when the nucleic acid is mRNA, the nucleic acid may comprise a 5' cap structure, a 5'-UTR, a 3'-UTR, a poly-A sequence, and so forth in addition to the polynucleotide that encodes Gag p15 or an immunogenic fragment thereof, Gag p19 or an immunogenic fragment thereof, and/or Gag p24 or an immunogenic fragment thereof.

In addition, the Gag p15, Gag p19, and Gag p24 may be further processed to a length of, for example, 50 amino acids or 9 amino acids. In some embodiments, the lipid membrane complex may encapsulate nucleic acid that comprises a polynucleotide that encodes the 50 amino acids or 9 amino acids yielded by the further processing of Gag p15, Gag p19, or Gag p24.

(a) Gag p15 or an immunogenic fragment thereof

Gag p15 can be exemplified by the following proteins (a1), (a2), and (a3).

(a1) a protein composed of the amino acid sequence of SEQ ID NO: 11 or 12
(a2) a protein composed of an amino acid sequence in which one or a plurality of amino acids are deleted, inserted, substituted, or added in the amino acid sequence of SEQ ID NO: 11 or 12
(a3) a protein composed of an amino acid sequence having an at least 80% identity with the amino acid sequence of SEQ ID NO: 11 or 12

Amino acid sequence (SEQ ID NO: 11) of Gag p15

VVQPKKPPPNQPCFRCGKAGHWSRDCTQPRPPPGPCPLCQDPTHWKRDCPRLKPTIP

EPEPEEDALLLDLPADIPHPKN<u>LHRGGGLTSPPTLQQVLPNQDPASIL</u>

Amino acid sequence (SEQ ID NO: 12) of Gag p15

VVQPKKPPPNQPCFRCGKAGHWSRDCTQPRPPPGPCPLCQDPTHWKRDCPRLKPTIP

EPEPEEDALLLDLPADIPHPKN<u>SIGGEV</u>

In the preceding (a1), the amino acid sequence given by SEQ ID NO: 11 or 12 is an amino acid sequence derived from HTLV-1. In addition, in the preceding (a1), the amino acid sequence given by SEQ ID NO: 11 is, for example, the sequence of endogenous mature Gag p15 that can be expressed in infected cells and is produced by the ribosome generating a frameshift. The amino acid sequence given by SEQ ID NO: 12 is, for example, a Gag p15 sequence that is produced when the ribosome does not generate a frameshift. For example, the amino acid sequence of SEQ ID NO: 12, which is predicted to be expressed in large amounts in vivo when used for a vaccine composition, is preferred for the amino acid sequence of (a1).

The "one or a plurality of" in the case of the protein (a2), for example, should be a range in which (a2) is a protein having immunogenicity with respect to HTLV-1. The "one or a plurality of" for (a2) is, for example, 1 to 31, 1 to 26, 1 to 25, 1 to 21, 1 to 15, 1 to 12, 1 to 10, 1 to 8, 1 to 5, 1 to 4, 1 to 3, 1 or 2, or 1 in the amino acid sequence of (a1). In the present disclosure, a numerical value range for a number of items, for example, indicates all of the positive integers belonging to that range. That is, for example, the phrase "1 to 5" indicates all of "1, 2, 3, 4, and 5" (this also applies in the following).

In the case of the protein (a2), the substitution is preferably a conservative substitution. A conservative substitution means that an amino acid residue is substituted by an amino acid residue having a similar side chain. Examples of conservative substitution are as follows: substitution between amino acid residues having a basic side chain, such as lysine, arginine, and histidine; substitution between amino acid residues having an acidic side chain, such as aspartic acid and glutamic acid; substitution between amino acid residues having an uncharged polar side chain, such as glycine, asparagine, glutamine, serine, threonine, tyrosine, and cysteine; substitution between amino acid residues having a nonpolar side chain, such as alanine, valine, leucine, isoleucine, proline, phenylalanine, methionine, and tryptophan; substitution between amino acid residues having a β-branched side chain, such as threonine, valine, and isoleucine; and substitution between amino acid residues having an aromatic side chain, such as tyrosine, phenylalanine, tryptophan, and histidine.

In the case of the protein (a3), the "identity", for example, should be a range in which (a3) is a peptide having immunogenicity with respect to HTLV-1. The "identity" of (a3) is, for example, at least 70%, at least 75%, at least 80%, at least 85%, at least 90%, at least 95%, at least 96%, at least 97%, at least 98%, or at least 99% relative to the amino acid sequence of (a1). The "identity" can be calculated, for example, by using default parameters in the BLAST homology algorithm (http://www.ncbi.nlm.nih.gov/BLASTI) of the National Center for Biotechnology Information (NCBI) (this also applies in the following).

(b) Gag p19 or an immunogenic fragment thereof

Gag p19 can be exemplified by the following proteins (b1), (b2), and (b3).

(b1) a protein composed of the amino acid sequence of SEQ ID NO: 13
(b2) a protein composed of an amino acid sequence in which one or a plurality of amino acids are deleted, inserted,

substituted, or added in the amino acid sequence of SEQ ID NO: 13
(b3) a protein composed of an amino acid sequence having an at least 80% identity with the amino acid sequence of SEQ ID NO: 13

Amino acid sequence (SEQ ID NO: 13) of Gag p19

MGQIFSRSASPIPRPPRGLAAHHWLNFLQAAYRLEPGPSSYDFHQLKKFLKIALETPV
WICPINYSLLASLLPKGYPGRVNEILHILIQTQAQIPSRPAPPPPSSPTHDPPDSDPQIPPP
YVEPTAPQVL

The "one or a plurality of" in the case of the protein (b2), for example, should be a range in which (b2) is a protein having immunogenicity with respect to HTLV-1. The "one or a plurality of" for (b2) is, for example, 1 to 38, 1 to 32, 1 to 25, 1 to 19, 1 to 12, 1 to 6, 1 to 5, 1 to 3, 1 or 2, or 1 in the amino acid sequence of (b1).
In the case of the protein (b3), the "identity", for example, should be a range in which (b3) is a peptide having immunogenicity with respect to HTLV-1. The "identity" of (b3) is, for example, at least 70%, at least 75%, at least 80%, at least 85%, at least 90%, at least 95%, at least 96%, at least 97%, at least 98%, or at least 99% relative to the amino acid sequence of (b1).

(c) Gag p24 or an immunogenic fragment thereof

Gag p24 can be exemplified by the following proteins (c1), (c2), and (c3).

(c1) a protein composed of the amino acid sequence of SEQ ID NO: 14
(c2) a protein composed of an amino acid sequence in which one or a plurality of amino acids are deleted, inserted, substituted, or added in the amino acid sequence of SEQ ID NO: 14
(c3) a protein composed of an amino acid sequence having an at least 80% identity with the amino acid sequence of SEQ ID NO: 14

Amino acid sequence (SEQ ID NO: 14) of Gag p24

PVMHPHGAPPNHRPWQMKDLQAIKQEVSQAAPGSPQFMQTIRLAVQQFDPTAKDL
QDLLQYLCSSLVASLHHQQLDSLISEAETRGITGYNPLAGPLRVQANNPQQQGLRRE
YQQLWLAAFAALPGSAKDPSWASILQGLEEPYHAFVERLNIALDNGLPEGTPKDPIL
RSLAYSNANKECQKLLQARGHTNSPLGDMLRACQTWTPKDKTKVL

The "one or a plurality of" in the case of the protein (c2), for example, should be a range in which (c2) is a protein having immunogenicity with respect to HTLV-1. The "one or a plurality of" for (c2) is, for example, 1 to 64, 1 to 53, 1 to 42, 1 to 32, 1 to 21, 1 to 10, 1 to 8, 1 to 6, 1 to 4, 1 or 2, or 1 in the amino acid sequence of (c1).
In the case of the protein (c3), the "identity", for example, should be a range in which (c3) is a peptide having immunogenicity with respect to HTLV-1. The "identity" of (c3) is, for example, at least 70%, at least 75%, at least 80%, at least 85%, at least 90%, at least 95%, at least 96%, at least 97%, at least 98%, or at least 99% relative to the amino acid sequence of (c1).
The immunogenic fragments of p15, p19, and p24, for example, should be a range in which the corresponding protein is a protein having immunogenicity with respect to HTLV-1. The immunogenic fragments of p15 and p19 may be, for example, a freely selected protein fragment of these proteins having a length of 5, 9, 10, 20, 30, 40, 50, 60, 70, 80, 90, 100, or more than 100 amino acids.
The lipid complex of the present disclosure, for example, may comprise nucleic acid that comprises a polynucleotide encoding any one of the following, or may comprise nucleic acid that comprises a polynucleotide encoding two of the following, or may comprise nucleic acid that comprises a polynucleotide encoding all of the following: Gag p15 or an immunogenic fragment thereof, Gag p19 or an immunogenic fragment thereof, and Gag p24 or an immunogenic fragment

thereof. The lipid complex of the present disclosure, for example, preferably comprises nucleic that comprises a polynucleotide that encodes Gag p15 or an immunogenic fragment thereof, plus Gag p19 or an immunogenic fragment thereof, plus Gag p24 or an immunogenic fragment thereof. By having such a construction, the lipid complex, for example, can efficiently induce an immune response to HTLV-1.

(d) Gag or an immunogenic fragment thereof

Gag p15, Gag p19, and Gag p24 are provided by the processing of Gag protein. As a consequence, the Gag protein or an immunogenic fragment thereof, for example, can also refer to precursors or precursor proteins for Gag p15 or an immunogenic fragment thereof, Gag p19 or an immunogenic fragment thereof, and/or Gag p24 or an immunogenic fragment thereof.

In some embodiments, the lipid membrane complex may encapsulate nucleic acid that comprises a polynucleotide that encodes, for example, Gag protein or an immunogenic fragment thereof.

The aforementioned Gag (unprocessed protein) can be exemplified by the following proteins (d1), (d2), and (d3).

(d1) a protein composed of the amino acid sequence of SEQ ID NO: 1
(d2) a protein composed of an amino acid sequence in which one or a plurality of amino acids are deleted, inserted, substituted, or added in the amino acid sequence of SEQ ID NO: 1
(d3) a protein composed of an amino acid sequence having an at least 80% identity with the amino acid sequence of SEQ ID NO: 1

The "one or a plurality of" in the case of the protein (d2), for example, should be a range in which (d2) is a protein having immunogenicity with respect to HTLV-1. The "one or a plurality of" for (d2) is, for example, 1 to 128, 1 to 107, 1 to 85, 1 to 64, 1 to 42, 1 to 31, 1 to 21, 1 to 14, 1 to 12, 1 to 8, 1 to 4, 1 or 2, or 1 in the amino acid sequence of (d1).

In the case of the protein (d3), the "identity", for example, should be a range in which (d3) is a peptide having immunogenicity with respect to HTLV-1. The "identity" of (d3) is, for example, at least 70%, at least 75%, at least 80%, at least 85%, at least 90%, at least 95%, at least 96%, at least 97%, at least 98%, or at least 99% relative to the amino acid sequence of (d1).

An immunogenic fragment of Gag, for example, should be a range in which the corresponding protein is a protein having immunogenicity with respect to HTLV-1. An immunogenic fragment of Gag may be, for example, a freely selected protein fragment of this protein having a length of 5, 9, 10, 20, 30, 40, 50, 60, 70, 80, 90, 100, or more than 100 amino acids.

(A) Polynucleotide encoding Gag p15

The polynucleotide that encodes Gag p15 can be exemplified by polynucleotides selected from the group consisting of the following (A1) to (A6) and (A7):

(A1) a polynucleotide composed of the base sequence with SEQ ID NO: 15;
(A2) a polynucleotide composed of a base sequence in which one or a plurality of bases are deleted, inserted, substituted, and/or added in the base sequence of (A1);
(A3) a polynucleotide composed of a base sequence having an at least 80% identity with any base sequence of (A1);
(A4) a polynucleotide composed of a base sequence that is complementary to a polynucleotide that hybridizes under stringent conditions with a polynucleotide composed of a base sequence according to any of (A1) to (A3);
(A5) a polynucleotide that encodes a protein composed of the amino acid sequence of SEQ ID NO: 11 or SEQ ID NO: 12;
(A6) a polynucleotide that encodes a protein composed of an amino acid sequence in which one or a plurality of amino acids are deleted, inserted, substituted, and/or added in the amino acid sequence of (A5); and
(A7) a polynucleotide that encodes a protein composed of an amino acid sequence having an at least 80% identity with the amino acid sequence of (A5).

The base sequence with SEQ ID NO: 15 of (A1) is given below. The base sequence with SEQ ID NO: 15 is the coding sequence (mRNA) for the Gag p15 composed of the amino acid sequence with SEQ ID NO: 12. The polynucleotide (A1) with SEQ ID NO: 15 can be obtained, for example, from HTLV-1.
Polynucleotide (SEQ ID NO: 15) of (A1)

UGGUGCAGCCCAAGAAGCCUCCUCCUAACCAGCCUUGCUUUCGCUGCGGCAAA

GCUGGACACUGGAGCCGCGAUUGCACACAGCCUCGCCCUCCUCCUGGACCUUG

CCCUCUUUGCCAGGAUCCUACCCACUGGAAGCGCGACUGCCCUCGCCUGAAAC

CUACCAUCCCUGAACCUGAACCUGAAGAAGACGCUCUGCUGCUGGAUCUGCCC

GCUGAUAUCCCCCACCCCAAGAACAGCAUCGGCGGCGAGGUG

When the nucleic acid is DNA, the polynucleotide encoding this Gag p15 can then be provided, for example, by replacing the u in SEQ ID NO: 15 with t.

For (A2), the "one or a plurality of", for example, should be a range in which the protein encoded by the polynucleotide of (A2) has immunogenicity with respect to HTLV-1. The "one or a plurality of" for (A2) is, for example, 1 to 94, 1 to 78, 1 to 76, 1 to 63, 1 to 51, 1 to 47, 1 to 31, 1 to 25, 1 to 15, 1 to 12, 1 to 10, 1 to 9, 1 to 7, 1 to 6, 1 to 5, 1 or 2, or 1 in the base sequence of (A1).

For (A3), the "identity", for example, should be a range in which the protein encoded by the polynucleotide of (A3) has immunogenicity with respect to HTLV-1. The "identity" of (A3) is, for example, at least 70%, at least 75%, at least 80%, at least 85%, at least 90%, at least 95%, at least 96%, at least 97%, at least 98%, or at least 99% relative to the base sequence of (A1).

For (A4), the "polynucleotide that hybridizes", for example, is a polynucleotide that is fully or partially complementary to the polynucleotide of (A1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted.

For (A4), the "stringent conditions" may be, for example, any of low stringent conditions, medium stringent conditions, or high stringent conditions. The "low stringent conditions" are, for example, 5×SSC, 5×Denhardt's solution, 0.5% SDS, 50% formamide, and 32°C. The "medium stringent conditions" are, for example, 5×SSC, 5×Denhardt's solution, 0.5% SDS, 50% formamide, and 42°C. The "high stringent conditions" are, for example, 5×SSC, 5×Denhardt's solution, 0.5% SDS, 50% formamide, and 50°C. The individual skilled in the art can establish the degree of stringency, for example, by the appropriate selection of conditions such as temperature, salt concentration, probe concentration and length, ionic strength, and time. The conditions described in, for example, "Molecular Cloning: A Laboratory Manual $2^{nd}$ Ed.", edited by Sambrook et al. [Cold Spring Harbor Laboratory Press (1989)], can also be adopted for the "stringent conditions".

The polynucleotide of (A5) can be established, for example, by conversion to the corresponding codons based on the amino acid sequence of SEQ ID NO: 11 or 12.

For (A6), the "one or a plurality of", for example, should be a range in which the protein encoded by the polynucleotide of (A6) has immunogenicity with respect to HTLV-1. The "one or a plurality of" for (A6) is, for example, 1 to 31, 1 to 26, 1 to 25, 1 to 21, 1 to 15, 1 to 12, 1 to 10, 1 to 8, 1 to 5, 1 to 4, 1 to 3, 1 or 2, or 1 in the amino acid sequence of (A5).

For (A7), the "identity", for example, should be a range in which the protein encoded by the polynucleotide of (A7) has immunogenicity with respect to HTLV-1. The "identity" of (A7) is, for example, at least 70%, at least 75%, at least 80%, at least 85%, at least 90%, at least 95%, at least 96%, at least 97%, at least 98%, or at least 99% relative to the amino acid sequence of (A5).

(B) Polynucleotide encoding Gag p19

The polynucleotide that encodes Gag p19 can be exemplified by polynucleotides selected from the group consisting of the following (B1) to (B6) and (B7):

(B1) a polynucleotide composed of the base sequence with SEQ ID NO: 16;
(B2) a polynucleotide composed of a base sequence in which one or a plurality of bases are deleted, inserted, substituted, and/or added in the base sequence of (B1);
(B3) a polynucleotide composed of a base sequence having an at least 80% identity with any base sequence of (B1);
(B4) a polynucleotide composed of a base sequence that is complementary to a polynucleotide that hybridizes under stringent conditions with a polynucleotide composed of a base sequence according to any of (B1) to (B3);
(B5) a polynucleotide that encodes a protein composed of the amino acid sequence of SEQ ID NO: 13;

(B6) a polynucleotide that encodes a protein composed of an amino acid sequence in which one or a plurality of amino acids are deleted, inserted, substituted, and/or added in the amino acid sequence of (B5); and
(B7) a polynucleotide that encodes a protein composed of an amino acid sequence having an at least 80% identity with the amino acid sequence of (B5).

The base sequence with SEQ ID NO: 16 of (B1) is given below. The base sequence with SEQ ID NO: 16 is the coding sequence (mRNA) for Gag p19 composed of the amino acid sequence with SEQ ID NO: 13. The polynucleotide (B1) with SEQ ID NO: 16 can be obtained, for example, from HTLV-1.
Polynucleotide (SEQ ID NO: 16) of (B1)

GGCCAGAUCUUCUCUCGCUCUGCUUCUCCUAUUCCUCGCCCUCCUCGCGGACU

UGCUGCUCACCACUGGCUUAACUUCCUGCAGGCUGCUUACCGCCUGGAACCUG

GCCCUAGCAGCUACGACUUCCACCAGCUGAAGAAGUUCCUGAAGAUCGCCCUG

GAGACCCCCGUGUGGAUCUGCCCCAUCAACUACAGCCUGCUGGCUAGCCUGCU

GCCUAAAGGCUACCCUGGCCGCGUGAACGAGAUCCUGCACAUCCUGAUCCAGA

CCCAGGCCCAGAUCCCUUCUCGCCCUGCUCCUCCUCCUCCUUCUUCUCCUACCC

ACGAUCCCCCCGAUAGCGAUCCCCAGAUCCCCCCUCCUUACGUGGAACCUACC

GCUCCUCAGGUGCUG

When the nucleic acid is DNA, the polynucleotide encoding this Gag p19 can then be provided, for example, by replacing the u in SEQ ID NO: 16 with t.
For (B2), the "one or a plurality of", for example, should be a range in which the protein encoded by the polynucleotide of (B2) has immunogenicity with respect to HTLV-1. The "one or a plurality of" for (B2) is, for example, 1 to 116, 1 to 96, 1 to 94, 1 to 77, 1 to 58, 1 to 38, 1 to 19, 1 to 15, 1 to 11, 1 to 7, 1 to 3, 1 or 2, or 1 in the base sequence of (B1).
For (B3), the "identity", for example, should be a range in which the protein encoded by the polynucleotide of (B3) has immunogenicity with respect to HTLV-1. The "identity" of (B3) is, for example, at least 70%, at least 75%, at least 80%, at least 85%, at least 90%, at least 95%, at least 96%, at least 97%, at least 98%, or at least 99% relative to the base sequence of (B1).
For (B4), the "polynucleotide that hybridizes", for example, is a polynucleotide that is fully or partially complementary to the polynucleotide of (B1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for these stringent conditions for (B4).
The polynucleotide of (B5) can be established, for example, by conversion to the corresponding codons based on the amino acid sequence of SEQ ID NO: 13.
For (B6), the "one or a plurality of", for example, should be a range in which the protein encoded by the polynucleotide of (B6) has immunogenicity with respect to HTLV-1. The "one or a plurality of" for (B6) is, for example, 1 to 38, 1 to 32, 1 to 31, 1 to 25, 1 to 19, 1 to 12, 1 to 6, 1 to 5, 1 to 3, 1 or 2, or 1 in the amino acid sequence of (B5).
For (B7), the "identity", for example, should be a range in which the protein encoded by the polynucleotide of (B7) has immunogenicity with respect to HTLV-1. The "identity" of (B7) is, for example, at least 70%, at least 75%, at least 80%, at least 85%, at least 90%, at least 95%, at least 96%, at least 97%, at least 98%, or at least 99% relative to the amino acid sequence of (B5).

(C) Polynucleotide encoding Gag p24

The polynucleotide that encodes Gag p24 can be exemplified by polynucleotides selected from the group consisting of the following (C1) to (C6) and (C7):

(C1) a polynucleotide composed of the base sequence with SEQ ID NO: 17;
(C2) a polynucleotide composed of a base sequence in which one or a plurality of bases are deleted, inserted, substituted, and/or added in the base sequence of (C1);
(C3) a polynucleotide composed of a base sequence having an at least 80% identity with any base sequence of (C1);
(C4) a polynucleotide composed of a base sequence that is complementary to a polynucleotide that hybridizes under stringent conditions with a polynucleotide composed of a base sequence according to any of (C1) to (C3);
(C5) a polynucleotide that encodes a protein composed of the amino acid sequence of SEQ ID NO: 14;
(C6) a polynucleotide that encodes a protein composed of an amino acid sequence in which one or a plurality of amino acids are deleted, inserted, substituted, and/or added in the amino acid sequence of (C5); and
(C7) a polynucleotide that encodes a protein composed of an amino acid sequence having an at least 80% identity with the amino acid sequence of (C5).

The base sequence with SEQ ID NO: 17 of (C1) is given below. The base sequence with SEQ ID NO: 17 is the coding sequence (mRNA) for Gag p24 composed of the amino acid sequence with SEQ ID NO: 14. The polynucleotide (C1) with SEQ ID NO: 17 can be obtained, for example, from HTLV-1.
Polynucleotide (SEQ ID NO: 17) of (C1)

CCUGUGAUGCACCCUCACGGCGCUCCUCCUAAUCACCGCCCUUGGCAGAUGAA

GGACCUGCAGGCCAUCAAACAGGAAGUGAGCCAGGCUGCUCCCGGCAGCCCCC

AGUUUAUGCAGACCAUCCGCCUGGCUGUGCAGCAGUUCGAUCCCACCGCUAAA

GAUCUGCAGGACCUGCUGCAGUACCUGUGCAGCAGCCUGGUGGCUAGCCUGCA

CCACCAGCAGCUGGAUAGCCUGAUCAGCGAAGCCGAAACCCGCGGCAUCACCG

GCUACAAUCCUCUGGCUGGCCCUCUGCGCGUGCAGGCUAACAACCCUCAGCAG

CAGGGACUGCGCCGCGAAUACCAGCAGCUGUGGCUGGCUGCUUUUGCUGCUCU

GCCCGGCAGCGCUAAAGAUCCCAGCUGGGCUAGCAUCCUGCAGGGCCUGGAAG

AACCCUACCACGCCUUCGUGGAGCGCCUGAACAUCGCCCUGGACAACGGCCUG

CCUGAAGGCACCCCUAAAGAUCCUAUCCUGCGCAGCCUGGCCUACAGCAACGC

CAACAAGGAGUGCCAGAAGCUGCUGCAGGCUCGCGGACACACCAAUAGCCCUC

UGGGAGAUAUGCUGCGCGCUUGCCAGACCUGGACCCCCAAGGACAAGACCAAG

GUGCUGG

When the nucleic acid is DNA, the polynucleotide encoding this Gag p24 can then be provided, for example, by replacing the u in SEQ ID NO: 17 with t.
For (C2), the "one or a plurality of", for example, should be a range in which the protein encoded by the polynucleotide of (C2) has immunogenicity with respect to HTLV-1. The "one or a plurality of" for (C2) is, for example, 1 to 192, 1 to 160, 1 to 128, 1 to 96, 1 to 94, 1 to 64, 1 to 32, 1 to 25, 1 to 19, 1 to 12, 1 to 6, or 1 or 2 in the base sequence of (C1).
For (C3), the "identity", for example, should be a range in which the protein encoded by the polynucleotide of (C3) has immunogenicity with respect to HTLV-1. The "identity" of (C3) is, for example, at least 70%, at least 75%, at least 80%, at least 85%, at least 90%, at least 95%, at least 96%, at least 97%, at least 98%, or at least 99% relative to the base sequence of (C1).
For (C4), the "polynucleotide that hybridizes", for example, is a polynucleotide that is fully or partially complementary to the polynucleotide of (C1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for these stringent conditions for (C4).

The polynucleotide of (C5) can be established, for example, by conversion to the corresponding codons based on the amino acid sequence of SEQ ID NO: 14.

For (C6), the "one or a plurality of", for example, should be a range in which the protein encoded by the polynucleotide of (C6) has immunogenicity with respect to HTLV-1. The "one or a plurality of" for (C6) is, for example, 1 to 64, 1 to 53, 1 to 42, 1 to 32, 1 to 31, 1 to 21, 1 to 10, 1 to 8, 1 to 6, 1 to 4, 1 or 2, or 1 in the amino acid sequence of (C5).

For (C7), the "identity", for example, should be a range in which the protein encoded by the polynucleotide of (C7) has immunogenicity with respect to HTLV-1. The "identity" of (C7) is, for example, at least 70%, at least 75%, at least 80%, at least 85%, at least 90%, at least 95%, at least 96%, at least 97%, at least 98%, or at least 99% relative to the amino acid sequence of (C5).

(Polynucleotides encoding immunogenic fragments of Gag p15, p19, or p24)

Polynucleotides encoding immunogenic fragments of Gag p15, p19, or p24, for example, should be in a range in which the protein encoded by the particular polynucleotide has immunogenicity with respect to HTLV-1.

Polynucleotide comprised in the nucleic acid encapsulated in the lipid complex of the present disclosure in particular preferably encodes at least one of the amino acid sequence at positions 1 to 59 (p19-1 in the examples provided below), the amino acid sequence at positions 52 to 109 (p19-2 in the examples provided below), or the amino acid sequence at positions 102 to 130 (p19-3 in the examples provided below) of SEQ ID NO: 1, and more preferably encodes at least one of the amino acid sequence at positions 1 to 59 (p19-1 in the examples provided below) or the amino acid sequence at positions 52 to 109 (p19-2 in the examples provided below) of SEQ ID NO: 1.

Preferably polynucleotide comprised in the nucleic acid encapsulated in the lipid complex of the present disclosure in particular encodes at least one of the amino acid sequence at positions 1 to 59 (p19-1 in the examples provided below), the amino acid sequence at positions 52 to 109 (p19-2 in the examples provided below), or the amino acid sequence at positions 102 to 130 (p19-3 in the examples provided below) of SEQ ID NO: 1, with the region outside this polynucleotide being composed of the amino acid sequence outside this polynucleotide in SEQ ID NO: 1 or being composed of an amino acid sequence having at least 80%, at least 85%, at least 90%, at least 95%, at least 96%, at least 97%, at least 98%, or at least 99% homology with the amino acid sequence outside this polynucleotide in SEQ ID NO: 1. More preferably, it encodes at least one of the amino acid sequence at positions 1 to 59 (p19-1 in the examples provided below) or the amino acid sequence at positions 52 to 109 (p19-2 in the examples provided below) in SEQ ID NO: 1, with the region outside this polynucleotide being composed of the amino acid sequence outside this polynucleotide in SEQ ID NO: 1 or being composed of an amino acid sequence having at least 80%, at least 85%, at least 90%, at least 95%, at least 96%, at least 97%, at least 98%, or at least 99% homology with the amino acid sequence outside this polynucleotide in SEQ ID NO: 1.

The nucleic acid encapsulated in the lipid complex of the present disclosure in particular is preferably a polynucleotide that encodes at least one of the amino acid sequence at positions 1 to 59 (p19-1 in the examples provided below), the amino acid sequence at positions 52 to 109 (p19-2 in the examples provided below), or the amino acid sequence at positions 102 to 130 (p19-3 in the examples provided below) of SEQ ID NO: 1, and is more preferably a polynucleotide that encodes at least one of the amino acid sequence at positions 1 to 59 (p19-1 in the examples provided below) or the amino acid sequence at positions 52 to 109 (p19-2 in the examples provided below) of SEQ ID NO: 1.

The polynucleotide comprised in the nucleic acid encapsulated in the lipid complex of the present disclosure in particular preferably comprises at least one base sequence of the base sequence at positions 1 to 177 (polynucleotide encoding p19-1 in the examples provided below) of SEQ ID NO: 3, the base sequence at positions 154 to 327 (polynucleotide encoding p19-2 in the examples provided below) of SEQ ID NO: 3, or the base sequence at positions 304 to 390 (polynucleotide encoding p19-3 in the examples provided below) of SEQ ID NO: 3.

The polynucleotide comprised in the nucleic acid encapsulated in the lipid complex of the present disclosure more preferably comprises at least one base sequence of the base sequence at positions 1 to 177 (polynucleotide encoding p19-1 in the examples provided below) of SEQ ID NO: 3 or the base sequence at positions 154 to 327 (polynucleotide encoding p19-2 in the examples provided below) of SEQ ID NO: 3.

The polynucleotide comprised in the nucleic acid encapsulated in the lipid complex of the present disclosure in particular is preferably a polynucleotide composed of at least one base sequence of the base sequence at positions 1 to 177 (polynucleotide encoding p19-1 in the examples provided below) of SEQ ID NO: 3, the base sequence at positions 154 to 327 (polynucleotide encoding p19-2 in the examples provided below) of SEQ ID NO: 3, or the base sequence at positions 304 to 390 (polynucleotide encoding p19-3 in the examples provided below) of SEQ ID NO: 3, with the region outside this polynucleotide in the nucleic acid being the base sequence of SEQ ID NO: 3 outside this polynucleotide or

being a base sequence having at least 80%, at least 85%, at least 90%, at least 95%, at least 96%, at least 97%, at least 98%, or at least 99% identity with the base sequence of SEQ ID NO: 3 outside this polynucleotide.

The polynucleotide comprised in the nucleic acid encapsulated in the lipid complex of the present disclosure is more preferably at least one base sequence of the base sequence at positions 1 to 177 (polynucleotide encoding p19-1 in the examples provided below) of SEQ ID NO: 3 or the base sequence at positions 154 to 327 (polynucleotide encoding p19-2 in the examples provided below) of SEQ ID NO: 3, with the region outside this polynucleotide in the nucleic acid being the base sequence of SEQ ID NO: 3 outside this polynucleotide or being a base sequence having at least 80%, at least 85%, at least 90%, at least 95%, at least 96%, at least 97%, at least 98%, or at least 99% identity with the base sequence of SEQ ID NO: 3 outside this polynucleotide.

Preferably the polynucleotide comprised in the nucleic acid encapsulated in the lipid complex of the present disclosure encodes at least one of the amino acid sequence at positions 1 to 59 (p19-1 in the examples provided below) of SEQ ID NO: 1, the amino acid sequence at positions 52 to 109 (p19-2 in the examples provided below) of SEQ ID NO: 1, or the amino acid sequence at positions 102 to 130 (p19-3 in the examples provided below) of SEQ ID NO: 1, with the region outside this polynucleotide in the nucleic acid being composed of a base sequence that encodes the amino acid sequence of SEQ ID NO: 1 outside the aforementioned amino acid sequence or that encodes an amino acid sequence having at least 80%, at least 85%, at least 90%, at least 95%, at least 96%, at least 97%, at least 98%, or at least 99% homology with the amino acid sequence of SEQ ID NO: 1 outside the aforementioned amino acid sequence.

More preferably, the polynucleotide comprised in the nucleic acid encapsulated in the lipid complex of the present disclosure encodes at least one of the amino acid sequence at positions 1 to 59 (p19-1 in the examples provided below) of SEQ ID NO: 1 or the amino acid sequence at positions 52 to 109 (p19-2 in the examples provided below) in SEQ ID NO: 1, with the region outside this polynucleotide in the nucleic acid being composed of a base sequence that encodes the amino acid sequence of SEQ ID NO: 1 outside the aforementioned amino acid sequence or that encodes an amino acid sequence having at least 80%, at least 85%, at least 90%, at least 95%, at least 96%, at least 97%, at least 98%, or at least 99% homology with the amino acid sequence of SEQ ID NO: 1 outside the aforementioned amino acid sequence.

In some embodiments, the lipid complex of the present disclosure, for example, may comprise a nucleic acid comprising a polynucleotide encoding any one of the following, or may comprise a nucleic acid comprising a polynucleotide encoding two of the following, or may comprise a nucleic acid comprising a polynucleotide encoding all of the following: Gag p15 or an immunogenic fragment thereof, Gag p19 or an immunogenic fragment thereof, and Gag p24 or an immunogenic fragment thereof.

In a specific embodiment, the lipid complex of the present disclosure, for example, preferably comprises a nucleic that comprises a polynucleotide that encodes Gag p15 or an immunogenic fragment thereof, plus Gag p19 or an immunogenic fragment thereof, plus Gag p24 or an immunogenic fragment thereof. By having such a construction, the lipid complex of the present disclosure, for example, can efficiently induce an immune response to HTLV-1.

(D) Polynucleotide encoding Gag

A nucleic acid encoding Gag (unprocessed protein) can be exemplified by nucleic acid that comprises a polynucleotide selected from the group consisting of the following (D1) to (D6) and (D7):

(D1) a polynucleotide composed of the base sequence with SEQ ID NO: 2 or 3;
(D2) a polynucleotide composed of a base sequence in which one or a plurality of bases are deleted, inserted, substituted, and/or added in the base sequence of (D1);
(D3) a polynucleotide composed of a base sequence having an at least 80% identity with either base sequence of (D1);
(D4) a polynucleotide composed of a base sequence that is complementary to a polynucleotide that hybridizes under stringent conditions with a polynucleotide composed of a base sequence according to any of (D1) to (D3);
(D5) a polynucleotide that encodes a protein composed of the amino acid sequence of SEQ ID NO: 1;
(D6) a polynucleotide that encodes a protein composed of an amino acid sequence in which one or a plurality of amino acids are deleted, inserted, substituted, and/or added in the amino acid sequence of (D5); and
(D7) a polynucleotide that encodes a protein composed of an amino acid sequence having an at least 80% identity with the amino acid sequence of (D5).

The base sequences with SEQ ID NO: 2 and SEQ ID NO: 3 of (D1) are each given below. The base sequences with SEQ ID NO: 2 and SEQ ID NO: 3 are the coding sequences (mRNA) for unprocessed Gag composed of the amino acid sequence with SEQ ID NO: 1. Polynucleotides (D1) with SEQ ID NO: 2 and SEQ ID NO: 3 can be obtained, for example, from HTLV-1.

Polynucleotide (SEQ ID NO: 2) of (D1)

AGGAAAUAAGAGAGAAAAGAAGAGUAAGAAGAAAUAUAAGAGCCACCAUGGG
CCAGAUCUUCUCUCGCUCUGCUUCUCCUAUUCCUCGCCCUCCUCGCGGACUUG
CUGCUCACCACUGGCUUAACUUCCUGCAGGCUGCUUACCGCCUGGAACCUGGC
CCUAGCAGCUACGACUUCCACCAGCUGAAGAAGUUCCUGAAGAUCGCCCUGGA
GACCCCCGUGUGGAUCUGCCCCAUCAACUACAGCCUGCUGGCUAGCCUGCUGC
CUAAAGGCUACCCUGGCCGCGUGAACGAGAUCCUGCACAUCCUGAUCCAGACC
CAGGCCCAGAUCCCUUCUCGCCCUGCUCCUCCUCCUCCUUCUUCUCCUACCCAC
GAUCCCCCCGAUAGCGAUCCCCAGAUCCCCCCUCCUUACGUGGAACCUACCGC
UCCUCAGGUGCUGCCUGUGAUGCACCCUCACGGCGCUCCUCCUAAUCACCGCC
CUUGGCAGAUGAAGGACCUGCAGGCCAUCAAACAGGAAGUGAGCCAGGCUGCU
CCCGGCAGCCCCCAGUUUAUGCAGACCAUCCGCCUGGCUGUGCAGCAGUUCGA
UCCCACCGCUAAAGAUCUGCAGGACCUGCUGCAGUACCUGUGCAGCAGCCUGG
UGGCUAGCCUGCACCACCAGCAGCUGGAUAGCCUGAUCAGCGAAGCCGAAACC
CGCGGCAUCACCGGCUACAAUCCUCUGGCUGGCCCUCUGCGCGUGCAGGCUAA
CAACCCUCAGCAGCAGGGACUGCGCCGCGAAUACCAGCAGCUGUGGCUGGCUG
CUUUUGCUGCUCUGCCCGGCAGCGCUAAAGAUCCCAGCUGGGCUAGCAUCCUG
CAGGGCCUGGAAGAACCCUACCACGCCUUCGUGGAGCGCCUGAACAUCGCCCU

GGACAACGGCCUGCCUGAAGGCACCCCUAAAGAUCCUAUCCUGCGCAGCCUGG
CCUACAGCAACGCCAACAAGGAGUGCCAGAAGCUGCUGCAGGCUCGCGGACAC
ACCAAUAGCCCUCUGGGAGAUAUGCUGCGCUUGCCAGACCUGGACCCCCAA
GGACAAGACCAAGGUGCUGGUGGUGCAGCCCAAGAAGCCUCCUCCUAACCAGC
CUUGCUUUCGCUGCGGCAAAGCUGGACACUGGAGCCGCGAUUGCACACAGCCU
CGCCCUCCUCCUGGACCUUGCCCUCUUUGCCAGGAUCCUACCCACUGGAAGCG
CGACUGCCCUCGCCUGAAACCUACCAUCCCUGAACCUGAACCUGAAGAAGACG
CUCUGCUGCUGGAUCUGCCCGCUGAUAUCCCCCACCCCAAGAACAGCAUCGGC
GGCGAGGUGUGAUAAGCUGCCUUCUGCGGGGCUUGCCUUCUGGCCAUGCCCUU
CUUCUCUCCCUUGCACCUGUACCUCUUGGUCUUUGAAUAAAGCCUGAGUAGGA
AGGCGGAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAA
AAAAAAAAAAAAAAAAAAAAAA

Polynucleotide (SEQ ID NO: 3) of (D1)

AUGGGCCAGAUCUUCUCUCGCUCUGCUUCUCCUAUUCCUCGCCCUCCUCGCGG
ACUUGCUGCUCACCACUGGCUUAACUUCCUGCAGGCUGCUUACCGCCUGGAAC
CUGGCCCUAGCAGCUACGACUUCCACCAGCUGAAGAAGUUCCUGAAGAUCGCC
CUGGAGACCCCCGUGUGGAUCUGCCCCAUCAACUACAGCCUGCUGGCUAGCCU
GCUGCCUAAAGGCUACCCUGGCCGCGUGAACGAGAUCCUGCACAUCCUGAUCC
AGACCCAGGCCCAGAUCCCUUCUCGCCCUGCUCCUCCUCCUCCUUCUUCUCCUA
CCCACGAUCCCCCCGAUAGCGAUCCCCAGAUCCCCCCUCCUUACGUGGAACCUA
CCGCUCCUCAGGUGCUGCCUGUGAUGCACCCUCACGGCGCUCCUCCUAAUCAC
CGCCCUUGGCAGAUGAAGGACCUGCAGGCCAUCAAACAGGAAGUGAGCCAGGC
UGCUCCCGGCAGCCCCCAGUUUAUGCAGACCAUCCGCCUGGCUGUGCAGCAGU

UCGAUCCCACCGCUAAAGAUCUGCAGGACCUGCUGCAGUACCUGUGCAGCAGC
CUGGUGGCUAGCCUGCACCACCAGCAGCUGGAUAGCCUGAUCAGCGAAGCCGA
AACCCGCGGCAUCACCGGCUACAAUCCUCUGGCUGGCCCUCUGCGCGUGCAGG
CUAACAACCCUCAGCAGCAGGGACUGCGCCGCGAAUACCAGCAGCUGUGGCUG
GCUGCUUUUGCUGCUCUGCCCGGCAGCGCUAAAGAUCCCAGCUGGGCUAGCAU
CCUGCAGGGCCUGGAAGAACCCUACCACGCCUUCGUGGAGCGCCUGAACAUCG
CCCUGGACAACGGCCUGCCUGAAGGCACCCCUAAAGAUCCUAUCCUGCGCAGC
CUGGCCUACAGCAACGCCAACAAGGAGUGCCAGAAGCUGCUGCAGGCUCGCGG
ACACACCAAUAGCCCUCUGGGAGAUAUGCUGCGCGCUUGCCAGACCUGGACCC
CCAAGGACAAGACCAAGGUGCUGGUGGUGCAGCCCAAGAAGCCUCCUCCUAAC
CAGCCUUGCUUUCGCUGCGGCAAAGCUGGACACUGGAGCCGCGAUUGCACACA
GCCUCGCCCUCCUCCUGGACCUUGCCCUCUUUGCCAGGAUCCUACCCACUGGA
AGCGCGACUGCCCUCGCCUGAAACCUACCAUCCCUGAACCUGAACCUGAAGAA
GACGCUCUGCUGCUGGAUCUGCCCGCUGAUAUCCCCCACCCCAAGAACAGCAU
CGGCGGCGAGGUG

For (D2), the "one or a plurality of", for example, should be a range in which the protein encoded by the polynucleotide of (D2) has immunogenicity with respect to HTLV-1. The "one or a plurality of" for (D2) is, for example, 1 to 387, 1 to 322, 1 to 258, 1 to 192, 1 to 129, 1 to 94, 1 to 64, 1 to 51, 1 to 42, 1 to 38, 1 to 25, 1 to 12, 1 to 6, 1 to 3, 1 or 2, or 1 in the base sequence of (D1).

For (D3), the "identity", for example, should be a range in which the protein encoded by the polynucleotide of (D3) has immunogenicity with respect to HTLV-1. The "identity" of (D3) is, for example, at least 70%, at least 75%, at least 80%, at least 85%, at least 90%, at least 95%, at least 96%, at least 97%, at least 98%, or at least 99% relative to the base sequence of (D1).

For (D4), the "polynucleotide that hybridizes", for example, is a polynucleotide that is fully or partially complementary to the polynucleotide of (D1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for these stringent conditions for (D4).

The polynucleotide of (D5) can be established, for example, by conversion to the corresponding codons based on the amino acid sequence of SEQ ID NO: 1.

For (D6), the "one or a plurality of", for example, should be a range in which the protein encoded by the polynucleotide of (D6) has immunogenicity with respect to HTLV-1. The "one or a plurality of" for (D6) is, for example, 1 to 128, 1 to 107, 1 to 85, 1 to 64, 1 to 42, 1 to 21, 1 to 14, 1 to 12, 1 to 8, 1 to 4, 1 or 2, or 1 in the amino acid sequence of (D5).

For (D7), the "identity", for example, should be a range in which the protein encoded by the polynucleotide of (D7) has immunogenicity with respect to HTLV-1. The "identity" of (D7) is, for example, at least 70%, at least 75%, at least 80%, at least 85%, at least 90%, at least 95%, at least 96%, at least 97%, at least 98%, or at least 99% relative to the amino acid sequence of (D5).

(Polynucleotides encoding immunogenic fragments of Gag)

In the present disclosure, polynucleotides encoding immunogenic fragments of Gag, for example, should be in a range in which the protein encoded by the particular polynucleotide has immunogenicity with respect to HTLV-1.

(II) Nucleic acid comprising a polynucleotide that encodes an immunogenic fragment of HTLV-1 antigenic Tax

The nucleic acid can be a nucleic acid that comprises a polynucleotide that encodes an immunogenic fragment of HTLV-1 antigenic Tax protein (Tax). The polynucleotide that encodes an immunogenic fragment of Tax is a polynucleotide that necessarily comprises a region that encodes an immunogenic fragment of Tax. This immunogenic fragment of Tax may be any range that is a protein having immunogenicity with respect to HTLV-1.

The nucleic acid may be a nucleic acid that comprises a polynucleotide that encodes Tax or an immunogenic fragment thereof. In addition, the nucleic acid may be a polynucleotide that encodes Tax or an immunogenic fragment thereof. For example, when the nucleic acid is mRNA, the nucleic acid may comprise a 5' cap structure, a 5'-UTR, a 3'-UTR, a poly-A sequence, and so forth, in addition to a polynucleotide that encodes an immunogenic fragment of Tax.

The amino acid length of the immunogenic fragment of Tax may be a length of at least 5, 9, 10, 20, 30, 40, 50, 60, 70, 80, 90, 100, or more than 100 amino acids.

In some embodiments, the polynucleotide encoding an immunogenic fragment of Tax comprises a polynucleotide that encodes Tax or an immunogenic fragment thereof. Thus, in some embodiments, the nucleic acid is a nucleic acid comprising a polynucleotide that encodes Tax or an immunogenic fragment thereof.

(e) a Tax or an immunogenic fragment thereof

Tax can be exemplified by the following proteins (e1), (e2), and (e3).

(e1) a protein composed of the amino acid sequence of SEQ ID NO: 4
(e2) a protein composed of an amino acid sequence in which one or a plurality of amino acids are deleted, inserted, substituted, or added in the amino acid sequence of SEQ ID NO: 4
(e3) a protein composed of an amino acid sequence having an at least 80% identity with the amino acid sequence of SEQ ID NO: 4

The "one or a plurality of" in the case of the protein (e2), for example, should be a range in which (e2) is a protein having immunogenicity with respect to HTLV-1. The "one or a plurality of" for (e2) is, for example, 1 to 105, 1 to 88, 1 to 70, 1 to 52, 1 to 35, 1 to 17, 1 to 14, 1 to 10, 1 to 7, 1 to 3, 1 or 2, or 1 in the amino acid sequence of (e1).

In the case of the protein (e3), the "identity", for example, should be a range in which (e3) is a peptide having immunogenicity with respect to HTLV-1. The "identity" of (e3) is, for example, at least 70%, at least 75%, at least 80%, at least 85%, at least 90%, at least 95%, at least 96%, at least 97%, at least 98%, or at least 99% relative to the amino acid sequence of (e1).

In the present disclosure, an immunogenic fragment of Tax, for example, should be a range in which the corresponding protein is a protein having immunogenicity with respect to HTLV-1. An immunogenic fragment of Tax may be, for example, a freely selected protein fragment of this protein having a length of 5, 9, 10, 20, 30, 40, 50, 60, 70, 80, 90, 100, or more than 100 amino acids.

(E) Polynucleotide encoding Tax

The polynucleotide that encodes Tax can be exemplified by polynucleotides selected from the group consisting of the following (E1) to (E6) and (E7):

(E1) a polynucleotide composed of the base sequence with SEQ ID NO: 5 or 6;
(E2) a polynucleotide composed of a base sequence in which one or a plurality of bases are deleted, inserted, substituted, and/or added in the base sequence of (E1);
(E3) a polynucleotide composed of a base sequence having an at least 80% identity with either base sequence of (E1);
(E4) a polynucleotide composed of a base sequence that is complementary to a polynucleotide that hybridizes under stringent conditions with a polynucleotide composed of a base sequence according to any of (E1) to (E3);
(E5) a polynucleotide that encodes a protein composed of the amino acid sequence of SEQ ID NO: 4;
(E6) a polynucleotide that encodes a protein composed of an amino acid sequence in which one or a plurality of amino acids are deleted, inserted, substituted, and/or added in the amino acid sequence of (E5); and
(E7) a polynucleotide that encodes a protein composed of an amino acid sequence having an at least 80% identity with

the amino acid sequence of (E5).

The base sequences with SEQ ID NO: 5 and SEQ ID NO: 6 of (E1) are given below. The base sequences with SEQ ID NO: 5 and SEQ ID NO: 6 are the coding sequences (mRNA) for the Tax composed of the amino acid sequence with SEQ ID NO: 4. The polynucleotides (E1) with SEQ ID NO: 5 and SEQ ID NO: 6 can be obtained, for example, from HTLV-1.

Polynucleotide (SEQ ID NO: 5) of (E1)

AGGAAAUAAGAGAGAAAAGAAGAGUAAGAAGAAAUAUAAGAGCCACCAUGGC

CCACUUCCCCGGCUUUGGCCAGAGCCUGCUGUUUGGCUACCCCGUGUACGUGU

UCGGCGAUUGCGUGCAGGGCGAUUGGUGCCCCAUCUCUGGAGGACUGUGCUCU

GCUCGCCUUCACCGCCACGCACUUCUUGCUACAUGCCCUGAGCACCAGAUCAC

CUGGGAUCCCAUCGAUGGCCGCGUGAUCGGCAGCGCUCUGCAGUUCCUGAUCC

CUCGCCUGCCUAGCUUUCCUACCCAGCGCACCAGCAAAACCCUGAAGGUGCUG

ACCCCUCCUAUCACCCACACCACCCCUAACAUCCCUCCUAGCUUCCUGCAGGCC

AUGCGCAAGUACAGCCCCUUCCGCAACGGCUACAUGGAACCCACCCUGGGCCA

GCACCUGCCUACCCUGAGCUUUCCUGAUCCUGGACUGCGCCCUCAGAAUCUGU

ACACCCUGUGGGGAGGAAGCGUGGUGUGCAUGUACCUGUACCAGCUGAGCCCU

CCUAUCACCUGGCCUCUGCUGCCUCACGUGAUCUUUGCCACCCUGGCCAGCU

GGGCGCUUUUCUGACCAACGUGCCUUACAAGCGCAUCGAGGAGCUGCUGUACA

AGAUCAGCCUGACCACCGGCGCCCUGAUCAUCCUGCCCGAAGAUUGCCUGCCC
ACCACCCUGUUUCAGCCUGCUCGCGCUCCUGUGACACUGACAGCUUGGCAGAA
CGGCCUGCUGCCUUUUCACAGCACCCUGACCACCCCUGGCCUGAUCUGGACCU
UCACCGAUGGCACCCCCAUGAUCAGCGGCCCUUGCCCUAAAGAUGGCCAGCCU
AGCCUGGUGCUGCAGAGCAGCAGCUUCAUCUUCCACAAGUUCCAGACCAAGGC
CUACCACCCCAGCUUCCUGCUGAGCCACGGCCUGAUCCAGUACAGCAGCUUCC
ACAGCCUGCACCUGCUGUUCGAGGAGUACACCAACAUCCCCAUCAGCCUGCUG
UUCAACGAGAAGGAGGCCGACGACAACGACCACGAGCCUCAGAUCAGCCCUGG
CGGCCUGGAACCUCCUAGCGAAAAGCACUUCCGCGAGACCGAGGUGUGAUAAG
CUGCCUUCUGCGGGGCUUGCCUUCUGGCCAUGCCCUUCUUCUCUCCCUUGCAC
CUGUACCUCUUGGUCUUUGAAUAAAGCCUGAGUAGGAAGGCGGAAAAAAAAA
AAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAA
AAAAAAA

Polynucleotide (SEQ ID NO: 6) of (E1)

AUGGCCCACUUCCCCGGCUUUGGCCAGAGCCUGCUGUUUGGCUACCCCGUGUA
CGUGUUCGGCGAUUGCGUGCAGGGCGAUUGGUGCCCCAUCUCUGGAGGACUGU
GCUCUGCUCGCCUUCACCGCCACGCACUUCUUGCUACAUGCCCUGAGCACCAG
AUCACCUGGGAUCCCAUCGAUGGCCGCGUGAUCGGCAGCGCUCUGCAGUUCCU
GAUCCCUCGCCUGCCUAGCUUUCCUACCCAGCGCACCAGCAAAACCCUGAAGG
UGCUGACCCCUCCUAUCACCCACACCACCCCUAACAUCCCUCCUAGCUUCCUGC
AGGCCAUGCGCAAGUACAGCCCCUUCCGCAACGGCUACAUGGAACCCACCCUG
GGCCAGCACCUGCCUACCCUGAGCUUUCCUGAUCCUGGACUGCGCCCUCAGAA
UCUGUACACCCUGUGGGGAGGAAGCGUGGUGUGCAUGUACCUGUACCAGCUG

AGCCCUCCUAUCACCUGGCCUCUGCUGCCUCACGUGAUCUUUUGCCACCCUGG

CCAGCUGGGCGCUUUUCUGACCAACGUGCCUUACAAGCGCAUCGAGGAGCUGC

UGUACAAGAUCAGCCUGACCACCGGCGCCCUGAUCAUCCUGCCCGAAGAUUGC

CUGCCCACCACCCUGUUUCAGCCUGCUCGCGCUCCUGUGACACUGACAGCUUG

GCAGAACGGCCUGCUGCCUUUUCACAGCACCCUGACCACCCCUGGCCUGAUCU

GGACCUUCACCGAUGGCACCCCCAUGAUCAGCGGCCCUUGCCCUAAAGAUGGC

CAGCCUAGCCUGGUGCUGCAGAGCAGCAGCUUCAUCUUCCACAAGUUCCAGAC

CAAGGCCUACCACCCCAGCUUCCUGCUGAGCCACGGCCUGAUCCAGUACAGCA

GCUUCCACAGCCUGCACCUGCUGUUCGAGGAGUACACCAACAUCCCCAUCAGC

CUGCUGUUCAACGAGAAGGAGGCCGACGACAACGACCACGAGCCUCAGAUCAG

CCCUGGCGGCCUGGAACCUCCUAGCGAAAAGCACUUCCGCGAGACCGAGGUG

For (E2), the "one or a plurality of", for example, should be a range in which the protein encoded by the polynucleotide of (E2) has immunogenicity with respect to HTLV-1. The "one or a plurality of" for (E2) is, for example, 1 to 318, 1 to 265, 1 to 212, 1 to 159, 1 to 106, 1 to 53, 1 to 42, 1 to 31, 1 to 21, 1 to 10, 1 to 6, 1 or 2, or 1 in the base sequence of (E1).

For (E3), the "identity", for example, should be a range in which the protein encoded by the polynucleotide of (E3) has immunogenicity with respect to HTLV-1. The "identity" of (E3) is, for example, at least 70%, at least 75%, at least 80%, at least 85%, at least 90%, at least 95%, at least 96%, at least 97%, at least 98%, or at least 99% relative to the base sequence of (E1).

For (E4), the "polynucleotide that hybridizes", for example, is a polynucleotide that is fully or partially complementary to the polynucleotide of (E1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for these stringent conditions for (E4).

The polynucleotide of (E5) can be established, for example, by conversion to the corresponding codons based on the amino acid sequence of SEQ ID NO: 4.

For (E6), the "one or a plurality of", for example, should be a range in which the protein encoded by the polynucleotide of (E6) has immunogenicity with respect to HTLV-1. The "one or a plurality of" for (E6) is, for example, 1 to 105, 1 to 88, 1 to 70, 1 to 52, 1 to 35, 1 to 31, 1 to 17, 1 to 14, 1 to 10, 1 to 7, 1 to 3, 1 or 2, or 1 in the amino acid sequence of (E5).

For (E7), the "identity", for example, should be a range in which the protein encoded by the polynucleotide of (E7) has immunogenicity with respect to HTLV-1. The "identity" of (E7) is, for example, at least 70%, at least 75%, at least 80%, at least 85%, at least 90%, at least 95%, at least 96%, at least 97%, at least 98%, or at least 99% relative to the amino acid sequence of (E5).

(Polynucleotides encoding immunogenic fragments of Tax)

Polynucleotides encoding immunogenic fragments of Tax, for example, should be in a range in which the protein encoded by the particular polynucleotide has immunogenicity with respect to HTLV-1.

The polynucleotide comprised in the nucleic acid encapsulated in the lipid complex of the present disclosure in particular preferably encodes at least one of the amino acid sequence at positions 151 to 208 (Tax-4 in the examples provided below) or the amino acid sequence at positions 251 to 308 (Tax-6 in the examples provided below) of SEQ ID NO: 4.

Preferably the polynucleotide comprised in the nucleic acid encapsulated in the lipid complex of the present disclosure in particular encodes at least one of the amino acid sequence at positions 151 to 208 (Tax-4 in the examples provided below) or the amino acid sequence at positions 251 to 308 (Tax-6 in the examples provided below) of SEQ ID NO: 4, with the region outside this polynucleotide being composed of the amino acid sequence outside this polynucleotide in SEQ ID NO: 4 or being composed of an amino acid sequence having at least 80%, at least 85%, at least 90%, at least 95%, at least 96%, at least 97%, at least 98%, or at least 99% homology with the amino acid sequence outside this polynucleotide in SEQ ID NO: 4.

The nucleic acid encapsulated in the lipid complex of the present disclosure in particular is more preferably a nucleic acid that encodes at least one of the amino acid sequence at positions 151 to 208 (Tax-4 in the examples provided below) or the amino acid sequence at positions 251 to 308 (Tax-6 in the examples provided below) of SEQ ID NO: 4.

The polynucleotide comprised in the nucleic acid encapsulated in the lipid complex of the present disclosure in particular preferably comprises at least one base sequence of the base sequence at positions 451 to 624 (polynucleotide encoding Tax-4 in the examples provided below) of SEQ ID NO: 6 or the base sequence at positions 751 to 924 (polynucleotide encoding Tax-6 in the examples provided below) of SEQ ID NO: 6.

The polynucleotide comprised in the nucleic acid encapsulated in the lipid complex of the present disclosure in particular is preferably polynucleotide composed of at least one base sequence of the base sequence at positions 451 to 624 (polynucleotide encoding Tax-4 in the examples provided below) of SEQ ID NO: 6 or the base sequence at positions 751 to 924 (polynucleotide encoding Tax-6 in the examples provided below) of SEQ ID NO: 6, with the region outside this polynucleotide in the nucleic acid being the base sequence of SEQ ID NO: 6 outside this polynucleotide or being a base sequence having at least 80%, at least 85%, at least 90%, at least 95%, at least 96%, at least 97%, at least 98%, or at least 99% identity with the base sequence of SEQ ID NO: 6 outside this polynucleotide.

Preferably the polynucleotide comprised in the nucleic acid encapsulated in the lipid complex of the present disclosure in particular encodes at least one of the amino acid sequence at positions 151 to 208 (Tax-4 in the examples provided below) of SEQ ID NO: 4 or the amino acid sequence at positions 251 to 308 (Tax-6 in the examples provided below) of SEQ ID NO: 4, with the region outside this polynucleotide in the nucleic acid being composed of a base sequence that encodes the amino acid sequence in SEQ ID NO: 4 outside the aforementioned amino acid sequence or that encodes an amino acid sequence having at least 80%, at least 85%, at least 90%, at least 95%, at least 96%, at least 97%, at least 98%, or at least 99% homology with the amino acid sequence in SEQ ID NO: 4 outside the aforementioned amino acid sequence.

(III) Nucleic acid comprising a polynucleotide that encodes an immunogenic fragment of HTLV-1 antigenic HBZ protein

The nucleic acid can be a nucleic acid that comprises a polynucleotide that encodes an immunogenic fragment of HTLV-1 antigenic HBZ protein (HBZ). The polynucleotide that encodes an immunogenic fragment of HBZ is a polynucleotide that necessarily comprises a region that encodes an immunogenic fragment of HBZ. This immunogenic fragment of HBZ may be any range that is a protein having immunogenicity with respect to HTLV-1.

The nucleic acid may be a nucleic acid that comprises a polynucleotide that encodes HBZ or an immunogenic fragment thereof. In addition, the nucleic acid may be a polynucleotide that encodes HBZ or an immunogenic fragment thereof. For example, when the nucleic acid is mRNA, the nucleic acid may comprise a 5' cap structure, a 5'-UTR, a 3'-UTR, a poly-A sequence, and so forth, in addition to a polynucleotide that encodes an immunogenic fragment of HBZ.

The amino acid length of the immunogenic fragment of HBZ may be a length of at least 5, 9, 10, 20, 30, 40, 50, 60, 70, 80, 90, 100, or more than 100 amino acids.

In some embodiments, the polynucleotide encoding an immunogenic fragment of HBZ comprises a polynucleotide that encodes HBZ or an immunogenic fragment thereof. Thus, in some embodiments, the nucleic acid is a nucleic acid comprising a polynucleotide that encodes HBZ or an immunogenic fragment thereof.

(f) HBZ or an immunogenic fragments thereof

HBZ can be exemplified by the following proteins (f1), (f2), and (f3).

(f1) a protein composed of the amino acid sequence of SEQ ID NO: 7 or 7
(f2) a protein composed of an amino acid sequence in which one or a plurality of amino acids are deleted, inserted, substituted, or added in the amino acid sequence of SEQ ID NO: 7 or 7
(f3) a protein composed of an amino acid sequence having an at least 80% identity with the amino acid sequence of SEQ ID NO: 7 or 7

The "one or a plurality of" in the case of the protein (f2), for example, should be a range in which (f2) is a protein

having immunogenicity with respect to HTLV-1. The "one or a plurality of" for (f2) is, for example, 1 to 62, 1 to 52, 1 to 41, 1 to 31, 1 to 20, 1 to 10, 1 to 8, 1 to 6, 1 to 4, or 1 or 2 in the amino acid sequence of (f1).

In the case of the protein (f3), the "identity", for example, should be a range in which (f3) is a peptide having immunogenicity with respect to HTLV-1. The "identity" of (f3) is, for example, at least 70%, at least 75%, at least 80%, at least 85%, at least 90%, at least 95%, at least 96%, at least 97%, at least 98%, or at least 99% relative to the amino acid sequence of (f1).

An immunogenic fragment of HBZ, for example, should be a range in which the corresponding protein is a protein having immunogenicity with respect to HTLV-1. An immunogenic fragment of HBZ may be, for example, a freely selected protein fragment of this protein having a length of 5, 10, 20, 30, 40, 50, 60, 70, 80, 90, 100, or more than 100 amino acids.

(F) Polynucleotide encoding HBZ

The polynucleotide that encodes HBZ can be exemplified by polynucleotides selected from the group consisting of the following (F1) to (F6) and (F7):

(F1) a polynucleotide composed of the base sequence with SEQ ID NO: 9 or 10;
(F2) a polynucleotide composed of a base sequence in which one or a plurality of bases are deleted, inserted, substituted, and/or added in the base sequence of (F1);
(F3) a polynucleotide composed of a base sequence having an at least 80% identity with any base sequence of (F1) to (F3);
(F4) a polynucleotide composed of a base sequence that is complementary to a polynucleotide that hybridizes under stringent conditions with a polynucleotide composed of either base sequence of (F1);
(F5) a polynucleotide that encodes a protein composed of the amino acid sequence of SEQ ID NO: 7 or 8;
(F6) a polynucleotide that encodes a protein composed of an amino acid sequence in which one or a plurality of amino acids are deleted, inserted, substituted, and/or added in the amino acid sequence of (F5); and
(F7) a polynucleotide that encodes a protein composed of an amino acid sequence having an at least 80% identity with the amino acid sequence of (F5).

The base sequences with SEQ ID NO: 9 and SEQ ID NO: 10 of (F1) are given below. The base sequences with SEQ ID NO: 9 and SEQ ID NO: 10 are the coding sequences (mRNA) for the HBZ composed of the amino acid sequence with SEQ ID NO: 8. The polynucleotides (F1) with SEQ ID NO: 9 and SEQ ID NO: 10 can be obtained, for example, from HTLV-1.

Polynucleotide (SEQ ID NO: 9) of (F1)

AGGAAAUAAGAGAGAAAAGAAGAGUAAGAAGAAAUAUAAGAGCCACCAUGGC
UGCUAGCGGCCUGUUUCGCUGCCUGCCUGUGAGCUGCCCUGAAGACCUGCUGG
UGGAGGAACUGGUGGACGGCCUGCUGAGCCUGGAAGAAGAACUGAAGGACAA
GGAGGAGGAAGAAGCCGUGCUGGACGGCCUGCUGAGCCUGGAAGAAGAAUCU
CGCGGACGCCUUCGCCGCGGACCUCCUGGAGAAAAAGCUCCUCCUAGAGGAGA
AACACACAGAGAUAGACAAAGAAAAGCUGAGGAGAAGCGCAAGCGCAAGAAG
GAGCGCGAGAAGGAGGAGGAGAAGCAGAUCGCCGAGUACCUGAAGCGCAAAG
AAGAAGAAAAAGCUCGCCGCCGCCGCCGCGCUGAAAAAAAAGCUGCUGAUGUG
GCCCGCCGCAAACAGGAAGAACAGGAACGCCGCGAACGCAAAUGGCGCCAGGG
CGCUGAAAAAGCUAAGCAGCACAGCGCCCGCAAGGAGAAGAUGCAGGAACUGG
GCAUCGACGGCUACACCCGCCAGCUGGAAGGCGAAGUGGAAAGCCUGGAAGCC
GAACGCCGCAAGCUGCUGCAGGAGAAGGAGGACCUGAUGGGCGAGGUGAACU
ACUGGCAGGGCCGCCUGGAAGCCAUGUGGCUGCAGUGAUAAGCUGCCUUCUGC
GGGGCUUGCCUUCUGGCCAUGCCCUUCUUCUCUCCCUUGCACCUGUACCUCUU
GGUCUUUGAAUAAAGCCUGAGUAGGAAGGCGGAAAAAAAAAAAAAAAAAAA
AAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAA

Polynucleotide (SEQ ID NO: 10) of (F1)

AUGGCUGCUAGCGGCCUGUUUCGCUGCCUGCCUGUGAGCUGCCCUGAAGACCU
GCUGGUGGAGGAACUGGUGGACGGCCUGCUGAGCCUGGAAGAAGAACUGAAG
GACAAGGAGGAGGAAGAAGCCGUGCUGGACGGCCUGCUGAGCCUGGAAGAAG
AAUCUCGCGGACGCCUUCGCCGCGGACCUCCUGGAGAAAAAGCUCCUCCUAGA
GGAGAAACACACAGAGAUAGACAAAGAAAAGCUGAGGAGAAGCGCAAGCGCA
AGAAGGAGCGCGAGAAGGAGGAGGAGAAGCAGAUCGCCGAGUACCUGAAGCG

CAAAGAAGAAGAAAAAGCUCGCCGCCGCCGCCGCGCUGAAAAAAAAGCUGCUG

AUGUGGCCCGCCGCAAACAGGAAGAACAGGAACGCCGCGAACGCAAAUGGCGC

CAGGGCGCUGAAAAAGCUAAGCAGCACAGCGCCCGCAAGGAGAAGAUGCAGGA

ACUGGGCAUCGACGGCUACACCCGCCAGCUGGAAGGCGAAGUGGAAAGCCUGG

AAGCCGAACGCCGCAAGCUGCUGCAGGAGAAGGAGGACCUGAUGGGCGAGGU

GAACUACUGGCAGGGCCGCCUGGAAGCCAUGUGGCUGCAG

For (F2), the "one or a plurality of", for example, should be a range in which the protein encoded by the polynucleotide of (F2) has immunogenicity with respect to HTLV-1. The "one or a plurality of" for (F2) is, for example, 1 to 186, 1 to 156, 1 to 123, 1 to 94, 1 to 93, 1 to 60, 1 to 30, 1 to 24, 1 to 18, 1 to 12, 1 to 6, 1 to 3, 1 or 2, or 1 in the base sequences of (F1).

For (F3), the "identity", for example, should be a range in which the protein encoded by the polynucleotide of (F3) has immunogenicity with respect to HTLV-1. The "identity" of (F3) is, for example, at least 70%, at least 75%, at least 80%, at least 85%, at least 90%, at least 95%, at least 96%, at least 97%, at least 98%, or at least 99% relative to the base sequences of (F1).

For (F4), the "polynucleotide that hybridizes", for example, is a polynucleotide that is fully or partially complementary to the polynucleotide of (F1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for these stringent conditions for (F4).

The polynucleotide of (F5) can be established, for example, by conversion to the corresponding codons based on the amino acid sequence of SEQ ID NO: 7 or 8.

For (F6), the "one or a plurality of", for example, should be a range in which the protein encoded by the polynucleotide of (F6) has immunogenicity with respect to HTLV-1. The "one or a plurality of" for (F6) is, for example, 1 to 62, 1 to 52, 1 to 41, 1 to 31, 1 to 20, 1 to 10, 1 to 8, 1 to 6, 1 to 4, or 1 or 2 in the amino acid sequences of (F5).

For (F7), the "identity", for example, should be a range in which the protein encoded by the polynucleotide of (F7) has immunogenicity with respect to HTLV-1. The "identity" of (F7) is, for example, at least 70%, at least 75%, at least 80%, at least 85%, at least 90%, at least 95%, at least 96%, at least 97%, at least 98%, or at least 99% relative to the amino acid sequences of (F5).

(Polynucleotides encoding immunogenic fragments of HBZ)

The polynucleotides encoding immunogenic fragments of HBZ, for example, should be in a range in which the protein encoded by the particular polynucleotide has immunogenicity with respect to HTLV-1.

The polynucleotide comprised in the nucleic acid encapsulated in the lipid complex of the present disclosure in particular preferably encodes the amino acid sequence at positions 1 to 58 (HBZ-1 in the examples provided below) of SEQ ID NO: 8.

Preferably the polynucleotide comprised in the nucleic acid encapsulated in the lipid complex of the present disclosure in particular encodes the amino acid sequence at positions 1 to 58 (HBZ-1 in the examples provided below) of SEQ ID NO: 8, with the region outside this polynucleotide being composed of the amino acid sequence outside this polynucleotide in SEQ ID NO: 1 or being composed of an amino acid sequence having at least 80%, at least 85%, at least 90%, at least 95%, at least 96%, at least 97%, at least 98%, or at least 99% homology with the amino acid sequence outside this polynucleotide in SEQ ID NO: 8.

The nucleic acid encapsulated in the lipid complex of the present disclosure in particular is more preferably a nucleic acid that encodes the amino acid sequence at positions 1 to 58 (HBZ-1 in the examples provided below) of SEQ ID NO: 8.

The polynucleotide comprised in the nucleic acid encapsulated in the lipid complex of the present disclosure in particular preferably comprises the base sequence at positions 1 to 174 (polynucleotide encoding HBZ-1 in the examples provided below) of SEQ ID NO: 10.

The polynucleotide comprised in the nucleic acid encapsulated in the lipid complex of the present disclosure in particular is preferably a polynucleotide composed of the base sequence at positions 1 to 174 (polynucleotide encoding

HBZ-1 in the examples provided below) of SEQ ID NO: 10, with the region outside this polynucleotide in the nucleic acid being the base sequence in SEQ ID NO: 10 outside this polynucleotide or being a base sequence having at least 80%, at least 85%, at least 90%, at least 95%, at least 96%, at least 97%, at least 98%, or at least 99% identity with the base sequence of SEQ ID NO: 10 outside this polynucleotide.

Preferably the polynucleotide comprised in the nucleic acid encapsulated in the lipid complex of the present disclosure in particular encodes the amino acid sequence at positions 1 to 58 (HBZ-1 in the examples provided below) of SEQ ID NO: 1, with the region outside this polynucleotide in the nucleic acid being composed of a base sequence that encodes the amino acid sequence in SEQ ID NO: 10 outside the aforementioned amino acid sequence or that encodes an amino acid sequence having at least 80%, at least 85%, at least 90%, at least 95%, at least 96%, at least 97%, at least 98%, or at least 99% homology with the amino acid sequence in SEQ ID NO: 10 outside the aforementioned amino acid sequence.

The nucleic acid encapsulated in the lipid complex of the present disclosure in particular more preferably comprises polynucleotide composed of polynucleotide that encodes the base sequence at positions 1 to 174 (polynucleotide encoding HBZ-1 in the examples provided below) of SEQ ID NO: 10.

(Methods for producing the individual proteins)

Due to acquisition of the nucleic acids, the individual proteins can be synthesized by genetic engineering techniques, and, as a specific example, can be produced using the transformants described below. The protein may be produced, for example, by in vitro synthesis.

For example, the aforementioned transformant may be cultured and the Gag p15 or an immunogenic fragment thereof, Gag p19 or an immunogenic fragment thereof, and/or Gag p24 or an immunogenic fragment thereof may be collected from this culture material. The culture material may be the culture supernatant or may be the transformant, e.g., cultured cells or cultured bacterial cells, or a processed material or disrupted material thereof.

After culturing, when the Gag p15 or an immunogenic fragment thereof, Gag p19 or an immunogenic fragment thereof, and/or Gag p24 or an immunogenic fragment thereof of the present disclosure has been produced within the host, in a production method of the present disclosure, for example, the Gag p15 or an immunogenic fragment thereof, Gag p19 or an immunogenic fragment thereof, and/or Gag p24 or an immunogenic fragment thereof is extracted by disruption of the host. When the Gag p15 or an immunogenic fragment thereof, Gag p19 or an immunogenic fragment thereof, and/or Gag p24 or an immunogenic fragment thereof of the present disclosure has been produced or secreted outside the host, in a production method of the present disclosure, for example, the culture fluid is used as such or the host is removed by, e.g., centrifugation. The protein, e.g., Gag p15 or an immunogenic fragment thereof, Gag p19 or an immunogenic fragment thereof, and/or Gag p24 or an immunogenic fragment thereof, can then be isolated or purified in a production method of the present disclosure, for example, by using the general biochemical methods used to isolate and purify proteins, and specifically using a single one of, or a suitable combination of, concentration using an ultrafiltration membrane; salting out, e.g., ammonium sulfate precipitation; chromatography using various columns, e.g., gel filtration, ion-exchange chromatography, affinity chromatography, and so forth.

(Structure of the lipid complex)

The lipid complex of the present disclosure has a structure in which at least one species of the nucleic acids described in the preceding is encapsulated in lipid.

In some embodiments, the lipid complex is a lipid particle. This lipid particle has a structure in which the nucleic acid is encapsulated within a microparticle that is composed of lipid.

The lipid complex in some embodiments is a lipid nanoparticle (LNP). This LNP has a structure in which the nucleic acid is encapsulated within a particle that is composed of lipid and has a nanosize average particle diameter (for example, about 1 nm to 1,000 nm).

(The lipid)

In some embodiments, the lipid composing the lipid complex comprises (i) a cationic lipid and (ii) at least one lipid selected from the group consisting of neutral lipids, polyethylene glycol-modified lipids, and sterols. The configuration of the lipid complex comprising this (i) and (ii) can be exemplified by complexes of the nucleic acid and a membrane composed of a lipid mono (monomolecular) layer (reverse micelle), complexes of the nucleic acid and a liposome, and complexes of the nucleic acid and a micelle.

(i) Cationic lipids

There are no particular limitations on the cationic lipid. For example, the cationic lipids described in WO 2015/105131, WO 2016/104580, WO 2017/222016, and WO 2019/131580 can be used. The cationic lipid may be a salt form, a hydrate, or a solvate.

Cationic lipid according to one embodiment can be exemplified by the compound with the following formula (I) or a pharmaceutically acceptable salt thereof.

[C4]

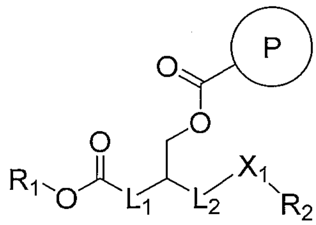

(I)

In formula (I), $L_1$ and $L_2$ each independently represent a $C_{3-10}$ (for example, $C_{3-8}$) alkylene group. In one embodiment, $L_1$ and $L_2$ each independently represent a $C_{3-10}$ (for example, $C_{3-8}$) straight-chain alkylene group.

In formula (I), $R_1$ and $R_2$ each independently represent a $C_{4-22}$ alkyl group or a $C_{4-22}$ alkenyl group.

In formula (I), $X_1$ represents a single bond or - CO - O -.

In formula (I), the ring P represents any of the following formulas (P-1) to (P-6). In one embodiment, the ring P represents any of the following formula (P-1), formula (P-2), (P-4), (P-5), and (P-6). In one embodiment, the ring P represents the following formula (P-1) or (P-6). In one embodiment, the ring P represents the following formula (P-1).

[C5]

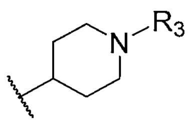 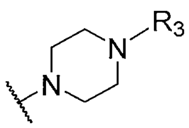 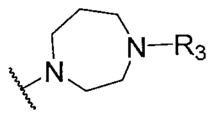 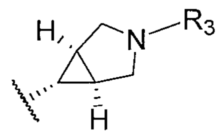 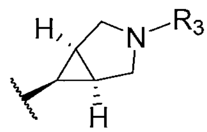 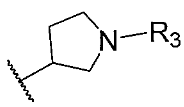

(P-1) (P-2) (P-3) (P-4) (P-5) (P-6)

The $R_3$ in these formulas (P-1), (P-2), (P-3), (P-4), (P-5), and (P-6) represents a $C_{1-3}$ alkyl group.

The cationic lipid can be exemplified by 1-oxo-1-(undecan-5-yloxy)nonadecan-10-yl-1-methylpiperidine-4-carboxylate, 1-((2-butyloctyl)oxy)-1-oxononadecan-10-yl-1-methylpiperidine-4-carboxylate, 1-oxo-1-(undecan-5-yloxy) heptadecan-8-yl-1-methylpiperidine-4-carboxylate, 21-oxo-21-(undecan-5-yloxy)heneicosan-10-yl-1-methylpiperidine-4-carboxylate, 21-(octan-3-yloxy)-21-oxoheneicosan-10-yl-1-methylpiperidine-4-carboxylate, 1-((2-butyloctyl) oxy)-1-oxoeicosan-10-yl-1-methylpiperidine-4-carboxylate, (Z)-1-((2-butylnon-3-en-1-yl)oxy)-1-oxoeicosan-10-yl-1-methylpiperidine-4-carboxylate, 1-oxo-1-((3-pentyloctyl)oxy)eicosan-10-yl-1-methylpiperidine-4-carboxylate, 1-((3,4-dipropylheptyl)oxy)-1-oxoeicosan-10-yl-1-methylpiperidine-4-carboxylate, 1-((6-(butyldisulfanyl)-3-(3-(butyldisulfanyl) propyl)hexyl)oxy)-1-oxoeicosan-10-yl-1-methylpiperidine-4-carboxylate, 2-butyloctyl-10-((4-(dimethylamino)butanoyl) oxy)eicosanoate, 2-{9-[(2-butyloctyl)oxy]-9-oxononyl}dodecyl 1-methylpiperidine-4-carboxylate, 2-{9-oxo-9-[(3-pentyloetyl)oxy]nonyl}dodecyl 1-methylpiperidine-4-carboxylate, 2-nonyl-11-oxo-11-[(3-pentyloctyl)oxy]undecyl 1-methylpiperidine-4-carboxylate, bis(3-pentyloctyl) 9-{[(1-methylpiperidine-4-carbonyl)oxy]methyl}heptadecanedioate, di[(Z)-2-nonen-1-yl] 9-{[(1-methylpiperidine-4-carbonyl)oxy]methyl}heptadecanedioate, 1-(2-octylcyclopropyl)heptadecan-8-yl-1-methylpiperidine-4-carboxylate, (3S)-2-{9-oxo-9-[(3-pentyloctyl)oxy]nonyl}dodecyl 1-methylpyrrolidine-3-carboxylate, (3R)-2-{9-oxo-9-[(3-pentyloctyl)oxy]nonyl}dodecyl 1-methylpyrrolidine-3-carboxylate, and pharmaceutically acceptable salts of the preceding.

In some embodiments, the cationic lipid comprises at least one selected from 1-((2-butyloctyl)oxy)-1-oxoeicosan-10-yl-1-methylpiperidine-4-carboxylate, 1-((2-butyloctyl)oxy)-1-oxononadecan-10-yl-1-methylpiperidine-4-carboxylate, 2-{9-oxo-9-[(3-pentyloctyl)oxy]nonyl}dodecyl 1-methylpiperidine-4-carboxylate, 1-(2-octylcyclopropyl)heptadecan-8-yl-1-methylpiperidine-4-carboxylate, (3S)-2-{9-oxo-9-[(3-pentyloctyl)oxy]nonyl}dodecyl 1-methylpyrrolidine-3-carboxylate, or (3R)-2-{9-oxo-9-[(3-pentyloctyl)oxy]nonyl}dodecyl 1-methylpyrrolidine-3-carboxylate, or pharmaceutically acceptable salts of the preceding.

In a particular embodiment, the cationic lipid is 2-{9-oxo-9-[(3-pentyloctyl)oxynonyl}dodecyl 1-methylpiperidine-4-carboxylate or a pharmaceutically acceptable salt thereof. The structure of 2-{9-oxo-9-[(3-pentyloctyl)oxy]nonyl} dodecyl 1-methylpiperidine-4-carboxylate is given below.

[C6]

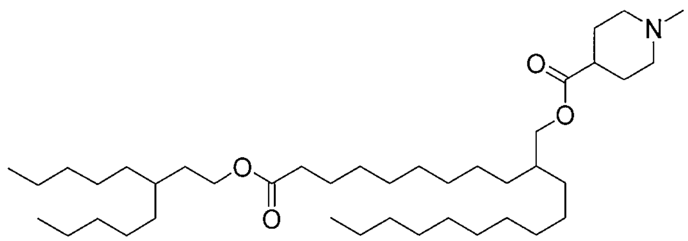

A single cationic lipid may be used by itself for the cationic lipid or a mixture of two or more cationic lipids may be used.

(ii) Neutral lipids, polyethylene glycol-modified lipids, and sterols

There are no particular limitations on the neutral lipids, which can be exemplified by phospholipids such as dioleoylphosphatidylethanolamine (DOPE), phosphatidylethanolamine (POPE), dioleoylphosphatidylethanolamine 4-(N-maleimidomethyl)cyclohexane-1-carboxylate (DOPE-mal), palmitoyloleoylphosphatidylcholine (POPC), stearyloleoylphosphatidylcholine (SOPC), hydrogenated soybean phosphatidylcholine (HSPC), egg phosphatidylcholine (EPC), dimyristoylphosphatidylcholine (DMPC), dipalmitoylphosphatidylcholine (DPPC), distearoylphosphatidylcholine (DSPC), diarachidoylphosphatidylcholine (DAPC), dibehenoylphosphatidylcholine (DBPC), dilignoceroylphosphatidylcholine (DLPC), dioleoylphosphatidylcholine (DOPC), dioleoylphosphatidylglycerol (DOPG), dipalmitoylphosphatidylglycerol (DPPG), distearoylphosphatidylglycerol (DSPG), dioleylphosphatidylserine (DOPS), and sphingomyelin; ceramide (Cer); and so forth.

In some embodiments, the neutral lipid comprises at least one selected from DOPE, HSPC, DPPC, DSPC, or DAPC.

In a particular embodiment the neutral lipid comprises DPPC.

A single neutral lipid can be used by itself as the neutral lipid or a mixture of two or more can be used.

There are no particular limitations on the polyethylene glycol-modified lipids, which can be exemplified by PEG2000-DMG (PEG2000-dimyristylglycerol), PEG2000-DPG (PEG2000-dipalmitoylglycerol), PEG2000-DSG (PEG2000-distearoylglycerol), PEG5000-DMG (PEG5000-dimyristylglycerol), PEG5000-DPG (PEG5000-dipalmitoylglycerol), PEG5000-DSG (PEG5000-distearoylglycerol), PEG-cDMA (N-[(methoxypoly(ethylene glycol)2000)carbamyl]-1,2-dimyristyloxylpropyl-3-amine), PEG-C-DOMG (R-3-[(ω-methoxypoly(ethylene glycol)2000)carbamoyl)]-1,2-dimyristyloxylpropyl-3-amine), PEG-diacylglycerol (DAG), PEG-dialkyloxypropyl (DAA), PEG-phospholipid, PEG-ceramide (Cer), and PEG-cholesterol. PEG-DAA can be exemplified by PEG-dilauryloxypropyl, PEG-dimyristyloxypropyl, PEG-dipalmityloxypropyl, and PEG-distearyloxypropyl.

In some embodiments, the polyethylene glycol-modified lipid comprises at least one selected from PEG2000-DMG, PEG2000-DPG, PEG2000-DSG, PEG-cDMA, or PEG-C-DOMG.

In a particular embodiment, the polyethylene glycol-modified lipid comprises PEG2000-DMG.

A single polyethylene glycol-modified lipid may be used by itself for the polyethylene glycol-modified lipid or a mixture of two or more may be used.

In addition, the end of the PEG in the polyethylene glycol-modified lipid may be methoxylated (MPEG, methoxypolyethylene glycol). For example, the PEG2000-DMG may comprise MPEG2000-DMG.

There are no particular limitations on the sterol, which can be exemplified by cholesterol, dihydrocholesterol, lanosterol, β-sitosterol, campesterol, stigmasterol, brassicasterol, ergosterol, fucosterol, and 3β-[N-(N',N'-dimethylaminoethyl)carbamoyl]cholesterol (DC-Chol).

In some embodiments, the sterol comprises at least one selected from cholesterol, dihydrocholesterol, lanosterol, or β-sitosterol.

In a particular embodiment, the sterol comprises cholesterol.

A single sterol can be used by itself as the sterol or a mixture of two or more can be used.

(Composition and blending proportions)

There are no particular limitations on the lipid content in the lipid complex or on the blending proportions of the individual lipid components that compose the lipid.
In some embodiments, the lipid complex comprises, with reference to the total weight of the lipid complex, for example, 50 to 99.99 weight% lipid component, or, for example, 70 to 99.99 weight% lipid component, or, for example, 90 to 99 weight% lipid component.
In some embodiments, the lipid complex comprises, with reference to the total lipid comprised by the lipid complex, for example, 10 to 99 mol% of the above-described cationic lipid, or 20 to 90 mol%, or 30 to 70 mol%.
In some embodiments, the lipid complex may comprise, with reference to the total lipid comprised by the lipid complex, for example, 0 to 50 mol% neutral lipid, or 0 to 40 mol%, or 0 to 30 mol%.
In some embodiments, the lipid complex may comprise, with reference to the total lipid comprised by the lipid complex, for example, 0 to 30 mol% polyethylene glycol-modified lipid, or 0 to 20 mol%, or 0 to 10 mol%.
In some embodiments, the sterol may be comprised at, with reference to the total lipid comprised by the lipid complex, for example, 0 to 90 mol%, or 10 to 80 mol%, or 20 to 50 mol%.
The nucleic acid content in the lipid complex is not particularly limited.
In some embodiments, the lipid complex comprises, with reference to the total weight of the lipid complex, for example, 0.01 to 50 weight% nucleic acid, or, for example, 0.1 to 30 weight% nucleic acid, or, for example, 1 to 10 weight% nucleic acid.
In some embodiments, the lipid complex comprises, with reference to the total weight of the lipid complex, 0.01 to 50 weight% nucleic acid and 50 to 99.99 weight% lipid, and comprises, based on the total lipid compriseed by the lipid complex, 30 to 70 mol% cationic lipid, 0 to 30 mol% neutral lipid, 0 to 10 mol% polyethylene glycol-modified lipid, and 20 to 50 mol% sterol. In some embodiments, the lipid complex comprises, with reference to the total weight of the lipid complex, 0.01 to 50 weight% nucleic acid and 50 to 99.99 weight% lipid, and comprises, based on the total lipid comprised by the lipid complex, 30 to 70 mol% cationic lipid, 1 to 30 mol% neutral lipid, 1 to 10 mol% polyethylene glycol-modified lipid, and 20 to 50 mol% sterol.
The lipid component combination in the lipid complex is not particularly limited.
In some embodiments, the lipid comprises a combination of cationic lipid, neutral lipid, and sterol.
In some embodiments, the lipid comprises a combination of the above-described cationic lipid, neutral lipid, polyethylene glycol-modified lipid, and sterol. It has been reported that cationic lipids are necessary for encapsulating nucleic acids and for efficiently delivering nucleic acids into a target cell, and that polyethylene glycol-modified lipids are necessary for inhibiting particle aggregation. Moreover, a stable particle in which nucleic acid is encapsulated can be formed through the simultaneous presence of the four types of lipids, which includes neutral lipid and sterol in addition to the cationic lipid and polyethylene glycol-modified lipid.
In some embodiments, the lipid complex is composed of cationic lipid / neutral lipid / polyethylene glycol-modified lipid / sterol as the lipids, and the molar ratio of the lipids may be, for example, 10-99 / 0-50 / 0-30 / 0-90, or 20-90 / 0-40 / 0-20 /10-80, or 30-70 / 0-30 / 0-10 / 20-50.
In some embodiments, the cationic lipid / neutral lipid / polyethylene glycol-modified lipid / sterol molar ratio in the lipid complex may be 10-99 / 1-50 / 1-30 / 1-90, or 20-90 / 1-40 / 1-20 / 10-80, or 30-70 / 1-30 / 1-10 / 20-50.
In a particular embodiment, the cationic lipid / neutral lipid / polyethylene glycol-modified lipid / sterol molar ratio in the lipid complex is 47 / 11 / 1.5 / 40.5.
In the lipid complex according to one embodiment, the lipid comprises 2-{9-oxo-9-[(3-pentyloctyl)oxy]nonyl} dodecyl 1-methylpiperidine-4-carboxylate as the cationic lipid, DPPC as the neutral lipid, (M)PEG2000-DMG as the polyethylene glycol-modified lipid, and cholesterol as the sterol.

(Characteristics of the lipid complex)

In the present Description, the "average particle diameter" of the lipid complex refers to the Z-average particle diameter, and the average particle diameter is measured using a dynamic light-scattering method. The average particle diameter (Z-average) of the lipid complex may be, for example, 10 to 1,000 nm, or, for example, 30 to 500 nm, or, for example, 30 to 200 nm.
The encapsulation rate of the nucleic acid in the lipid complex was measured, for example, using Quant-iT RiboGreen RNA Reagent (Invitrogen, Cat#R11491). Specifically, the encapsulation ratio can be calculated defining the nucleic acid concentration (A) measured with dilution with RNase Free Water as the nucleic acid present in the lipid complex external solution, and defining the nucleic acid concentration (B) measured with dilution with 1% Triton X-100 as the total nucleic acid concentration in the formulation.

$$\text{Encapsulation rate (\%)} = 100 - (A/B) \times 100$$

In some embodiments, the encapsulation rate (%) of nucleic acid in the lipid complex calculated using the above-described method is, for example, higher than 80%, 85%, or 90%. In one embodiment, the encapsulation rate (%) of nucleic acid in the lipid complex is higher than 90%.
In some embodiments, a polydispersity index (PDI) of less than 0.3 (in particular less than 0.2, or in particular less than 0.1) is exhibited.

(Methods for producing the lipid complex)

The methods for encapsulating effective molecules in lipid complexes can be exemplified by reverse-phase evaporative methods, zwitterion (NaCl) hydration methods, cationic core hydration methods, and a method using ethanol and calcium (refer also to Biomembr., 1468, 239-252 (2000)). The nucleic acid-encapsulating lipid complexes of the present disclosure can be prepared using methods known in the relevant technical fields as described in the preceding.
In some embodiments, the lipid complex can be prepared, for example, by mixing a nucleic acid-comprising acidic buffer with a lipid solution that comprises a cationic lipid and at least one lipid selected from the group consisting of neutral lipids, polyethylene glycol-modified lipids, and sterols. This method yields a lipid complex in which the interior is filled with a core of lipid and nucleic acid.
The solvent used to dissolve the lipid can be exemplified by polar organic solvents, e.g., alcohols and so forth, and may be, for example, ethanol, isopropanol, chloroform, or t-butanol.
The acidic buffer used to dissolve the nucleic acid can be exemplified by sulfate buffers, phosphate buffers, phthalate buffers, tartrate buffers, citrate buffers, formate buffers, oxalate buffers, and acetate buffers.
In an embodiment, the lipid complex can be produced, for example, by a method comprising: a step (a) of obtaining a mixture by mixing a nucleic acid-containing aqueous solution with a polar organic solvent-containing aqueous solution that comprises a cationic lipid and at least one lipid selected from the group consisting of neutral lipids, polyethylene glycol-modified lipids, and sterols; and a step (b) of reducing the content of the polar organic solvent in the mixture.
A lipid complex in which the nucleic acid is encapsulated in a particle composed of lipid can be formed through electrostatic interactions between the water-soluble nucleic acid and the cationic lipid and through hydrophobic interactions between the lipids. For example, the reduction in the content of the polar organic solvent in the mixture causes the lipid components, comprising the cationic lipid and the at least one lipid selected from the group consisting of neutral lipids, polyethylene glycol-modified lipids, and sterols, to undergo a change in solubility with respect to the polar organic solvent-containing aqueous solution, and the lipid complex can then be formed as a consequence of this. The polar organic solvent can be exemplified by alcohols such as ethanol.
First, in step (a), a mixture is obtained by mixing a nucleic acid (III)-containing aqueous solution with a polar organic solvent-containing aqueous solution in which there are dissolved (I) the above-described cationic lipid and (II) at least one lipid selected from the group consisting of neutral lipids, polyethylene glycol-modified lipids, and sterols. The concentration of the polar organic solvent in the polar organic solvent-containing aqueous solution is not particularly limited, except that the condition that the lipid molecules are dissolved should satisfied even after mixing with the aqueous solution of the nucleic acid. For example, the concentration of the polar organic solvent in the polar organic solvent-containing aqueous solution in step (a) can be 0.1 to 60 weight%. The nucleic acid-containing aqueous solution can be obtained, for example, by dissolving the nucleic acid in an acidic buffer.
Subsequently, in step (b), water, etc., is added to the mixture to reduce the content of the polar organic solvent. The formation of a lipid complex can be brought about by doing this. In order to efficiently form the lipid complex, the content of the polar organic solvent is preferably rapidly reduced. For example, the concentration of the polar organic solvent in the final polar organic solvent-containing aqueous solution in step (b) can be 0 to 5 weight%.
In addition, the mixture obtained in step (a) may be dialyzed to remove the polar organic solvent and the solvent may be replaced with a pharmaceutically acceptable medium. Formation of a lipid complex can be brought about by this reduction in the content of the polar organic solvent in the solution by the dialysis process.
In accordance with the production method of this embodiment, a lipid complex can be obtained in which a nucleic acid is efficiently encapsulated within a lipid particle.

2. The pharmaceutical composition

One aspect of the present disclosure relates to a pharmaceutical composition (hereinafter also referred to as the "pharmaceutical composition of the present disclosure" or the "pharmaceutical composition") that comprises the lipid

complex of the present disclosure. In some embodiments, the present disclosure provides a use of the lipid complex in the manufacture of a pharmaceutical composition.

In some embodiments, the pharmaceutical composition may comprise the lipid complex as described in the preceding, a pharmaceutically acceptable carrier or vehicle, and, depending on the particular circumstances, other additives.

The pharmaceutically acceptable carriers or vehicles can be exemplified by pharmaceutically acceptable media, suspending agents, solubilizing agents, antiseptics, fillers, bulking agents, binders, wetting agents, disintegrants, lubricants, dispersants, flavorants, stabilizers, isotonicity agents, preservatives, adsorption inhibitors, surfactants, diluents, pH adjusters, soothing agents, buffers, sulfur-containing reducing agents, and antioxidants, which are added as appropriate within a range that does not impair the effects of the present disclosure.

The pharmaceutically acceptable media can be exemplified by sterile water; physiological saline; isotonic solutions compriseing auxiliary agents such as glucose, D-sorbitol, D-mannose, D-mannitol, and sodium chloride; and buffer solutions such as phosphate buffers, citrate buffers, and acetate buffers.

The suspending agent is not particularly limited and can be exemplified by methyl cellulose, polysorbate 80, hydroxyethyl cellulose, gum arabic, tragacanth powder, sodium carboxymethyl cellulose, and polyoxyethylene sorbitan monolaurate.

The solubilizing agent is not particularly limited and can be exemplified by alcohols such as ethanol, propylene glycol, and polyethylene glycol, and by polyoxyethylene hydrogenated castor oil, polysorbate 80, nicotinamide, polyoxyethylene sorbitan monolaurate, macrogol, and ethyl esters of castor oil fatty acids.

The stabilizer is not particularly limited and can be exemplified by dextran 40, methyl cellulose, gelatin, sodium sulfite, and sodium metasulfate.

The isotonicity agent is not particularly limited and can be exemplified by D-mannitol and sorbitol.

The preservative is not particularly limited and can be exemplified by methyl para-hydroxybenzoate, ethyl para-hydroxybenzoate, sorbic acid, phenol, cresol, and chlorocresol.

The adsorption inhibitor is not particularly limited can be exemplified by human serum albumin, lecithin, dextran, ethylene oxide-propylene oxide copolymer, hydroxypropyl cellulose, methyl cellulose, hydrogenated castor oil, and polyethylene glycol.

The sulfur-containing reducing agent is not particularly limited and can be exemplified by sulfur-containing reducing agents that comprise a sulfhydryl group, such as N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and thioalkanoic acids having 1 to 7 carbon atoms.

The antioxidant is not particularly limited and can be exemplified by erythorbic acid, dibutylhydroxytoluene, butylhydroxyanisole, $\alpha$-tocopherol, tocopherol acetate, L-ascorbic acid and salts thereof, L-ascorbyl palmitate, L-ascorbyl stearate, sodium hydrogen sulfite, sodium sulfite, triamyl gallate, and propyl gallate, or chelating agents such as sodium ethylenediaminetetraacetate (EDTA), sodium pyrophosphate, and sodium metaphosphate.

Other additives can be exemplified by sugars such as sucrose, glucose, sorbitol, lactose, etc.; amino acids such as glutamine, glutamic acid, sodium glutamate, histidine, etc.; salts of organic acids such as citric acid (e.g., sodium citrate, potassium citrate), phosphoric acid, acetic acid (e.g., sodium acetate), lactic acid, carbonic acid, tartaric acid, etc.; and inorganic salts such as sodium chloride, potassium chloride, calcium chloride, sodium phosphate, potassium phosphate, sodium bicarbonate, etc.

The pharmaceutical composition can be formulated into various dosage forms. An injectable is an example of a dosage form of the pharmaceutical composition.

The pharmaceutical composition, for example, may be in a powder form provided by removal of the solvent by, for example, lyophilization, or may be in a liquid form.

The pharmaceutical composition according to some embodiments is a powder composition that comprises a lipid complex according to embodiments that are described in the preceding. The powder composition may be prepared by removing the solvent, for example, by filtration, centrifugal separation, and so forth, from a liquid composition (dispersion) that comprises the lipid complex, or may be prepared by lyophilization of such a dispersion. When the pharmaceutical composition takes the form of a powder, it can be used as an injectable by suspension or dissolution in a pharmaceutically acceptable medium prior to use.

The composition according to one embodiment of the present disclosure is a liquid composition that comprises a lipid complex according to embodiments that are described in the preceding and a pharmaceutically acceptable medium. When the composition is a liquid, it can be directly used as an injectable or can be used as an injectable by suspension or dissolution in a pharmaceutically acceptable medium.

There are no particular limitations on the subject to whom the pharmaceutical composition of the present disclosure may be administered, and it can be used with various animals. The pharmaceutical composition of the present disclosure can be applied to, for example, humans or non-human mammals (monkeys, mice, rats, rabbits, cows, horses, goats, etc.) and preferably to experimental animals in clinical trials, screenings, and experiments and to humans.

The use conditions (administration conditions) for the pharmaceutical composition of the present disclosure are not particularly limited, and, for example, the form of administration, timing of administration, dosage, and so forth can be established as appropriate depending on the type of the above-described active ingredient in the pharmaceutical composition, the type of subject for administration, and so forth.

The method of administering the pharmaceutical composition of the present disclosure to a subject (route of administration, dosage, number of administrations per day, timing of administration, and so forth) is not limited and can be appropriately determined by the person skilled in the art (e.g., a physician) in accordance with the health of the subject, the severity of the disease, the type of concomitant drug, and so forth.

For example, the number of administrations of the pharmaceutical composition is one or a plurality of times. The plurality of times is, for example, two, three, four, five, or more times. The number of administrations may be appropriately determined while confirming the prophylactic effect on the subject of the administration. In the case of administration a plurality of times, the administration interval can be appropriately determined while confirming the prophylactic effect on the subject of the administration, and can be exemplified by once a day, once a week, once every two weeks, once a month, once every three months, once every six months, and so forth.

The administration of the pharmaceutical composition of the present disclosure to a subject is not particularly limited and may be parenteral administration, for example, intracerebral administration, intrathecal administration, intramuscular administration, subcutaneous administration, intradermal administration, intravenous administration, and so forth. For example, intramuscular administration or subcutaneous administration is preferred because this enables a safe and stable administration regardless of the skill of the individual performing the administration.

The dosage of the pharmaceutical composition will also vary depending on the subject, target organ, symptoms, and method of administration.

In one embodiment, the pharmaceutical composition of the present disclosure may be administered in an amount sufficient to induce an immune response against HTLV-1, i.e., an effective dosage, in accordance with the form of administration. The dosage may be appropriately determined depending on, for example, the age, weight, symptoms, and so forth of the subject. In one embodiment, the dosage of the pharmaceutical composition may be, for example, 0.01 mg to 100 mg, or 0.1 mg to 50 mg, or 0.3 mg to 10 mg per 1 kg of body weight of the subject.

The pharmaceutical composition of the present disclosure, for example, may be used in vitro or may be used in vivo. The pharmaceutical composition of the present disclosure, for example, may also be used as a reagent employed in research or may be used as a pharmaceutical product. In the former case, the pharmaceutical composition of the present disclosure may also be referred to as a test reagent or test kit.

The target for administration of the pharmaceutical composition of the present disclosure is not particularly limited. When the pharmaceutical composition of the present disclosure is used in vitro, the target for administration can be, for example, cells, tissue, an organ, and so forth, and the cells can be, for example, cells acquired from a living organism or cultured cells, while the tissue or organ can be, for example, tissue (living tissue) or an organ acquired from a living organism. The cells can be, for example, immune cells such as T cells, B cells, NK cells, dendritic cells, and so forth.

When the pharmaceutical composition of the present disclosure is used in vivo, the subject (target for administration) can be, for example, as described in the exemplary illustrations provided in the preceding.

The pharmaceutical composition of the present disclosure, through its administration to a subject, can induce an immune response against HTLV-1 (in particular, cellular immunity against HTLV-1 and in particular cellular immunity mediated by cytotoxic T cells). As a consequence, the pharmaceutical composition of the present disclosure can also be referred to as a vaccine, a vaccine composition, or a vaccine preparation. The vaccine, vaccine composition, or vaccine preparation of the present disclosure can prevent the onset of HTLV-1-associated diseases (e.g., ATL onset) by activating cellular immunity and suppressing an increase in infected cells. The pharmaceutical composition of the present disclosure, through its administration in the form of a nucleic acid-encapsulating lipid complex, can efficiently release the nucleic acid into the cytoplasm. A pharmaceutical composition for inducing an immune response against HTLV-1 (in particular, cellular immunity against HTLV-1 and in particular cellular immunity mediated by cytotoxic T cells) is provided according to some embodiments of the present disclosure. A method for inducing an immune response against HTLV-1, comprising a step of administering the pharmaceutical composition to a subject in need thereof, is provided according to another embodiment of the present disclosure.

The pharmaceutical composition of the present disclosure, since it can induce an immune response against HTLV-1, can be useful for the prevention and/or treatment of HTLV-1-associated diseases. That is, a pharmaceutical composition for use for the prevention and/or treatment of HTLV-1-associated diseases is provided according to some embodiments of the present disclosure. A method for preventing and/or treating HTLV-1-associated diseases, comprising a step of administering the pharmaceutical composition to a subject in need thereof, is provided according to another embodiment of the present disclosure. Use of the lipid complex or pharmaceutical composition for use in the prevention and/or treatment of HTLV-1-associated diseases is provided according to another embodiment of the present disclosure.

The subject in need may be a subject infected with HTLV-1, a subject determined to be at high risk of infection with HTLV-1, a subject possibly infected with HTLV-1, or a healthy individual not infected with HTLV-1. Also, the subject in need

is a subject determined to be at high risk of development or recurrence of an HTLV-1-associated disease, and a subject who has developed an HTLV-1-associated disease.

In some embodiments, the pharmaceutical composition of the present disclosure may prevent or alleviate at least one symptom caused by HTLV-1 infection in a recipient subject. An example of a symptom of HTLV-1 infection is the onset of ATL. Symptom prevention, for example, can be evaluated subjectively or objectively, and specific examples include self-assessment by the recipient subject; evaluation by a physician; quality of life (QOL) evaluation; and evaluation of the onset of ATL or a delay in the progression of ATL symptoms, or an evaluation of the reduction in the severity of ATL symptoms. The objective evaluation may be an evaluation using an animal or an evaluation by a human.

In some embodiments of the pharmaceutical composition, the nucleic acid is mRNA and the pharmaceutical composition can be used to induce an immune response against HTLV-1 (in particular cellular immunity against HTLV-1 and in particular cellular immunity mediated by cytotoxic T cells). In particular, the pharmaceutical composition according to these embodiments can also be referred to as an mRNA vaccine, an mRNA vaccine composition, or an mRNA vaccine formulation. In some embodiments, the pharmaceutical composition can be used for the prevention and/or treatment of HTLV-1-associated diseases.

The pharmaceutical composition is a vaccine in some embodiments.

In some embodiments of the pharmaceutical composition, the nucleic acid is mRNA and the pharmaceutical composition can be utilized as an mRNA vaccine.

In a particular embodiment, the pharmaceutical composition can be utilized as an mRNA vaccine for the prevention of HTLV-1-associated diseases.

In some embodiments, the pharmaceutical composition of the present disclosure is used for the prevention and/or treatment of ATL. In a particular embodiment, the pharmaceutical composition of the present disclosure is used for the prevention of the onset of ATL, aggravation of ATL, or recurrence of ATL. In a particular embodiment, the nucleic acid comprises a nucleic acid that comprises a polypeptide that encodes at least one selected from Gag p15 or an immunogenic fragment thereof, Gag p19 or an immunogenic fragment thereof, and Gag p24 or an immunogenic fragment thereof.

As described in the examples provided below, it was shown that Gag, which is a structural protein of HTLV-1, is expressed in the cells of ATL patients and that cytotoxic T cells against Gag are present in patients not suffering from a recurrence of ATL, and it was confirmed that an effective immune response against HTLV-1 can be induced by using the Gag of HTLV-1 as an antigen. The present inventors have discovered that a T cell cellular immune response can be induced by the use of a pharmaceutical composition for which the Gag p15, p19, and p24 of HTLV-1 are active ingredients.

As a consequence, the pharmaceutical composition of the present disclosure, for example, is capable of inducing an immune response against HTLV-1.

In some embodiments, the pharmaceutical composition of the present disclosure, for example, is a vaccine for use to prevent the onset of ATL, or the aggravation of ATL, or a recurrence of ATL.

In these embodiments, the recipient subject may be a patient infected with HTLV-1 who has not developed ATL, or who has developed ATL, or who is in remission from ATL. The recipient subject is preferably a subject who has become infected with HTLV-1 and for whom the prevention of ATL onset or ATL recurrence is desired. The recipient subject may be a patient who has received a bone marrow transplant or hematopoietic stem cell transplant after developing ATL.

According to a further embodiment of the present disclosure, there is provided a method for preventing the onset of ATL, aggravation of ATL, or recurrence of ATL, comprising a step of administering the pharmaceutical composition of the present disclosure to a subject in need thereof. The prophylactic method of this embodiment is characterized in that a pharmaceutical composition comprising the lipid complex of the present disclosure is administered to a subject, and other features and conditions are not particularly limited. The prophylactic method of this embodiment - due to the presence as an active ingredient of a nucleic acid that comprises a polypeptide that encodes at least one selected from Gag p15 or an immunogenic fragment thereof, Gag p19 or an immunogenic fragment thereof, and Gag p24 or an immunogenic fragment thereof - can induce an immune response against HTLV-1 when a subject is treated.

Since the prophylactic method of this embodiment, for example, can induce an immune response against HTLV-1, it can also be referred to as, for example, a vaccination method against HTLV-1, a method for inducing protective immunity, a method for stimulating an immune response, or a method for inducing an immune response.

In the prophylactic method of this embodiment, immunity to HTLV-1 can be conferred on a subject by administering the above-described active ingredient to the subject and inducing an immune response in the subject. In the prophylactic method of this embodiment, for example, an immune response against Gag p15 or an immunogenic fragment thereof, Gag p19 or an immunogenic fragment thereof, and/or Gag p24 or an immunogenic fragment thereof is induced in the subject, and a cellular immune response by, e.g., T cells, is produced in the subject against Gag p15 or an immunogenic fragment thereof, Gag p19 or an immunogenic fragment thereof, and/or Gag p24 or an immunogenic fragment thereof. The administration step is performed once or a plurality of times in the prophylactic method of this embodiment.

In some embodiments, the present disclosure provides use of the pharmaceutical composition of the present disclosure for use in methods for preventing the onset of ATL, the aggravation of ATL, or the recurrence of ATL.

The present disclosure includes use of the lipid complex or pharmaceutical composition of the present disclosure for use in the production of a vaccine for use in suppressing the onset of ATL, the aggravation of ATL, or the recurrence of ATL. Examples

The present disclosure is further described in the following through examples, production examples, and test examples, but the present disclosure is not limited to or by these examples.

In the present Description, "room temperature" generally indicates from approximately 10°C to approximately 35°C.

[Example 1: Preparation of LNPs that encapsulate mRNA]

(1) Construction of template DNA

In order to construct the template plasmid DNA used for in vitro transcription (IVT), a plasmid was synthesized having a DNA fragment in which the following were linked in sequence: T7 promoter sequence (TAATACGACTCACTATA, SEQ ID NO: 18), 5'-UTR sequence (AGGAAATAAGAGAGAAAAGAAGAGTAAGAAGAAATATAAGA, SEQ ID NO: 19), Kozak sequence (GCCACC, SEQ ID NO: 20), the particular antigen sequence (Gag antigen, Tax antigen, HBZ antigen), and 3'-UTR sequence

(TAAGCTGCCTTCTGCGGGGCTTGCCTTCTGGCCATGCCCTTCTTCTCTCCCTTGCA
CCTGTACCTCTTGGTCTTTGAATAAAGCCTGAGTAGGAAGGCGG, SEQ ID NO: 21).

The following were added to nuclease-free water (210 μL) in which 1 μg of the plasmid was dissolved: Q5 Hot Start High-Fidelity 2X Master Mix (250 μL, NEB# M0494L), 10 μM sense primer (20 μL), and 10 μM poly-T-containing antisense primer (20 μL). After incubation at 95°C for 1 minute, PCR amplification of the template DNA was carried out by executing 35 cycles of 95°C for 30 seconds, 60°C for 30 seconds, and 72°C for 3 minutes and an additional incubation for 5 minutes at 72°C. After the reaction, purification was performed using a NucleoSpin Gel and PCR Clean Up Kit (TaKaRa #U0609A) to obtain template DNA having the desired antigen sequence (SEQ ID NO: 22, SEQ ID NO: 23, SEQ ID NO: 24).

<SEQ ID NO: 22> Template DNA comprising the sequence for Gag antigen

TAATACGACTCACTATAAGGAAATAAGAGAGAAAAGAAGAGTAAGAAGAAATA
TAAGAGCCACCATGGGCCAGATCTTCTCTCGCTCTGCTTCTCCTATTCCTCGCCCT
CCTCGCGGACTTGCTGCTCACCACTGGCTTAACTTCCTGCAGGCTGCTTACCGCCT
GGAACCTGGCCCTAGCAGCTACGACTTCCACCAGCTGAAGAAGTTCCTGAAGAT
CGCCCTGGAGACCCCCGTGTGGATCTGCCCCATCAACTACAGCCTGCTGGCTAGC
CTGCTGCCTAAAGGCTACCCTGGCCGCGTGAACGAGATCCTGCACATCCTGATCC

AGACCCAGGCCCAGATCCCTTCTCGCCCTGCTCCTCCTCCTTCTTCTCCTACC
CACGATCCCCCCGATAGCGATCCCCAGATCCCCCCTCCTTACGTGGAACCTACCG
CTCCTCAGGTGCTGCCTGTGATGCACCCTCACGGCGCTCCTCCTAATCACCGCCC
TTGGCAGATGAAGGACCTGCAGGCCATCAAACAGGAAGTGAGCCAGGCTGCTCC
CGGCAGCCCCCAGTTTATGCAGACCATCCGCCTGGCTGTGCAGCAGTTCGATCCC
ACCGCTAAAGATCTGCAGGACCTGCTGCAGTACCTGTGCAGCAGCCTGGTGGCT
AGCCTGCACCACCAGCAGCTGGATAGCCTGATCAGCGAAGCCGAAACCCGCGGC
ATCACCGGCTACAATCCTCTGGCTGGCCCTCTGCGCGTGCAGGCTAACAACCCTC
AGCAGCAGGGACTGCGCCGCGAATACCAGCAGCTGTGGCTGGCTGCTTTTGCTG
CTCTGCCCGGCAGCGCTAAAGATCCCAGCTGGGCTAGCATCCTGCAGGGCCTGG
AAGAACCCTACCACGCCTTCGTGGAGCGCCTGAACATCGCCCTGGACAACGGCC
TGCCTGAAGGCACCCCTAAAGATCCTATCCTGCGCAGCCTGGCCTACAGCAACGC
CAACAAGGAGTGCCAGAAGCTGCTGCAGGCTCGCGGACACACCAATAGCCCTCT
GGGAGATATGCTGCGCGCTTGCCAGACCTGGACCCCCAAGGACAAGACCAAGGT
GCTGGTGGTGCAGCCCAAGAAGCCTCCTCCTAACCAGCCTTGCTTTCGCTGCGGC
AAAGCTGGACACTGGAGCCGCGATTGCACACAGCCTCGCCCTCCTCCTGGACCTT
GCCCTCTTTGCCAGGATCCTACCCACTGGAAGCGCGACTGCCCTCGCCTGAAACC
TACCATCCCTGAACCTGAACCTGAAGAAGACGCTCTGCTGCTGGATCTGCCCGCT
GATATCCCCCACCCCAAGAACAGCATCGGCGGCGAGGTGTGATAAGCTGCCTTC
TGCGGGGCTTGCCTTCTGGCCATGCCCTTCTTCTCTCCCTTGCACCTGTACCTCTT
GGTCTTTGAATAAAGCCTGAGTAGGAAGGCGG

<SEQ ID NO: 23> Template DNA comprising the sequence for Tax antigen

TAATACGACTCACTATAAGGAAATAAGAGAGAAAAGAAGAGTAAGAAGAAATA
TAAGAGCCACCATGGCCCACTTCCCCGGCTTTGGCCAGAGCCTGCTGTTTGGCTA
CCCCGTGTACGTGTTCGGCGATTGCGTGCAGGGCGATTGGTGCCCCATCTCTGGA

GGACTGTGCTCTGCTCGCCTTCACCGCCACGCACTTCTTGCTACATGCCCTGAGC
ACCAGATCACCTGGGATCCCATCGATGGCCGCGTGATCGGCAGCGCTCTGCAGTT
CCTGATCCCTCGCCTGCCTAGCTTTCCTACCCAGCGCACCAGCAAAACCCTGAAG
GTGCTGACCCCTCCTATCACCCACACCACCCCTAACATCCCTCCTAGCTTCCTGCA
GGCCATGCGCAAGTACAGCCCCTTCCGCAACGGCTACATGGAACCCACCCTGGG
CCAGCACCTGCCTACCCTGAGCTTTCCTGATCCTGGACTGCGCCCTCAGAATCTG
TACACCCTGTGGGGAGGAAGCGTGGTGTGCATGTACCTGTACCAGCTGAGCCCTC
CTATCACCTGGCCTCTGCTGCCTCACGTGATCTTTTGCCACCCTGGCCAGCTGGGC
GCTTTTCTGACCAACGTGCCTTACAAGCGCATCGAGGAGCTGCTGTACAAGATCA
GCCTGACCACCGGCGCCCTGATCATCCTGCCCGAAGATTGCCTGCCCACCACCCT
GTTTCAGCCTGCTCGCGCTCCTGTGACACTGACAGCTTGGCAGAACGGCCTGCTG
CCTTTTCACAGCACCCTGACCACCCCTGGCCTGATCTGGACCTTCACCGATGGCA
CCCCCATGATCAGCGGCCCTTGCCCTAAAGATGGCCAGCCTAGCCTGGTGCTGCA
GAGCAGCAGCTTCATCTTCCACAAGTTCCAGACCAAGGCCTACCACCCCAGCTTC
CTGCTGAGCCACGGCCTGATCCAGTACAGCAGCTTCCACAGCCTGCACCTGCTGT
TCGAGGAGTACACCAACATCCCCATCAGCCTGCTGTTCAACGAGAAGGAGGCCG
ACGACAACGACCACGAGCCTCAGATCAGCCCTGGCGGCCTGGAACCTCCTAGCG
AAAAGCACTTCCGCGAGACCGAGGTGTGATAAGCTGCCTTCTGCGGGGCTTGCCT
TCTGGCCATGCCCTTCTTCTCTCCCTTGCACCTGTACCTCTTGGTCTTTGAATAAA
GCCTGAGTAGGAAGGCGG

<SEQ ID NO: 24> Template DNA comprising the sequence for HBZ antigen

TAATACGACTCACTATAAGGAAATAAGAGAGAAAAGAAGAGTAAGAAGAAATA
TAAGAGCCACCATGGCTGCTAGCGGCCTGTTTCGCTGCCTGCCTGTGAGCTGCCC
TGAAGACCTGCTGGTGGAGGAACTGGTGGACGGCCTGCTGAGCCTGGAAGAAGA
ACTGAAGGACAAGGAGGAGGAAGAAGCCGTGCTGGACGGCCTGCTGAGCCTGG

AAGAAGAATCTCGCGGACGCCTTCGCCGCGGACCTCCTGGAGAAAAAGCTCCTC
CTAGAGGAGAAACACACAGAGATAGACAAAGAAAAGCTGAGGAGAAGCGCAAG
CGCAAGAAGGAGCGCGAGAAGGAGGAGGAGAAGCAGATCGCCGAGTACCTGAA
GCGCAAAGAAGAAGAAAAAGCTCGCCGCCGCCGCCGCGCTGAAAAAAAAGCTG
CTGATGTGGCCCGCCGCAAACAGGAAGAACAGGAACGCCGCGAACGCAAATGG
CGCCAGGGCGCTGAAAAAGCTAAGCAGCACAGCGCCCGCAAGGAGAAGATGCA
GGAACTGGGCATCGACGGCTACACCCGCCAGCTGGAAGGCGAAGTGGAAAGCCT
GGAAGCCGAACGCCGCAAGCTGCTGCAGGAGAAGGAGGACCTGATGGGCGAGG
TGAACTACTGGCAGGGCCGCCTGGAAGCCATGTGGCTGCAGTGATAAGCTGCCT
TCTGCGGGGCTTGCCTTCTGGCCATGCCCTTCTTCTCTCCCTTGCACCTGTACCTC
TTGGTCTTTGAATAAAGCCTGAGTAGGAAGGCGG

(2) Preparation of mRNA

mRNA was prepared by in vitro transcription (IVT) using each of the obtained template DNAs. 300 μg/mL template DNA (80 μL), 100 mM CleanCap AG (32 μL, TriLink catalog #N-7113), 100 mM ATP (40 μL, TriLink catalog #N-1510), 100 mM CTP (40 μL, TriLink catalog #N-1511), 100 mM GTP (40 μL, TriLink catalog #N-1512), 100 mM N1-methyl-ψ-Uridine-5'-Triphosphate (40 μL), UltraPure DNase/RNase-Free Distilled Water (405.6 μL, Thermo Fisher catalog #10977015), T7 Transcription 10x buffer (80 μL), RNase inhibitor (20 μL, NEB, catalog #M0314L), Yeast Inorganic pyrophosphatase (16 μL, NEB, catalog #M2403L), and T7 RNA Polymerase (6.4 μL, Roche catalog #08140669103) were mixed and incubated at 37°C for 3 hours. RNase-Free DNase I (24 μL, TaKaRa catalog #2270A) was mixed in and incubation was carried out for 30 minutes at 37°C. 10x phosphatase buffer (96 μL, NEB, catalog #B0289S) and Antarctic phosphatase (48 μL, NEB, catalog #M0289L) were additionally mixed in and incubation was carried out for 30 minutes at 37°C. 8 M LiCl solution (484 μL, Sigma-Aldrich catalog catalog #L7026) was mixed in and, after standing for at least one hour at -20°C, centrifugation was carried out (4°C, 15,000 rpm, 30 minutes) and the supernatant was discarded. The following operation was then carried out three times: addition of 75% ethanol, centrifugation (4°C, 15,000 rpm, 5 minutes), and discard of the supernatant. The resulting precipitate was dissolved in nuclease-free water and purified using an RNeasy Maxi kit (Qiagen catalog #75162) in accordance with the accompanying manual. Alternatively, the resulting precipitate was treated with RNase III (NEB, catalog #M0245S) for 10 minutes at room temperature and was then purified with an Oligo dT column (Sartorius, catalog #311.1218-2). As a result, mRNAs (SEQ ID NO: 2, SEQ ID NO: 5, SEQ ID NO: 9) expressing proteins having the respective amino acid sequences (SEQ ID NO: 1, SEQ ID NO: 4, SEQ ID NO: 8) were obtained.

A cap structure and polyA sequence were added to each mRNA. The cap structure is the structure given below for CleanCap (Cap1) (Fujifilm Wako Pure Chemical Industries, Ltd.).

[C7]

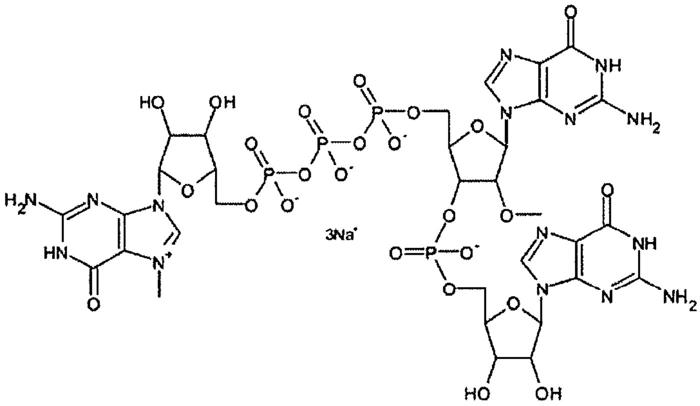

<SEQ ID NO: 2> Gag mRNA

AGGAAAUAAGAGAGAAAAGAAGAGUAAGAAGAAAUAUAAGAGCCACCAUGGG
CCAGAUCUUCUCUCGCUCUGCUUCUCCUAUUCCUCGCCCUCCUCGCGGACUUG
CUGCUCACCACUGGCUUAACUUCCUGCAGGCUGCUUACCGCCUGGAACCUGGC
CCUAGCAGCUACGACUUCCACCAGCUGAAGAAGUUCCUGAAGAUCGCCCUGGA

GACCCCCGUGUGGAUCUGCCCCAUCAACUACAGCCUGCUGGCUAGCCUGCUGC
CUAAAGGCUACCCUGGCCGCGUGAACGAGAUCCUGCACAUCCUGAUCCAGACC
CAGGCCCAGAUCCCUUCUCGCCCUGCUCCUCCUCCUCCUUCUUCUCCUACCCAC
GAUCCCCCCGAUAGCGAUCCCCAGAUCCCCCCUCCUUACGUGGAACCUACCGC
UCCUCAGGUGCUGCCUGUGAUGCACCCUCACGGCGCUCCUCCUAAUCACCGCC
CUUGGCAGAUGAAGGACCUGCAGGCCAUCAAACAGGAAGUGAGCCAGGCUGCU
CCCGGCAGCCCCCAGUUUAUGCAGACCAUCCGCCUGGCUGUGCAGCAGUUCGA
UCCCACCGCUAAAGAUCUGCAGGACCUGCUGCAGUACCUGUGCAGCAGCCUGG
UGGCUAGCCUGCACCACCAGCAGCUGGAUAGCCUGAUCAGCGAAGCCGAAACC
CGCGGCAUCACCGGCUACAAUCCUCUGGCUGGCCCUCUGCGCGUGCAGGCUAA
CAACCCUCAGCAGCAGGGACUGCGCCGCGAAUACCAGCAGCUGUGGCUGGCUG
CUUUUGCUGCUCUGCCCGGCAGCGCUAAAGAUCCCAGCUGGGCUAGCAUCCUG
CAGGGCCUGGAAGAACCCUACCACGCCUUCGUGGAGCGCCUGAACAUCGCCCU
GGACAACGGCCUGCCUGAAGGCACCCCUAAAGAUCCUAUCCUGCGCAGCCUGG
CCUACAGCAACGCCAACAAGGAGUGCCAGAAGCUGCUGCAGGCUCGCGGACAC
ACCAAUAGCCCUCUGGGAGAUAUGCUGCGCGCUUGCCAGACCUGGACCCCCAA
GGACAAGACCAAGGUGCUGGUGGUGCAGCCCAAGAAGCCUCCUCCUAACCAGC
CUUGCUUUCGCUGCGGCAAAGCUGGACACUGGAGCCGCGAUUGCACACAGCCU
CGCCCUCCUCCUGGACCUUGCCCUCUUUGCCAGGAUCCUACCCACUGGAAGCG
CGACUGCCCUCGCCUGAAACCUACCAUCCCUGAACCUGAACCUGAAGAAGACG
CUCUGCUGCUGGAUCUGCCCGCUGAUAUCCCCCACCCCAAGAACAGCAUCGGC
GGCGAGGUGUGAUAAGCUGCCUUCUGCGGGGCUUGCCUUCUGGCCAUGCCCUU
CUUCUCUCCCUUGCACCUGUACCUCUUGGUCUUUGAAUAAAGCCUGAGUAGGA
AGGCGGAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAA

AAAAAAAAAAAAAAAAAAAAAA

<SEQ ID NO: 5> Tax mRNA

AGGAAAUAAGAGAGAAAAGAAGAGUAAGAAGAAAUAUAAGAGCCACCAUGGC
CCACUUCCCCGGCUUUGGCCAGAGCCUGCUGUUUGGCUACCCCGUGUACGUGU
UCGGCGAUUGCGUGCAGGGCGAUUGGUGCCCCAUCUCUGGAGGACUGUGCUCU
GCUCGCCUUCACCGCCACGCACUUCUUGCUACAUGCCCUGAGCACCAGAUCAC
CUGGGAUCCCAUCGAUGGCCGCGUGAUCGGCAGCGCUCUGCAGUUCCUGAUCC
CUCGCCUGCCUAGCUUUCCUACCCAGCGCACCAGCAAAACCCUGAAGGUGCUG
ACCCCUCCUAUCACCCACACCACCCCUAACAUCCCUCCUAGCUUCCUGCAGGCC
AUGCGCAAGUACAGCCCCUUCCGCAACGGCUACAUGGAACCCACCCUGGGCCA
GCACCUGCCUACCCUGAGCUUUCCUGAUCCUGGACUGCGCCCUCAGAAUCUGU
ACACCCUGUGGGGAGGAAGCGUGGUGUGCAUGUACCUGUACCAGCUGAGCCCU
CCUAUCACCUGGCCUCUGCUGCCUCACGUGAUCUUUUGCCACCCUGGCCAGCU
GGGCGCUUUUCUGACCAACGUGCCUUACAAGCGCAUCGAGGAGCUGCUGUACA
AGAUCAGCCUGACCACCGGCGCCCUGAUCAUCCUGCCCGAAGAUUGCCUGCCC
ACCACCCUGUUUCAGCCUGCUCGCGCUCCUGUGACACUGACAGCUUGGCAGAA
CGGCCUGCUGCCUUUUCACAGCACCCUGACCACCCCUGGCCUGAUCUGGACCU
UCACCGAUGGCACCCCCAUGAUCAGCGGCCCUUGCCCUAAAGAUGGCCAGCCU
AGCCUGGUGCUGCAGAGCAGCAGCUUCAUCUUCCACAAGUUCCAGACCAAGGC
CUACCACCCCAGCUUCCUGCUGAGCCACGGCCUGAUCCAGUACAGCAGCUUCC
ACAGCCUGCACCUGCUGUUCGAGGAGUACACCAACAUCCCCAUCAGCCUGCUG
UUCAACGAGAAGGAGGCCGACGACAACGACCACGAGCCUCAGAUCAGCCCUGG
CGGCCUGGAACCUCCUAGCGAAAAGCACUUCCGCGAGACCGAGGUGUGAUAAG
CUGCCUUCUGCGGGGCUUGCCUUCUGGCCAUGCCCUUCUUCUCUCCCUUGCAC
CUGUACCUCUUGGUCUUUGAAUAAAGCCUGAGUAGGAAGGCGGAAAAAAAAA

AAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAA

AAAAAAA

<SEQ ID NO: 9> HBZ mRNA

AGGAAAUAAGAGAGAAAAGAAGAGUAAGAAAUAUAAGAGCCACCAUGGC

UGCUAGCGGCCUGUUUCGCUGCCUGCCUGUGAGCUGCCCUGAAGACCUGCUGG

UGGAGGAACUGGUGGACGGCCUGCUGAGCCUGGAAGAAGAACUGAAGGACAA

GGAGGAGGAAGAAGCCGUGCUGGACGGCCUGCUGAGCCUGGAAGAAGAAUCU

CGCGGACGCCUUCGCCGCGGACCUCCUGGAGAAAAAGCUCCUCCUAGAGGAGA

AACACACAGAGAUAGACAAAGAAAAGCUGAGGAGAAGCGCAAGCGCAAGAAG

GAGCGCGAGAAGGAGGAGGAGAAGCAGAUCGCCGAGUACCUGAAGCGCAAAG

AAGAAGAAAAAGCUCGCCGCCGCCGCCGCGCUGAAAAAAAAGCUGCUGAUGUG

GCCCGCCGCAAACAGGAAGAACAGGAACGCCGCGAACGCAAAUGGCGCCAGGG

CGCUGAAAAAGCUAAGCAGCACAGCGCCCGCAAGGAGAAGAUGCAGGAACUGG

GCAUCGACGGCUACACCCGCCAGCUGGAAGGCGAAGUGGAAAGCCUGGAAGCC

GAACGCCGCAAGCUGCUGCAGGAGAAGGAGGACCUGAUGGGCGAGGUGAACU

ACUGGCAGGGCCGCCUGGAAGCCAUGUGGCUGCAGUGAUAAGCUGCCUUCUGC

GGGGCUUGCCUUCUGGCCAUGCCCUUCUUCUCUCCCUUGCACCUGUACCUCUU

GGUCUUUGAAUAAAGCCUGAGUAGGAAGGCGGAAAAAAAAAAAAAAAAAA

AAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAA

<SEQ ID NO: 1> Amino acid sequence of Gag

MGQIFSRSASPIPRPPRGLAAHHWLNFLQAAYRLEPGPSSYDFHQLKKFLKIALETPV

WICPINYSLLASLLPKGYPGRVNEILHILIQTQAQIPSRPAPPPPSSPTHDPPDSDPQIPPP

YVEPTAPQVLPVMHPHGAPPNHRPWQMKDLQAIKQEVSQAAPGSPQFMQTIRLAVQ

QFDPTAKDLQDLLQYLCSSLVASLHHQQLDSLISEAETRGITGYNPLAGPLRVQANN
PQQQGLRREYQQLWLAAFAALPGSAKDPSWASILQGLEEPYHAFVERLNIALDNGLP
EGTPKDPILRSLAYSNANKECQKLLQARGHTNSPLGDMLRACQTWTPKDKTKVLVV
QPKKPPPNQPCFRCGKAGHWSRDCTQPRPPPGPCPLCQDPTHWKRDCPRLKPTIPEPE
PEEDALLLDLPADIPHPKNSIGGEV

<SEQ ID NO: 4> Amino acid sequence of Tax

MAHFPGFGQSLLFGYPVYVFGDCVQGDWCPISGGLCSARLHRHALLATCPEHQITW
DPIDGRVIGSALQFLIPRLPSFPTQRTSKTLKVLTPPITHTTPNIPPSFLQAMRKYSPFRN
GYMEPTLGQHLPTLSFPDPGLRPQNLYTLWGGSVVCMYLYQLSPPITWPLLPHVIFC
HPGQLGAFLTNVPYKRIEELLYKISLTTGALIILPEDCLPTTLFQPARAPVTLTAWQNG
LLPFHSTLTTPGLIWTFTDGTPMISGPCPKDGQPSLVLQSSSFIFHKFQTKAYHPSFLLS
HGLIQYSSFHSLHLLFEEYTNIPISLLFNEKEADDNDHEPQISPGGLEPPSEKHFRETEV

<SEQ ID NO: 8> Amino acid sequence of HBZ

MAASGLFRCLPVSCPEDLLVEELVDGLLSLEEELKDKEEEEAVLDGLLSLEEESRGRL
RRGPPGEKAPPRGETHRDRQRRAEEKRKRKKEREKEEEKQIAEYLKRKEEEKARRR
RRAEKKAADVARRKQEEQERRERKWRQGAEKAKQHSARKEKMQELGIDGYTRQL
EGEVESLEAERRKLLQEKEDLMGEVNYWQGRLEAMWLQ

(3) Preparation of mRNA-encapsulating LNPs

Each mRNA was dissolved in 50 mM sodium citrate (pH 3.5) to prepare an mRNA dilution. 2-{9-oxo-9-[(3-pentyloctyl)oxy]nonyl} dodecyl 1-methylpiperidine-4-carboxylate (TS202), DPPC (Nippon Fine Chemical), cholesterol (Nippon Fine Chemical), and MPEG2000-DMG (NOF Corp.) were dissolved in ethanol at a molar ratio of 47 / 11 / 40.5 / 1.5 to prepare a lipid solution. Lipid nanoparticles (LNPs) were obtained by mixing the mRNA dilution and lipid solution at a flow rate for each of 3:1 as the volume ratio, using a weight ratio of 0.05 for the ratio between the mRNA and lipid. The resulting LNP aqueous solution was dialyzed using a Float-A-Lyzer G2 (SPECTRUM, 100K MWCO) to exchange the external solution to PBS followed by exchange to a sucrose solution. After concentration, the solution was sterilized by filtration and the formulation quality of the LNPs was evaluated.

The 2-{9-oxo-9-[(3-pentyloctyl)oxy]nonyl}dodecyl 1-methylpiperidine-4-carboxylate was synthesized according to Example A-2 of WO 2017/222016.

The mRNA concentration and encapsulation rate were measured using Quant-iT RiboGreen RNA Reagent (Invitrogen, Cat. #R11491). Specifically, the encapsulation rate was calculated using the mRNA concentration (A) measured after dilution with RNase-free water as the mRNA present in the LNP external solution and the mRNA concentration (B) measured after dilution with 1% Triton X-100 as the total mRNA concentration in the formulation.

$$\text{Encapsulation rate (\%)} = 100 - (A/B) \times 100$$

The average particle diameter (Z-average) and polydispersity index (PDI) of the LNPs were measured using a particle size analyzer (Malvern, Zetasizer Nano ZS).

The results of the evaluation of the formulation quality of the prepared LNPs are shown in Table 1.

[Table 1]

Table 1

| LNP ID | mRNA | antigen | average particle diameter (nm) | PDI | encapsulation rate (%) |
|---|---|---|---|---|---|
| #01 | SEQ ID NO: 2 | Gag | 100 | 0.04 | 93% |
| #02 | SEQ ID NO: 5 | Tax | 96 | 0.01 | 92% |
| #03 | SEQ ID NO: 9 | HBZ | 96 | 0.03 | 90% |
| #04 | SEQ ID NO: 2 | Gag | 102 | 0.02 | 96% |
| #05 | SEQ ID NO: 5 | Tax | 101 | 0.02 | 96% |
| #06 | SEQ ID NO: 9 | HBZ | 96 | 0.01 | 96% |

These results demonstrate that lipid nanoparticles (LNPs) of the present disclosure were obtained in which mRNA encoding Gag protein or an immunogenic fragment thereof, mRNA encoding Tax protein or an immunogenic fragment thereof, and mRNA encoding HBZ protein or an immunogenic fragment thereof were each encapsulated in lipid.

The encapsulation rate for all the LNPs was confirmed to be greater than 90%, demonstrating a high mRNA encapsulation rate. The polydispersity index (PDI) was also less than 0.05.

[Example 2: Investigation of the cellular immune response in mice]

(1) Mouse immunization and splenocyte preparation

C57BL/6 mice (female, 8 weeks old, n = 4 per Gag group; n = 3 per Tax or HBZ group) were inoculated intramuscularly in the thigh on days 0 and 5 with LNP encapsulating mRNA for the individual antigen at 10 μg mRNA/mouse. The mice were euthanized on day 12 and their spleens were removed. The spleens were pulverized on a cell strainer using the plunger of a sterile syringe and were washed with 10 mL RPMI and the cell suspension was collected. The cell suspension was centrifuged, the supernatant was removed, and the cells were hemolyzed with 1 mL of $NH_4Cl$ for 2 minutes. The reaction was then stopped with 9 mL RPMI and centrifugation was carried out. The cell pellet was resuspended in 20 mL RPMI with 10% FBS.

(2) Analysis of the cellular immunity response

The ability to induce a cellular immune response was evaluated using an ELISpot assay. The peptides used to stimulate the cells were designed and pooled using the following method.

Overlapping peptides with a length of 9 amino acids (offset: 1 amino acid) were designed based on the amino acid sequence of Gag (GenBank accession number AAA85841.1) (SEQ ID NO: 1). 97 peptides for Gag p15 were pooled as Gag-1 (p15-1 ($p15_{1-50}$)) and Gag-2 (p15-2 ($p15_{51-97}$)). 121 peptides for Gag p19 were pooled as Gag-3 (p19-1 ($p19_{1-50}$)), Gag-4 (p19-2 ($p19_{51-100}$)), and Gag-5 (p19-3 ($p19_{101-121}$)). For the Gag p24 peptides, 206 peptides were pooled as Gag-6 (p24-1 ($p24_{1-51}$)), Gag-7 (p24-2 ($p24_{52-103}$)), Gag-8 (p24-3 ($p24_{104-154}$)), and Gag-9 (p24-4 ($p24_{155-206}$)). Similarly, for Tax, 345 peptides were pooled as Tax-1 ($Tax_{1-50}$), Tax-2 ($Tax_{51-100}$), Tax-3 ($Tax_{101-150}$), Tax-4 ($Tax_{151-200}$), Tax-5 ($Tax_{201-250}$), Tax-6 ($Tax_{251-300}$), and Tax-7 ($Tax_{301-345}$). Similarly, for HBZ, 198 peptides were pooled as HBZ-1 ($HBZ_{1-50}$), HBZ-2 ($HBZ_{51-100}$), HBZ-3 ($HBZ_{101-150}$), and HBZ-4 ($HBZ_{151-198}$).

The ELISpot assay was performed in accordance with the following procedure using ELISpot Flex: IFN-γ, Mouse-ALP from MABTECH. A Millipore MAIPS4510 assay plate was treated with the coating antibody, and the prepared mouse splenocytes were seeded at $10^6$ cells/well. After stimulation for 24 hours with 1 μM of a peptide pool as described above, the IFN-γ-producing cells were stained with the detection antibody. After staining, the spots were analyzed using an ImmunoSpot S6 ENTRY Analyzer from Cellular Technology Limited and the number of spots was counted.

Figs. 1 to 3 report results showing the number of spots for each peptide pool. Fig. 1 gives the results for the Gag peptide pools; Fig. 2 gives the results for the peptide pools; and Fig. 3 gives the results for the HBZ peptide pools. "No

peptide" gives the results for the negative control (no peptide stimulation), and "Positive Control" gives the results for the positive control (stimulation by PMA/ionomycin, which is a mitogen). In Fig. 1, Gag-1 and Gag-2 correspond to p15-1 and p15-2; Gag-3 to Gag-5 correspond to p19-1 to p19-3; and Gag-6 to Gag-9 correspond to p24-1 to p24-4.

As shown in Fig. 1, spots were formed with the Gag p19 (Gag-3 to Gag-5), Gag p15 (Gag-1 and Gag-2) or Gag p24 (Gag-6 to Gag-9) peptide pools. Substantial spot formation occurred in particular with Gag p19-1 (Gag-3), p19-2 (Gag-4), and p19-3 (Gag-5) (particularly p19-1 (Gag-3) and p19-2 (Gag-4)). These results demonstrated that IFN-γ was produced in mice that had been immunized with LNPs that encapsulated mRNA encoding the Gag protein. This production of IFN-γ suggested that a cellular immune response had been induced.

As shown in Fig. 2, spot formation occurred with the Tax peptide pools. Substantial spot formation occurred in particular with the Tax-4 or Tax-6 (particularly Tax-6) peptide pool. These results demonstrated that IFN-γ was produced in mice that had been immunized with LNPs that encapsulated mRNA encoding the Tax protein. In addition, this production of IFN-γ suggested that a cellular immune response had been induced.

As shown in Fig. 3, spot formation occurred with the HBZ peptide pools. Substantial spot formation occurred in particular with the HBZ-1 peptide pool. These results demonstrated that IFN-γ was produced in mice that had been immunized with LNPs that encapsulated mRNA encoding the HBZ protein. In addition, this production of IFN-γ suggested that a cellular immune response had been induced.

The induction of cellular immunity (IFN-γ production) could be confirmed based on these results, suggesting that cellular immunity can also be induced and antitumor effects can be achieved in cancer-transplanted animal models.

Molecular Therapy 2024, 32(3): 704-721. Lymph node macrophages drive innate immune responses to enhance the anti-tumor efficacy of mRNA vaccines;
Molecular Therapy 2021, 29(7): 2227-2238. mRNA-encoded, constitutively active STINGV155M is a potent genetic adjuvant of antigen-specific CD8+ T cell response; Nature Biotechnology 2019, 37(10): 1174-1185. Delivery of mRNA vaccines with heterocyclic lipids increases anti-tumor efficacy by STING-mediated immune cell activation

[Example 3]

An experimental system based on a syngeneic mouse model is established in order to analyze the efficacy of a Gag mRNA vaccine using an animal model. EL4, a T-cell lymphoma line derived from an inbred C57BL/6 mouse strain, can form tumors by subcutaneous transplantation into wild-type C57BL/6 mice. EL4-Gag - an EL4 subline expressing the gag gene, which is one of the HTLV-1 antigens - is established as follows. First, the gene sequence encoding the full-length Gag protein is amplified by RT-PCR and is inserted into the multicloning site of the PiggyBac Transposon Vector System (System Biosciences, PB-CMV-EF1-Puro) to construct a plasmid that expresses full-length Gag. Gene introduction into EL4 of the Gag-expression PiggyBac vector and a transposase expression vector is performed by lipofection, and EL4 (EL4-Gag), which stably expresses Gag, is then established by selection by the addition of puromycin to the culture medium. Gag expression in EL4-Gag is confirmed by real-time PCR. Next, wild-type C57BL/6 mice (n = 4/group) are injected twice (for example, on day 1 and day 5) intramuscularly into the thigh muscle with the Gag mRNA vaccine at a dose of 10 μg mRNA/mouse. For the experimental control, mice are also vaccinated twice with physiological saline using the same protocol. After the second vaccination (for example, the day after vaccination to two weeks later, day 6 to day 19), $1 \times 10^6$ EL4-Gag cells are subcutaneously transplanted into the mice. Subsequently, on a twice a week schedule, the mice are weighed, tumor survival is confirmed, and the tumor diameter is measured. The tumor volume is calculated using the following formula.

$$\text{tumor volume} = 1/2 * (\text{length} \times \text{width}^2)$$

The mice are weighed, the presence/absence of a tumor is confirmed, and the volume is measured when a tumor is visible.

[Reference Example 1]

(1) Investigation of HTLV-1 antigens

Serum or plasma from HTLV-1-infected individuals, ATL patients, and so forth was used to study antibody responses with HTLV-1 antigens. Specifically, a luciferase immunoprecipitation procedure (LIPS assay) was carried out. Serum or plasma was obtained from HTLV-1-infected individuals, ATL patients, HAM/TSP patients, and HTLV-1-infection-free individuals. All clinical samples were collected after informed consent was obtained in writing in accordance with the Helsinki Declaration. The experiments described in the example were approved by the Institutional Ethics Committee of Kumamoto University (accession numbers: G489, G499, and E2214). The antigens used were Tax (SEQ ID NO: 4), Env

(SEQ ID NO: 25), Gag p15 (SEQ ID NO: 11), Gag p19 (SEQ ID NO: 13), and Gag p24 (SEQ ID NO: 14). Vectors expressing a fusion protein between the particular protein and NanoLuc were fabricated; after this fabrication, the vectors were introduced into a 293T cell line; and the cell lysate of these cells was used as the antigen. A preparation was carried out by mixing antigen corresponding to $10^7$ counts, LIPS buffer, and 1 μL of the serum or plasma on a 96-well plate to provide a total of 100 μL. After this preparation, the plate was incubated using a plate mixer and using conditions of room temperature (hereinafter, approximately 24°C) for 30 minutes. After this incubation, Protein A/G magnetic beads (Thermo Fisher Scientific) were washed 3 to 5 times with LIPS buffer using a magnetic plate. After washing, the magnetic beads were resuspended in LIPS buffer. After resuspension, 10 μL of the magnetic beads was added to each well of the plate. After this addition, the plate was incubated using a plate mixer under conditions of room temperature for 30 minutes. After the incubation, in order to concentrate the immune complexes consisting of the antibodies in the patient sample and the NanoLuc-viral antigen fusion protein, the plate was placed on a magnetic plate and the pellet in each well was washed five times with 150-200 μL of LIPS buffer. After the washing, the LIPS buffer used for washing was removed, and 100 μL of Nano-Glo luciferase assay substrate (Promega) was added to each well. After the addition, the luminescence was measured using a luminometer (Promega). The results are reported in Fig. 1.

Amino acid sequence of Tax (SEQ ID NO: 4)

MAHFPGFGQSLLFGYPVYVFGDCVQGDWCPISGGLCSARLHRHALLATCPEHQITW
DPIDGRVIGSALQFLIPRLPSFPTQRTSKTLKVLTPPITHTTPNIPPSFLQAMRKYSPFRN
GYMEPTLGQHLPTLSFPDPGLRPQNLYTLWGGSVVCMYLYQLSPPITWPLLPHVIFC
HPGQLGAFLTNVPYKRIEELLYKISLTTGALIILPEDCLPTTLFQPARAPVTLTAWQNG
LLPFHSTLTTPGLIWTFTDGTPMISGPCPKDGQPSLVLQSSSFIFHKFQTKAYHPSFLLS
HGLIQYSSFHSLHLLFEEYTNIPISLLFNEKEADDNDHEPQISPGGLEPPSEKHFRETEV

Amino acid sequence of Env (SEQ ID NO: 25)

GKFLATLILFFQFCPLIFGDYSPSCCTLTIGVSSYHSKPCNPAQPVCSWTLDLLALSAD
QALQPPCPNLVSYSSYHATYSLYLFPHWIKKPNRNGGGYYSASYSDPCSLKCPYLGC
QSWTCPYTGAVSSPYWKFQHDVNFTQEVSRLNINLHFSKCGFPFSLLVDAPGYDPIW
FLNTEPSQLPPTAPPLLPHSNLDHILEPSIPWKSKLLTLVQLTLQSTNYTCIVCIDRASL
STWHVLYSPNVSVPSSSSTPLLYPSLALPAPHLTLPFNWTHCFDPQIQAIVSSPCHNSLI
LPPFSLSPVPTLGSRSRRAVPVAVWLVSALAMGAGVAGGITGSMSLASGKSLLHEVD
KDISQLTQAIVKNHKNLLKIAQYAAQNRRGLDLLFWEQGGLCKALQEQCCFLNITNS
HVSILQERPPLENRVLTGWGLNWDLGLSQWAREALQTGITLVALLLLVILAGPCILR
QLRHLPSRVRYPHYSLINPESSL

Amino acid sequence of Gag p15 (SEQ ID NO: 11)

VVQPKKPPPNQPCFRCGKAGHWSRDCTQPRPPPGPCPLCQDPTHWKRDCPRLKPTIP

EPEPEEDALLLDLPADIPHPKNLHRGGGLTSPPTLQQVLPNQDPASIL

Amino acid sequence of Gag p19 (SEQ ID NO: 13)

MGQIFSRSASPIPRPPRGLAAHHWLNFLQAAYRLEPGPSSYDFHQLKKFLKIALETPV

WICPINYSLLASLLPKGYPGRVNEILHILIQTQAQIPSRPAPPPPSSPTHDPPDSDPQIPPP

YVEPTAPQVL

Amino acid sequence of Gag p24 (SEQ ID NO: 14)

PVMHPHGAPPNHRPWQMKDLQAIKQEVSQAAPGSPQFMQTIRLAVQQFDPTAKDL

QDLLQYLCSSLVASLHHQQLDSLISEAETRGITGYNPLAGPLRVQANNPQQQGLRRE

YQQLWLAAFAALPGSAKDPSWASILQGLEEPYHAFVERLNIALDNGLPEGTPKDPIL

RSLAYSNANKECQKLLQARGHTNSPLGDMLRACQTWTPKDKTKVL

Fig. 4 contains graphs that report the results of the LIPS assays. In Fig. 4, the horizontal axis indicates the type of subject and the vertical axis indicates the luminescence intensity. Fig. 4(A) shows the antibody response to Tax, Fig. 4(B) shows the antibody response to Env, Fig. 4(C) shows the antibody response to Gag p15, Fig. 4(D) shows the antibody response to Gag p19, and Fig. 4(E) shows the antibody response to Gag p24. As shown in Fig. 4, it was found that the antibody responses to Tax and Env were lower in ATL patients than in HTLV-1-infected individuals. It was also found that the antibody responses to Gag p19 and Gag p24 were higher in ATL patients than in HTLV-1-infected individuals.

(2) Investigation of Gag expression

In Reference Example 1(1), the ATL patients had low antibody responses to Tax and high antibody responses to Gag, and the gene expression of Tax and Gag was examined as a consequence. Specifically, quantitative reverse transcription PCR (RT-qPCR) was carried out. For the HTLV-1 cases, MT1 cells, MT4 cells, 55T(+) cells, ED cells, and TLOm1 cells, which are ATL cell lines, and Jurkat cells, an HTLV-1-negative T cell line, were used. For the ATL cases, MT1 cells and sera collected from ATL patients (ATL-1, ATL-2, ATL-3, ATL-4, and ATL-5) were used. The total RNA was extracted from each of these cells using TRIzol Reagent (Invitrogen). After extraction, cDNA was synthesized using SuperScript IV Reverse Transcriptase (Invitrogen). Gag cDNA was prepared using the Gag cDNA synthesis primers indicated below. Tax cDNA and 18SrRNA were synthesized using random primers. After the synthesis, Gag expression was measured using Taqman real-time PCR. Tax expression was measured using SYBR-green real-time PCR. The 18SrRNA was quantified using Taqman Gene Expression Assays (Hs99999901_s1, Applied Biosystems). The amplification conditions were conditions for 1 cycle = 50°C for 2 minutes, 95°C for 10 minutes, 95°C for 15 seconds, and 60°C for 1 minute, and a total of 40 cycles were carried out. These results are shown in Fig. 5.

· cDNA synthesis primer for Gag
primer 1 (SEQ ID NO: 26)
5'- TGCAGGATATGGGCC-3'.

· primer set for Gag

primer 2 (SEQ ID NO: 27)
5'-AGTACCTTTGCTCCTCCCTC-3'

primer 3 (SEQ ID NO: 28)
5'-TAATACCTCGGGTTTCGGCC-3'

· probe for Gag

probe (SEQ ID NO: 29)
5'-TTCCCTCCATCACCAGCTAGATAGCCT-3'

· primer set for Tax

primer 4 (SEQ ID NO: 30)
5'-CCGCCGATCCCAAAGAA-3'

primer 5 (SEQ ID NO: 31)
5'-CCTGTCCAAACCCTGGGAA-3'

Fig. 5 contains graphs showing the results of RT-qPCR. In Fig. 5, the horizontal axis indicates the type of sample and the vertical axis indicates the relative mRNA expression level. Fig. 5(A) reports the expression level of Gag mRNA in each individual cell line, Fig. 5(B) reports the expression level of Tax mRNA in each individual cell line, Fig. 5(C) reports the expression level of Gag mRNA in the ATL case samples, and Fig. 5(D) reports the expression level of Tax mRNA in the ATL case samples. As shown in Fig. 5, it was demonstrated that Gag was expressed in ATL cells and ATL case samples and even in ATL cells or ATL case samples that did not express Tax, revealing a Tax-independent Gag expression mechanism.

(3) Investigation of Gag expression in Tax-nonexpressing cells

Whether Gag was expressed in cells that did not express Tax was then investigated. Specifically, a proximity ligation assay (PLA) was performed. PLA was performed using a Duolink PLA kit (Sigma-Aldrich) according to the manufacturer's instructions. The cells used were MT4 cells, ATL-55T(+) cells, and ED cells, which are ATL cell lines, while Jurkat cells, a T cell line, were used as a negative control. The cells were concentrated onto glass coverslips using a Cytospin (Cytospin 2, Shandon). After concentration, fixing was carried out by the addition of 4% paraformaldehyde using conditions of 15 minutes at room temperature. After fixation, a permeabilization treatment was carried out by the addition of 0.2% Triton (registered trademark) X-100 using conditions of 15 minutes at room temperature. After the permeabilization treatment, blocking was carried out using Duolink In Situ PLA Probe-Blocking Solution. After blocking, the cells were stained with antibodies. The following were used for staining: mouse anti-HTLV-1 p24 monoclonal antibody (clone 46/3.24.4, ZeptoMetrix), rabbit polyclonal antibody against HTLV-1 p24 Gag (Cat No. 5418, ABL), or mouse anti-HTLV-1 p19 Gag monoclonal antibody (Cat No. 0801003, ZeptoMetrix). After staining, the glass coverslip was sealed with DAPI-containing In Situ Mounting Medium (Sigma-Aldrich). After sealing, the cells were observed using a Nikon C2 confocal microscope (Nikon). The results are reported in Fig. 6.

Photographs showing the results of the proximity ligation assay are given in Fig. 6. Fig. 6(A) shows the results for the Gag p19 antibody. Fig. 6(B) shows the results for the Gag p24 antibody. In Fig. 6, the upper row shows the results of DAPI staining, the middle row shows the results of Gag p19 or Gag p24 staining, and the lower row shows the results of DAPI and Gag p19 or Gag p24 staining. In Fig. 6, the area surrounded by the dashed line indicated by the arrows shows the expression of Gag p19 or Gag p24. As shown in Fig. 6(A), it was found that Gag p19 was expressed not only in Tax-expressing MT4 cells, but also in ATL-55T(+) cells and ED cells, which do not express Tax. Furthermore, as shown in Fig. 6(B), Gag p24 was found to be expressed not only in Tax-expressing MT4 cells, but also in ATL-55T(+) cells and ED cells, which do not express Tax. These results demonstrated that Gag p19 and p24 are expressed in ATL cells, regardless of the presence/absence of Tax expression.

(4) Investigation of the cellular immune response due to Gag in ATL patients after hematopoietic stem cell transplantation

An investigation was then carried out into whether a cellular immune response is induced by Gag in ATL patients who had undergone hematopoietic stem cell transplantation and had maintained long-term remission. Specifically, an ELISpot assay was used. Overlapping peptides with a length of 9 amino acids (offset: 1 amino acid) were designed based on the amino acid sequence of Gag (GenBank accession number AAA85841.1, SEQ ID NO: 1). 97 peptides for Gag p15 were pooled as p15-1 ($p15_{1-50}$) and p15-2 ($p15_{51-97}$). 121 peptides for Gag p19 were pooled as p19-1 ($p19_{1-50}$), p19-2 ($p19_{51-100}$), and p19-3 ($p19_{101-121}$). Peripheral blood mononuclear cells (PBMCs) were collected from ATL patients who had undergone hematopoietic stem cell transplantation and had maintained long-term remission. These were submitted to an ELISpot assay using a human IFN-$\gamma$ ELISpot kit (MABTECH). The PBMCs were seeded onto an ELISpot plate. After seeding, 0.25 $\mu$mol/L pooled peptide and 1 $\mu$g/mL Purified NA/LE Mouse Anti-Human CD28 antibody (Cat No: 555725, BD Biosciences) were added. Stimulation was carried out for 6 hours after the addition. Spots for IFN-$\gamma$-producing cells were developed using an AP Conjugate Kit (Bio-Rad) and were counted using an ImmunoSpot S6 Analyzer (Cellular Technology Limited). These results are given in Fig. 7.

Photographs showing the results of the ELISpot assays are given in Fig. 7. In Fig. 7, the left shows the case of stimulation with Gag p19 peptide. In Fig. 7, the upper and middle panels on the right show the case of stimulation with Gag p15 peptide, and the lower panel on the right panel shows the case of no stimulation. As shown in Fig. 7, spots were formed when stimulation with Gag p19 or Gag p15 peptide was carried out. These results demonstrated that IFN-γ was produced due to Gag p19 or Gag p15 in PBMCs from ATL patients who had undergone hematopoietic stem cell transplantation and had maintained long-term remission. In addition, the induction of a cellular immune response was suggested by the production of IFN-γ.

(5) Investigation of the cellular immune response due to Gag in mice

An investigation was then carried out into whether a cellular immune response was induced in mice immunized with recombinant Gag protein. Specifically, an ELISpot assay was used. Gag p15 peptides and Gag p19 peptides were designed and pooled using the same method as in Reference Example 1(4). Gag p24 peptides were designed using the same method as in Reference Example 1(4). 206 Gag p24 peptides were pooled as p24-1 ($p24_{1-51}$), p24-2 ($p24_{52-103}$), p24-3 ($p24_{104-154}$), and p24-4 ($p24_{155-206}$). Mice (C57BL/6J, Jackson Laboratory Japan) were infected with HTLV-1, and splenocytes were collected from the mice after 14 days. The ELISpot procedure was carried out using the same method as in Reference Example 1(4), except that the splenocytes collected from the mice were used as samples.

Photographs showing the results of the ELISpot assays are given in Fig. 8. In Fig. 8, the top row (A) shows the results for splenocytes from mouse 1, the middle row (B) shows the results for splenocytes from mouse 2, and the bottom row (C) shows the results for splenocytes from mouse 3. In Fig. 8 from left to right, 2 columns (Gag p15-1 and 2) show the case of stimulation with Gag p15; 3 columns (Gag p19-3 to 5) show the case of stimulation with Gag p19; 4 columns (Gag p24-6 to 9) show the case of stimulation with Gag p24; 1 column (10) shows the positive control; and 1 column (11) shows the negative control. As shown in Fig. 8, spots were formed when stimulation with Gag p19, Gag p15, or Gag p24 peptides was carried out. In particular, spot formation was substantial for stimulation with the Gag p19-3 peptides. These results demonstrated that IFN-γ was produced due to Gag p15, Gag p19, or Gag p24 in mice immunized with Gag proteins. The induction of a cellular immune response was suggested by this production of IFN-γ.

Industrial Applicability

The lipid complex or pharmaceutical composition of the present disclosure can induce an immune response against HTLV-1. The lipid complex and pharmaceutical composition of the present disclosure are thus applicable, for example, to the prevention and/or treatment of HTLV-1-associated diseases.

## Claims

**1.** A lipid complex comprising at least one nucleic acid selected from:

a nucleic acid comprising a polynucleotide that encodes an immunogenic fragment of human T-cell leukemia virus 1 (HTLV-1) antigenic Gag protein;
a nucleic acid comprising a polynucleotide that encodes an immunogenic fragment of HTLV-1 antigenic Tax protein; and
a nucleic acid comprising a polynucleotide that encodes an immunogenic fragment of HTLV-1 antigenic HBZ protein;

wherein the at least one nucleic acid is encapsulated in a lipid.

**2.** The lipid complex according to claim 1, wherein the nucleic acid is at least one selected from:

a nucleic acid comprising a polynucleotide that encodes HTLV-1 antigenic Gag protein p15 (Gag p15) or an immunogenic fragment thereof;
a nucleic acid comprising a polynucleotide that encodes HTLV-1 antigenic Gag protein p19 (Gag p19) or an immunogenic fragment thereof;
a nucleic acid comprising a polynucleotide that encodes HTLV-1 antigenic Gag protein p24 (Gag p24) or an immunogenic fragment thereof;
a nucleic acid comprising a polynucleotide that encodes HTLV-1 antigenic Tax protein or an immunogenic fragment thereof; and
a nucleic acid comprising a polynucleotide that encodes HTLV-1 antigenic HBZ protein or an immunogenic

fragment thereof.

3. The lipid complex according to claim 1 or 2, wherein the lipid comprises a cationic lipid and at least one lipid selected from the group consisting of: neutral lipids; polyethylene glycol-modified lipids; and sterols.

4. The lipid complex according to any one of claims 1 to 3, wherein the nucleic acid comprises a polynucleotide that encodes at least one selected from: Gag p15 or an immunogenic fragment thereof; Gag p19 or an immunogenic fragment thereof; and Gag p24 or an immunogenic fragment thereof.

5. The lipid complex according to any one of claims 1 to 4, wherein the nucleic acid comprises a polynucleotide that encodes:

(i) at least one amino acid sequence selected from SEQ ID NOs: 1, 4, 7, 8, and 11 to 14;
(ii) an amino acid sequence having at least 80% homology with at least one amino acid sequence selected from SEQ ID NOs: 1, 4, 7, 8, and 11 to 14;
(iii) an amino acid sequence in which 1 to 31 amino acids are deleted, substituted, inserted, and/or added in an amino acid sequence with SEQ ID NO: 1, 4, 7, 8, 11, 12, 13, or 14;
(iv) an amino acid sequence comprising at least one amino acid sequence selected from SEQ ID NO: 11 or SEQ ID NO: 12, SEQ ID NO: 13, and SEQ ID NO: 14, and having at least 80% homology with SEQ ID NO: 1;
(v) an amino acid sequence comprising at least one of the amino acid sequences at positions 1 to 59, 52 to 109, or 102 to 130 of SEQ ID NO: 1;
(vi) an amino acid sequence comprising at least one of the amino acid sequences at positions 1 to 59, 52 to 109, or 102 to 130 of SEQ ID NO: 1, and having at least 80% homology with SEQ ID NO: 1;
(vii) an amino acid sequence comprising at least one of the amino acid sequences at positions 151 to 208 or 251 to 308 of SEQ ID NO: 4;
(viii) an amino acid sequence comprising at least one of the amino acid sequences at positions 151 to 208 or 251 to 308 of SEQ ID NO: 4, and having at least 80% homology with SEQ ID NO: 4;
(ix) the amino acid sequence at positions 1 to 58 of SEQ ID NO: 8; or
(x) an amino acid sequence comprising the amino acid sequence at positions 1 to 58 of SEQ ID NO: 8 and having at least 80% homology with SEQ ID NO: 8.

6. The lipid complex according to any one of claims 1 to 5, wherein the nucleic acid comprises a polynucleotide that encodes an antigenic Gag protein.

7. The lipid complex according to any one of claims 1 to 6, wherein the lipid complex is a lipid nanoparticle (LNP).

8. The lipid complex according to any one of claims 1 to 6, wherein the nucleic acid is mRNA.

9. The lipid complex according to claim 7, wherein the nucleic acid comprises at least one polynucleotide selected from:

(i) a polynucleotide composed of at least one base sequence selected from the group consisting of SEQ ID NOs: 2, 3, 5, 6, 9, 10, and 15 to 17;
(ii) a polynucleotide composed of a base sequence in which 1 to 94 bases are deleted, inserted, substituted, and/or added in a base sequence according to (i);
(iii) a polynucleotide composed of a base sequence having at least 80% identity with a base sequence according to (i);
(iv) a polynucleotide that hybridizes under stringent conditions with a polynucleotide composed of a base sequence complementary to a base sequence according to (i);
(v) a polynucleotide composed of at least one base sequence at positions 1 to 177,154 to 327 or 304 to 390 of SEQ ID NO: 3;
(vi) a polynucleotide composed of a base sequence that comprises at least one of the base sequence at positions 1 to 177 ,154 to 327, or 304 to 390 of SEQ ID NO: 3, and that has at least 80% identity with the base sequence of SEQ ID NO: 3;
(vii) a polynucleotide composed of at least one base sequence at positions 451 to 624 or 751 to 924 of SEQ ID NO: 6;
(viii) a polynucleotide composed of a base sequence that comprises at least one of the base sequence at positions 451 to 624 or 751 to 924 of SEQ ID NO: 6, and that has at least 80% identity with the base sequence of SEQ ID NO: 6;

(ix) a polynucleotide composed of the base sequence at positions 1 to 174 of SEQ ID NO: 10;
(x) a polynucleotide composed of a base sequence that comprises the base sequence at positions 1 to 174 of SEQ ID NO: 10 and that has at least 80% identity with the base sequence of SEQ ID NO: 10.

**10.** The lipid complex according to any one of claims 1 to 9, wherein the cationic lipid comprises a compound represented by the following formula (1):

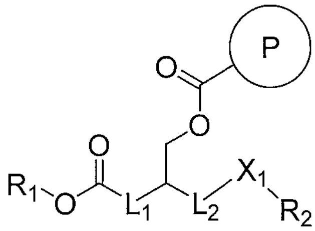

(I)

[In the formula, $L_1$ and $L_2$ each independently represent a $C_{3-10}$ alkylene group, $R_1$ and $R_2$ each independently represent a $C_{4-22}$ alkyl group or a $C_{4-22}$ alkenyl group, $X_1$ represents a single bond or - CO - O -, and the ring P represents any of the following formulas (P-1) to (P-6).]

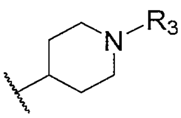
(P-1)

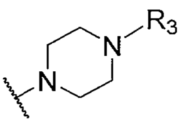
(P-2)

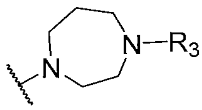
(P-3)

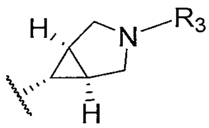
(P-4)

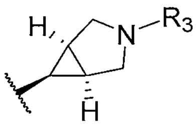
(P-5)

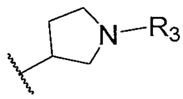
(P-6)

[In the formulas, $R_3$ represents a $C_{1-3}$ alkyl group.]
or a pharmaceutically acceptable salt thereof.

**11.** The lipid complex according to any one of claims 1 to 10, wherein the cationic lipid comprises 2-{9-oxo-9-[(3-pentyloctyl)oxy]nonyl}dodecyl 1-methylpiperidine-4-carboxylate represented by the following formula (II):

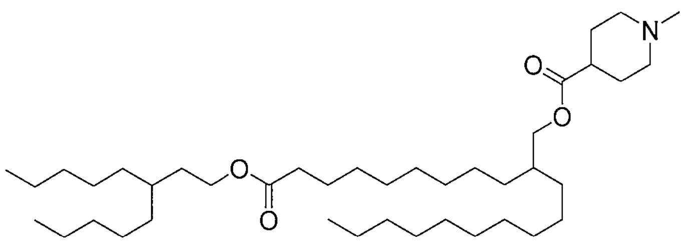

(II)

or a pharmaceutically acceptable salt thereof.

**12.** A pharmaceutical composition comprising the lipid complex according to any one of claims 1 to 11.

**13.** The pharmaceutical composition according to claim 12, wherein the pharmaceutical composition is a vaccine.

**14.** The pharmaceutical composition according to claim 12 or 13, for use for the prevention and/or treatment of an HTLV-1-associated disease.

**15.** Use of the lipid complex according to any one of claims 1 to 11 in the manufacture of a pharmaceutical composition.

[Fig. 1]

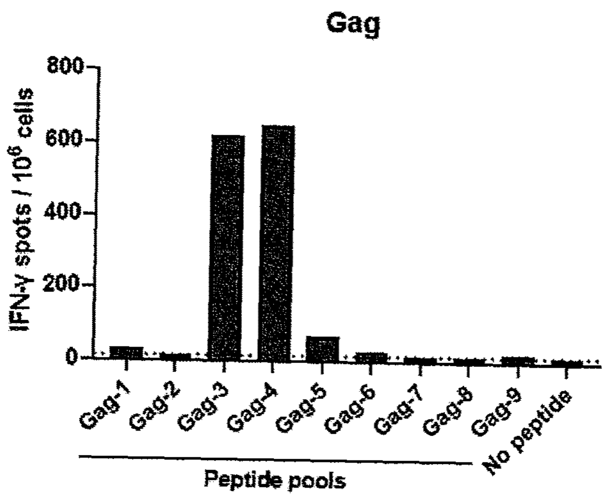
Gag
IFN-γ spots / 10^6 cells
800
600
400
200
0
Gag-1
Gag-2
Gag-3
Gag-4
Gag-5
Gag-6
Gag-7
Gag-8
Gag-9
No peptide
Peptide pools

[Fig. 2]

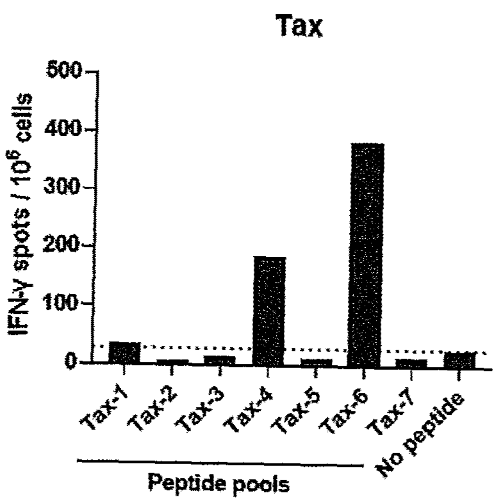
Tax
IFN-γ spots / 10^6 cells
500
400
300
200
100
0
Tax-1
Tax-2
Tax-3
Tax-4
Tax-5
Tax-6
Tax-7
No peptide
Peptide pools

[Fig. 3]

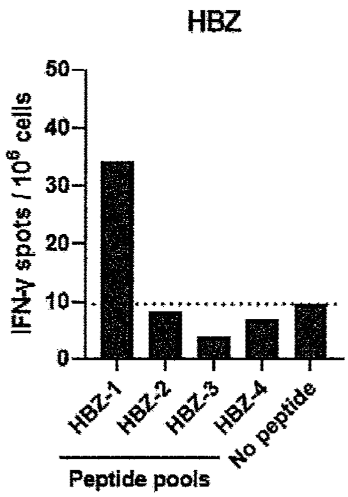
HBZ
IFN-γ spots / 10⁶ cells
50
40
30
20
10
0
HBZ-1
HBZ-2
HBZ-3
HBZ-4
No peptide
Peptide pools

[Fig. 4]

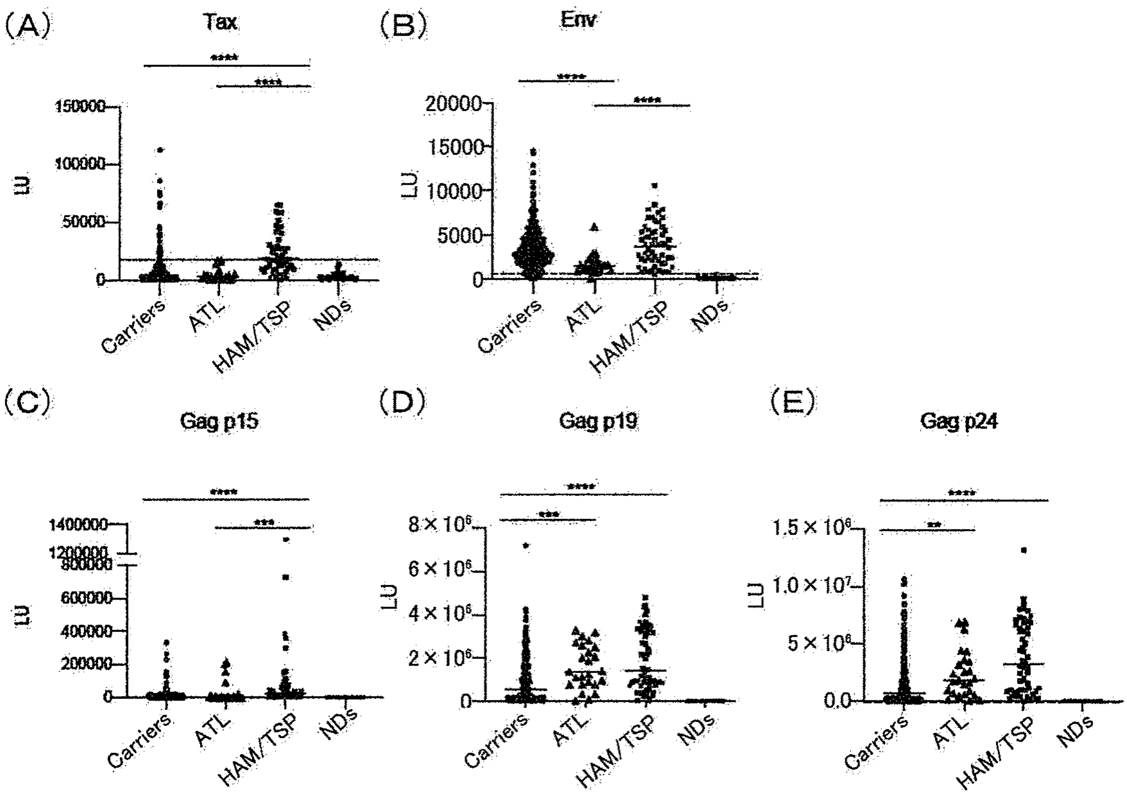
(A)
Tax
LU
150000
100000
50000
0
Carriers
ATL
HAM/TSP
NDs
(B)
Env
LU
20000
15000
10000
5000
0
(C)
Gag p15
1400000
1200000
800000
600000
400000
200000
(D)
Gag p19
$8\times10^6$
$6\times10^6$
$4\times10^6$
$2\times10^6$
(E)
Gag p24
$1.5\times10^6$
$1.0\times10^7$
$5\times10^6$
0.0

[Fig. 5]

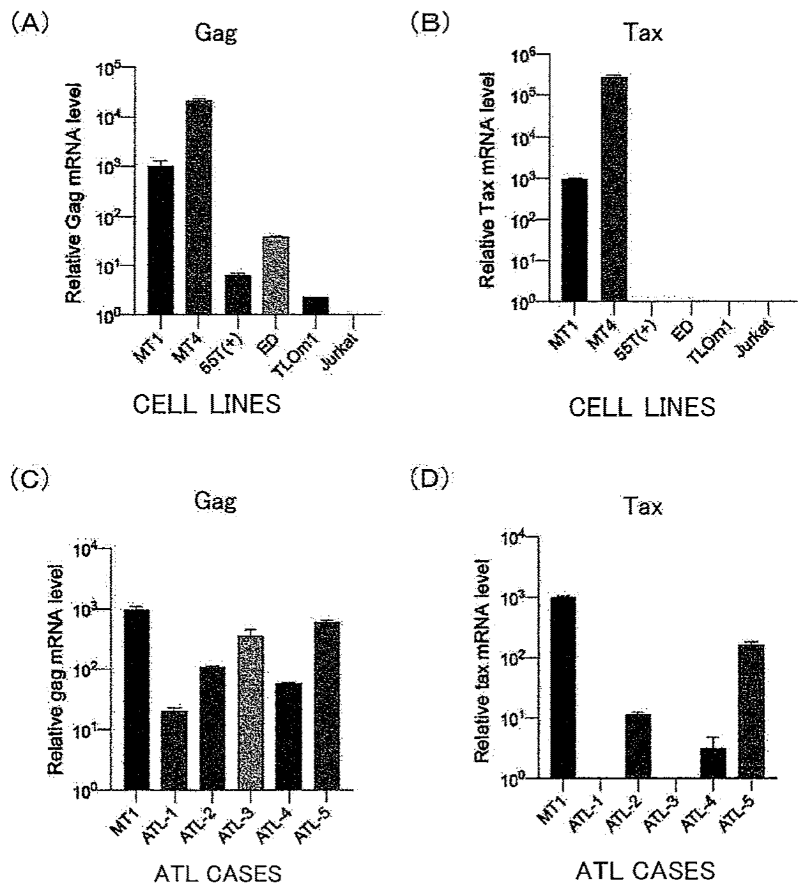
(A)
Gag
Relative Gag mRNA level
10^5
10^4
10^3
10^2
10^1
10^0
MT1
MT4
55T(+)
ED
TLOm1
Jurkat
CELL LINES
(B)
Tax
Relative Tax mRNA level
10^6
10^5
10^4
10^3
10^2
10^1
10^0
MT1
MT4
55T(+)
ED
TLOm1
Jurkat
CELL LINES
(C)
Gag
Relative gag mRNA level
10^4
10^3
10^2
10^1
10^0
MT1
ATL-1
ATL-2
ATL-3
ATL-4
ATL-5
ATL CASES
(D)
Tax
Relative tax mRNA level
10^4
10^3
10^2
10^1
10^0
MT1
ATL-1
ATL-2
ATL-3
ATL-4
ATL-5
ATL CASES

[Fig. 6]

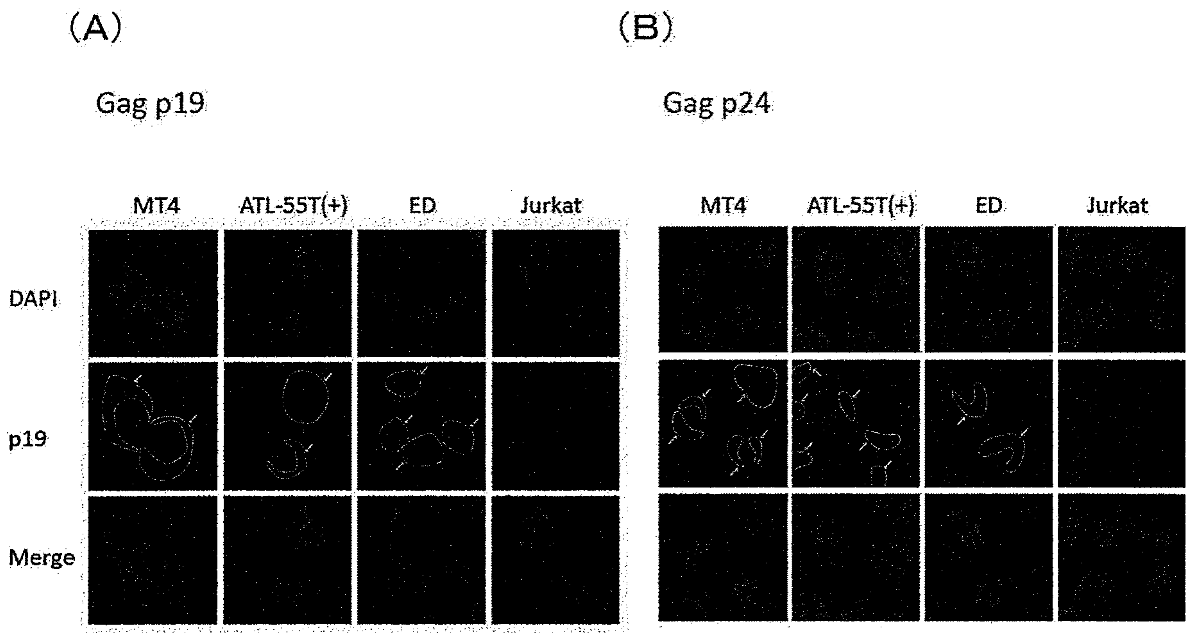
(A)
Gag p19
MT4
ATL-55T(+)
ED
Jurkat
DAPI
p19
Merge
(B)
Gag p24
MT4
ATL-55T(+)
ED
Jurkat

[Fig. 7]

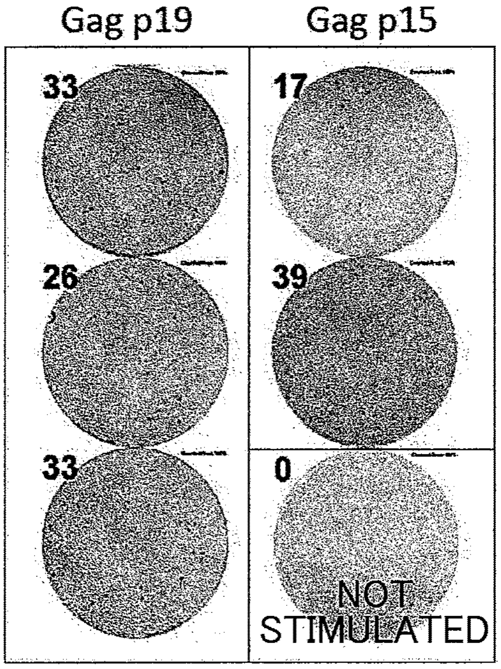
Gag p19
Gag p15
33
17
26
39
33
0
NOT
STIMULATED

[Fig. 8]

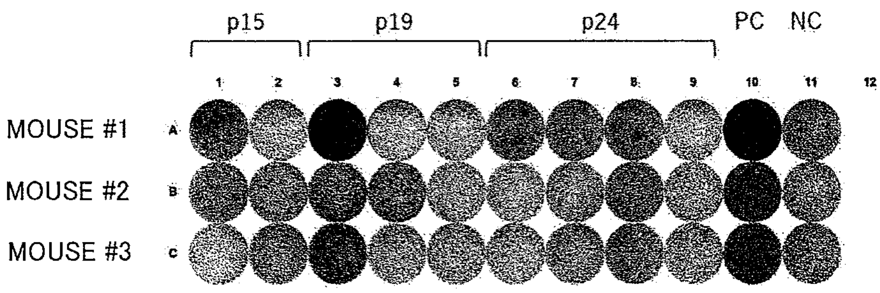
p15
p19
p24
PC
NC
1
2
3
4
5
6
7
8
9
10
11
12
MOUSE #1
A
MOUSE #2
B
MOUSE #3
C

**INTERNATIONAL SEARCH REPORT**

International application No.
**PCT/JP2024/017517**

**A. CLASSIFICATION OF SUBJECT MATTER**

***C12N 15/48***(2006.01)i; ***A61K 9/14***(2006.01)i; ***A61K 31/7088***(2006.01)i; ***A61K 47/22***(2006.01)i; ***A61K 47/24***(2006.01)i; ***A61K 47/28***(2006.01)i; ***A61K 47/34***(2017.01)i; ***A61P 37/04***(2006.01)i; ***C07K 14/15***(2006.01)i; ***C12N 15/88***(2006.01)i
FI: C12N15/48; A61K9/14; A61K47/22; A61P37/04; A61K31/7088; A61K47/34; A61K47/28; A61K47/24; C07K14/15; C12N15/88 Z ZNA

According to International Patent Classification (IPC) or to both national classification and IPC

**B. FIELDS SEARCHED**

Minimum documentation searched (classification system followed by classification symbols)

C12N15/00-15/90; A61K9/00-9/72; A61K31/00-33/44; A61K47/00-47/69; A61P1/00-43/00; C07K1/00-19/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/MEDLINE/EMBASE/BIOSIS (STN); JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br><br><br>Y | WO 2022/244801 A1 (NATIONAL INSTITUTES OF BIOMEDICAL INNOVATION, HEALTH AND NUTRITION) 24 November 2022 (2022-11-24)<br>claims, paragraphs [0009], [0039], [0044], [0066], [0075], examples | 1-3, 5, 7-9, 12-15<br><br><br>1-15 |
| Y | TU, J. J. et al. The Past, Present, and Future of a Human T-Cell Leukemia Virus Type 1 Vaccine. Frontiers in Microbiology. 2022, vol. 13, 897346<br>abstract, fig. 1, p. 6, right column, lines 36-51, p. 7, left column, lines 31-51 | 1-15 |
| Y | WO 2017/222016 A1 (EISAI R&D MANAGEMENT CO., LTD.) 28 December 2017 (2017-12-28)<br>claims, paragraph [0026] | 10-11 |
| A | WO 2003/031583 A2 (UNIVERSITY OF MIAMI) 17 April 2003 (2003-04-17)<br>claims | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 May 2024** | **09 July 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

INTERNATIONAL SEARCH REPORT

International application No.
**PCT/JP2024/017517**

**C. DOCUMENTS CONSIDERED TO BE RELEVANT**

| Category* | Citation of document, with indication, where appropriate, of the relevant passages | Relevant to claim No. |
| --- | --- | --- |
| P, X | WO 2023/220645 A1 (THE UNITED STATES OF AMERICA, AS REPRESENTED BY THE SECRETARY, DEPARTMENT OF HEALTH AND HUMAN SERVICES) 16 November 2023 (2023-11-16)<br>claims | 1-9, 12-15 |
| P, A | | 10-11 |

Form PCT/ISA/210 (second sheet) (July 2022)

**INTERNATIONAL SEARCH REPORT**

International application No.
**PCT/JP2024/017517**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/017517**

| Patent document cited in search report | Publication date (day/month/year) | Patent family member(s) | Publication date (day/month/year) |
|---|---|---|---|
| WO 2022/244801 A1 | 24 November 2022 | EP 4342941 A1<br>claim, paragraphs [0009], [0035], [0040], [0058], [0067], examples | |
| WO 2017/222016 A1 | 28 December 2017 | US 2019/0218180 A1<br>claims, paragraphs [0029]-[0032]<br>EP 3476832 A4 | |
| WO 2003/031583 A2 | 17 April 2003 | (Family: none) | |
| WO 2023/220645 A1 | 16 November 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2015105131 A
- WO 2016104580 A
- WO 2017222016 A
- WO 2019131580 A

### Non-patent literature cited in the description

- **COOK, LUCY B. et al.** Revised Adult T-Cell Leukemia-Lymphoma International Consensus Meeting Report.. *Journal of Clinical Oncology: official journal of the American Society of Clinical Oncology*, 2019, vol. 37 (8), 677-687
- Molecular Cloning: A Laboratory Manual. Cold Spring Harbor Laboratory Press, 1989
- *Biomembr.*, 2000, vol. 1468, 239-252
- *Nature Biotechnology*, 2019, vol. 37 (10), 1174-1185